(19)

Europäisches
Patentamt
European
Patent Office
Office européen
des brevets

(11)     EP 3 528 060 A1

(12)     **EUROPEAN PATENT APPLICATION**

(43) Date of publication:
21.08.2019   Bulletin 2019/34

(51) Int Cl.:
**G04C 17/00** (2006.01)        **G02B 26/00** (2006.01)
**G04G 9/02** (2006.01)

(21) Application number: **18215944.2**

(22) Date of filing: **20.08.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(30) Priority: **21.08.2009   US 34989709 P
31.05.2010   US 23572510 P**

(62) Document number(s) of the earlier application(s) in
accordance with Art. 76 EPC:
**10752392.0 / 2 480 267**

(71) Applicant: **Preciflex SA
2000 Neuchâtel (CH)**

(72) Inventor: **Vouillamoz, Lucien
8835 Feusisberg (CH)**

(74) Representative: **Mötteli-Mantelli, Novella
Da Vinci Partners LLC
Rathausgasse 1
9320 Arbon (CH)**

Remarks:
This application was filed on 31.12.2018 as a
divisional application to the application mentioned
under INID code 62.

(54)     **VISUAL INDICATOR**

(57)      A device for fluid display comprising a fluid, wherein the fluid is displaced by an electrowetting process. The device is filled with at least 2 immiscible fluids, whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode. Also provided is a method of switching the control electrodes of the device above-mentioned device in a sequence so that a portion of the fluid is displaced within the device.

FIG. 14

**Description**

**Cross Reference to Related Applications**

This is a divisional application of the European patent application number 10752392.0 which claims the benefit of U.S. Provisional Application No. 61/235,725, filed 21 August 2009 and U.S. Provisional Application 61/349,897, filed 31 May 2010, the contents of which are incorporated herein by reference and relied upon to define features for which protection may be sought hereby as it is believed that the entirety thereof contributes to solving the technical problem underlying the invention, some features that may be mentioned hereunder being of particular importance. This application incorporates by reference the contents of PCT Appl. No. PCT/IB2010/002054 of the same applicant, entitled FLUID INDICATOR, filed on the 20th of August, 2010.

**Copyright & Legal Notice**

A portion of the disclosure of this patent document contains material which is subject to copyright protection. The copyright owner has no objection to the facsimile reproduction by anyone of the patent document or the patent disclosure as it appears in the Patent and Trademark Office patent file or records, but otherwise reserves all copyright rights whatsoever. Further, no reference to third party patents or articles made herein is to be construed as an admission that the present invention is not entitled to antedate such material by virtue of prior invention.

**Background of the Invention**

This invention relates to indicators and in particular analog visual indicators used to dispense a measured amount of liquid.

Analog indicators have existed since time immemorial. The hour glass, for example, uses sand or fluid which, influenced by the weight of gravity, moves from one reservoir to another by passing through a small aperture therebetween. Another example of an ancient analog indicator is the "Clepsydra", as illustrated in "Horloges Anciennes" by Richard Mühe and Horand M. Vogel, French Edition, Office du Livre, Fribourg, 1978, page 9.

Referring to **FIG. 1**, US Patent No. 3,783,598 describes an instrument 1 having a movement 2, a drive shaft 3, cams 4, pistons 5, fluid filled capillaries 6 and a relief chamber 7 used to indicate time. Automated fluid dosage devices exist. A typical insulin pump is a computerized device that looks like a pager and is usually worn on the patient's waistband or belt. The pump is programmed to deliver small, steady doses of insulin throughout the day. Additional doses are given to cover food or high blood glucose levels. The pump holds a reservoir of insulin that is attached to a system of tubing called an infusion set. Most infusion sets are started with a guide needle, then the plastic cannula (a tiny, flexible plastic tube) is left in place, taped with dressing, and the needle is removed. The cannula is usually changed every 2 or 3 days or when blood glucose levels remain above target range. However, such devices are bulky and are not always located at a place on the body that is easy to access or read.

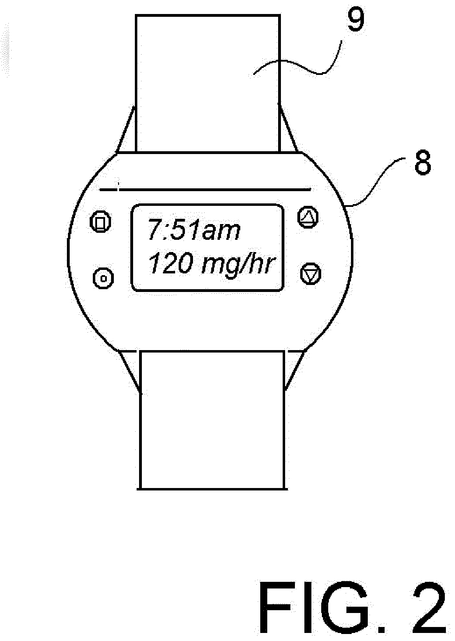

Referring to **FIG. 2**, a wrist worn device, such as the "GLUCOWATCH" is known. This prior art device, said to be developed in 2001, has a casing 8 supported on a bracelet 9. A reservoir dispenses insulin onto a patch similar to a transdermal medication patch used for smoking cessation and hormone therapy. It therefore provides a non-invasive, needle-free method of enhancing and controlling the transport of water-soluble ionic drugs out of the skin and surrounding tissues using a low level of electrical current.

French patent No. 1552838 teaches putting a blob of mercury in an electrical field, i.e., expose it to a voltage differential, which may deform the blob, and having a flow of electrons through the blob to displace it from one electrode to another. This is believed to involve 2 distinct phenomena: the deformation of the blob under the effect of the electrical field usually considered as involving electrowetting, and the displacement of the blob by a flow of electrons usually considered electrophoresis. Still further, such system has the disadvantage of creating a current flow through the mercury, which effects the mercury by, for example, heating it. Still further, mercury is considered a hazardous liquid.

These prior devices are cumbersome, requiring significant or dedicated space for indicating the value, lack accuracy, do not function as proposed, or are too costly for many users.

What is needed is a visual indicator that provides a quickly read indication of a measured dosage value and is inexpensive to manufacture.

**Summary of the Invention**

A visual indicator display device includes a bracelet, a transparent capillary chamber, and a displacement member. The transparent capillary chamber is matched to an indicia and has a primary length and a width less than the primary length. The displacement member is functionally disposed at one end of the capillary chamber and is responsive to a measureable input for moving a fluid contained therein a defined amount.

An object of the invention is to provide a visual indicator which takes up minimal space.

Another object of the invention is to provide a flexible visual indicator which adapts to requirements which do not readily permit a straight, rigid indicator, such as when such indicator is worn on a wrist, ankles, a head or around or along some part of human body, or on objects such as clothes and sporting articles.

Another object of the invention is to provide an aesthetic, comfortable, reliable and intellectually attractive indicator.

Another object of the invention is to provide a dispenser of fluids such as drugs, medication, ointment, oils or perfumes.

**Brief Description of the Drawings**

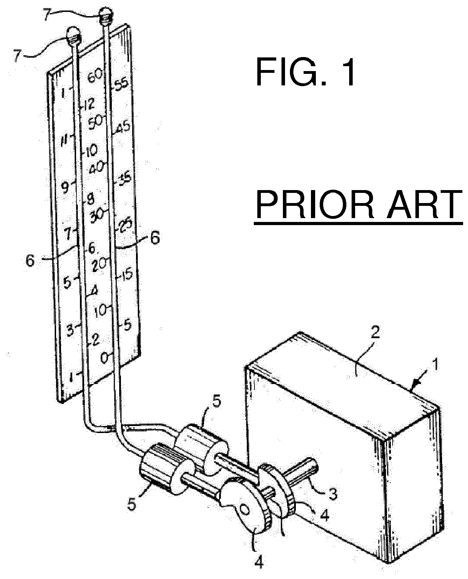

FIG. 1 is a side, cross-sectional view of an analog indicator of the prior art.

FIG. 2 is a top view of a second indicator of the prior art.

FIG. 3 **is** a side, cross-sectional view of a first embodiment of the invention.

FIG. 4A is a perspective view of a second embodiment of the invention.

FIG. 4B is a second perspective view of the second embodiment of the invention.

FIG. 5A is a second embodiment of the invention, used as a drug dispenser.

FIG. 5B is a side view of a cartridge for use in the embodiment of FIG. 5A.

FIG. 5C is a perspective view of a cartridge for use in the embodiment of FIG. 5A, shown in a flexed state.

FIG. 6 is a partially disassembled view of the fluid displacement device of the invention, having one reservoir.

FIG. 7 is a cross-sectional view of a reservoir and displacement member of the invention, showing features which aid in initializing the invention.

FIGS. 8A-8E are progressive views of different stages of operation of the mechanical embodiment of FIG. 8F.

FIG. 8F is a cross-sectional side view of a fully mechanical embodiment of the invention.

FIG. 9 is a schematic view of an embodiment of the invention for textile applications.

FIGS. 10A-10B are side by side photos of a droplet undergoing the electrowetting effect, in which FIG. 10A shows the droplet without voltage applied to an electrode and. FIG. 10B shows the droplet with voltage applied to an electrode.

FIG 11 is a cross-sectional, schematic view of an electrowetting display.

FIGS. 12A-12D are time sequence photos showing the displacement of a droplet of water in silicone oil, with an electrode pitch of 1 mm, and a height of 400 $\mu$m.

FIG. 13 is a cross-sectional view of an alternative embodiment of the analog sensor over the entire tube.

FIG. 14 is a cross-sectional view of an alternative embodiment of a digital sensor of the invention, implemented on an electrowetting display.

FIG. 15A to FIG. 15D are tables showing considerations of requirements of elements of the invention.

FIG. 16A is a side, cross-sectional view of a first embodiment of the invention, such as in FIG. 3.

**FIG. 16B** is a block diagram related to the embodiment shown in **FIG. 16A.**

**FIG. 16C** is a block diagram of a preliminary design of the invention.

**FIG. 17A** is another block diagram of the invention.

**FIG. 17B** is a still another block diagram of all actuators of the first phase.

**FIG. 17C** is a function diagram of phase 1.

**FIG. 18A** are optional solutions for the phase interfaces.

**FIG. 18B** is a table considering phases interface, displacement of the liquid and detection of the liquid position functions.

**FIG. 18C** is a diagram showing vapor pressure vs. temperature for different liquids.

**FIG. 18D** is a block diagram of alternate means for the displacement of the liquid of the invention.

**FIG. 19A** to **19D** is a table considering solutions for the displacement of the liquid.

**FIG. 20** is a table discussing evaluation criteria for the liquid displacement systems.

**FIG. 21** is a table discussion the ranking of solutions for the displacement of the liquid.

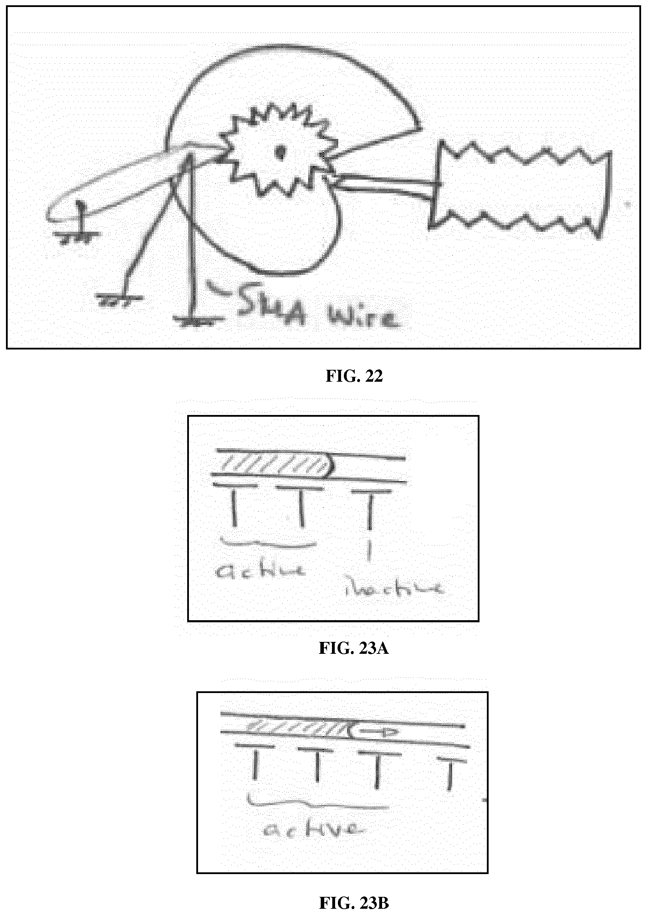

**FIG. 22** is a Shape-Memory Alloy (SMA) ratchet actuating a spiral wheel of the invention.

**FIGS. 23A** to **23B** are schematics of fluid moved by electrowetting of the invention.

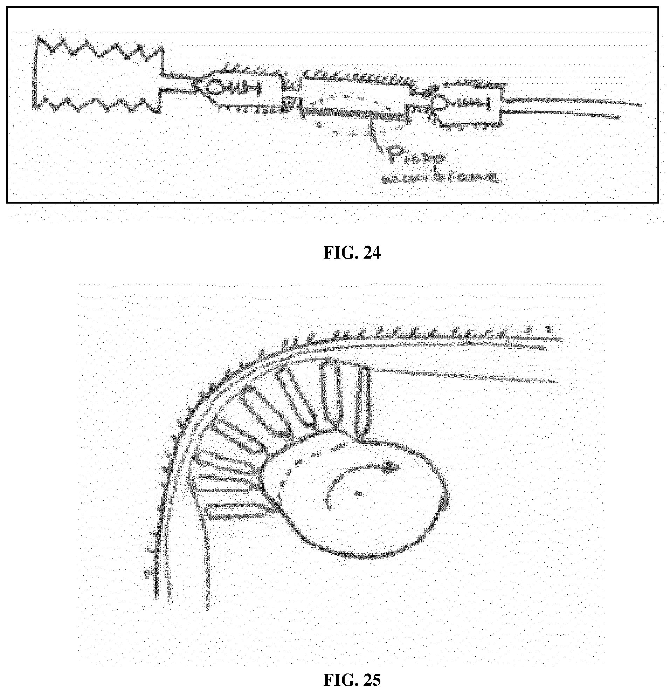

**FIG. 24** is a schematic of a piezo membrane pump of the invention.

**FIG. 25** is a schematic view of a circular peristaltic pump of the invention.

**FIGS. 26A** to **26B** are schematic representations of the spiral wheel design, with a possible implementation of a clutch to allow a manual setting of the display.

**FIG. 27** is a perspective view of a Nanopump, a device designed by Debiotech. of the invention.

**FIG. 28** is a schematic view of an electromagnetic membrane pump of the invention.

**FIGS. 29A** to **29B** are photos of the electrowetting effect, where, in **Fig. 29A**, no voltage is applied, and in **FIG. 29B**: voltage is applied.

**FIG. 30** is a schematic of the cross section of an electrowetting display.

**FIG. 31** is a sequence of displacement of a droplet of water in silicone oil with electrode pitch: 1 [mm], height: 400 [$\mu$m].

**FIG. 32** is an embodiment having an indicator of the invention with a liquid column, while inducing displacement on a droplet only.

**FIG. 33** is a plan view of a Squiggle drive of the invention.

**FIG. 34** are solution proposals for the detection of the indicator liquid position.

**FIG. 35** is a table discussing solutions for the detection of the liquid position.

**FIG. 36** is a table discussing evaluation criteria for the liquid sensing methods.

**FIG. 37** is a table discussing the ranking of the selected solutions of the liquid level sensors.

**FIGS. 38A** to **38B** are two different implementations of the capacitive sensor as either analog or a digital sensor on an electrowetting display.

**FIG. 39** is a schematic representation of an inductive sensor of the invention.

**FIG. 40A** is a schematic of an encoder system of the invention.

**FIG. 40B** is another schematic of an encoder wheel of the invention for an absolute positioning.

**FIG. 41** is a graph of the effect of temperature on liquid length in a tube.

**FIG. 42** is another graph of the effect of temperature on liquid length in a tube.

**FIG. 43** is a graph of final pressure in the decompression chamber vs. tube diameter and chamber volume.

**FIG. 44** is a contour plot of the final pressure in the decompression chamber vs. chamber volume and tube diameter.

**FIG. 45** is a 3D graph of isosurfaces of maximal force on the piston vs. tube diameter, chamber volume and piston diameter.

**FIG. 46** is a plot of piston stroke vs. tube diameter and piston diameter.

**FIG. 47** is a graph illustrating configurations allowing a function below 11 [mW] average power consumption (maximal admissible power), and below 3 [mW] (considering a 30% overall efficiency).

**FIG. 48** is a schematic of a liquid-vacuum interface.

**FIG. 49** is a graph of return time isosurfaces for a silicone-silicone interface.

**FIG. 50** is a graph of return time isosurfaces for a water-water interface.

**FIG. 51** is a schematic of the forces acting on the spiral ramp.

**FIG. 52** is a generalized spiral system with rigid compression chamber.

**FIG. 53** is an Archimedean spiral.

**FIG. 54** is a curve presenting required torque vs. angular position and chamber to tube volume ratio for a 2 [mm] tube.

**FIG. 55** is a graph of different ratios of torque vs. angular position for different chamber/tube volume ratios, for a 2 [mm] tube diameter.

**FIG. 56** is a graph of required torque on the spiral wheel vs. desired return time, for water and silicone oil.

**FIG. 57** is a cross-sectional schematic of the electrowetting principle, and equivalent electric schematic.

**FIG. 58** is a graph of displacement frequency of water in different media, as a function of the voltage.

**FIG. 59** are morphologic boxes presenting a summary of optional solutions, as well as global combinations.

**FIG. 60** is a table of five different options of displacement devices of the invention embodiment.

**FIG. 61** is a table discussing parameters of the embodiment 1 - spiral cam.

**FIG. 62** are photos of a watch movement of the invention.

**FIG. 63** are photos of off-the-shelf movements useable in the invention.

**FIG. 64A** is a schematics of a digital quartz watch.

**FIG. 64B** is a schematic of a mechanical watch.

**FIG. 65** is a graph of return spring force and reservoir thickness vs. reservoir diameter.

**FIG. 66A** is a top view of embodiment 1, flat, with the indicator tube and the watch movement.

**FIG. 66B** is a side view of the embodiment 1, flat.

**FIG. 66C** is a front view of the embodiment 1, flat.

**FIG. 67** is a cross sectional view through the reservoir of embodiment 1, flat.

**FIG. 68** is a perspective view of the cam wheel of embodiment 1, flat.

**FIG. 69A** is a top view of the embodiment 1 with a long reservoir.

**FIG. 69B** is a side view of a cross section through the embodiment 1 with a long reservoir.

**FIG. 70** is a top view of embodiment 1, packaged in a watch.

**FIG. 71** is a cross sectional view through the mechanism of the watch of **FIG. 70**.

**FIG. 72A** is a top view of embodiment 1 with a linear display, without the display mask.

**FIG. 72B** is a top view of embodiment 1 with a linear display, with the display mask.

**FIG. 72C** is side view of the embodiment 1 with the linear display of the invention.

**FIG. 73** is a flexible plastic bracelet of the invention.

**FIG. 74** is a side-by-side perspective and side view of an implementation of the spiral movement in a flexible bracelet.

**FIG. 75** is an optional implementation of the S shaped display, with the mechanism below the wrist.

**FIG. 76** is a schematic diagram of forces acting on the piston of the invention.

**FIG. 77** is a graph of torque vs. angular position for a 2 [mm] inner diameter wheel, 4.5 [mm] stroke.

**FIG. 78** is a table discussing torques.

**FIG. 79** is a schematic diagram of a 3 flip-flop based driver of the invention.

**FIG. 80** is a schematic diagram of the connection of the electrodes of the invention.

**FIG. 81** is a schematic diagram of the simplified sensing circuit of the invention.

**FIG. 82** is a more complete schematic diagram of the driving electronics of the invention.

**FIG. 83** is a table listing components required to drive the system of **FIG. 82**.

**FIG. 84** is a top and side view of an embodiment of the electrowetting display watch of the invention.

**FIGS. 85A** to **85E** is a schematic of an integration of a low cost electrical or high-end mechanical movement.

FIGS. 86A to 86D are views of assembly steps of the invention.

FIGS. 87A to 87F are views of embodiment 1 and the integration of a circular fluid channel in a watch of the invention.

FIGS. 88A to 88C are views of variable display variants and channel shapes of embodiment 1.

FIGS. 89A to 89H are perspective views of embodiment 2 and the integration in an elastic bracelet of the invention.

FIG. 90 is a perspective view of a variant of embodiment 2.

FIG. 91 is a top view of another variant of embodiment 2.

FIGS. 92A to 92F are perspective views of embodiment 3 and the integration in an "S" display of the invention.

FIG. 93 is a perspective view of a variant of embodiment 3.

FIG. 94 is a perspective view of a PCB with transparent ITO electrodes and electronic components of the invention.

FIG. 95A is a perspective view of detail A of FIG. 94, of the sensing electrodes of the invention.

FIG. 95B is the perspective view of detail A of FIG. 94, of the drive electrodes of the invention.

FIG. 96 is a schematic view of electrowetting.

FIG. 97 is a perspective view of the indication of time on a bracelet of the invention based upon electrowetting.

FIG. 98 is a perspective view of the time indication of FIG. 97 in detail.

FIG. 99 is a perspective view of the closing devices for the bracelet of the invention.

Those skilled in the art will appreciate that elements in the figures are illustrated for simplicity and clarity and have not necessarily been drawn to scale. For example, dimensions may be exaggerated relative to other elements to help improve understanding of the invention and its embodiments. Furthermore, when the terms "first", "second", and the like are used herein, their use is intended for distinguishing between similar elements and not necessarily for describing a sequential or chronological order. Moreover, relative terms like 'front', 'back', 'top' and "bottom", and the like in the description and/or in the claims are not necessarily used for describing exclusive relative position. Those skilled in the art will therefore understand that such terms may be interchangeable with other terms, and that the embodiments described herein are capable of operating in other orientations than those explicitly illustrated or otherwise described.

**Detailed Description of Preferred Embodiments**

The following description is not intended to limit the scope of the invention in any way as they are exemplary in nature and serve to describe the best mode of the invention known to the inventors as of the filing date hereof. Consequently, changes may be made in the arrangement and/or function of any of the elements described in the disclosed exemplary embodiments without departing from the spirit and scope of the invention.

A visual indicator display device includes a bracelet, a transparent capillary chamber, and a displacement member. The transparent capillary chamber is matched to an indicia and has a primary length and a width less than the primary length. The displacement member is functionally disposed at one end of the capillary chamber and is responsive to a measureable input for moving a fluid contained therein a defined amount.

A suitable fluid may be an oil, a lotion, or a liquid such as a drug or other medication. The displacement member is attached to one end of the capillary chamber which is responsive to a measureable input for displacing the indicator surface thus allowing the user to read a measurement from the indicia.

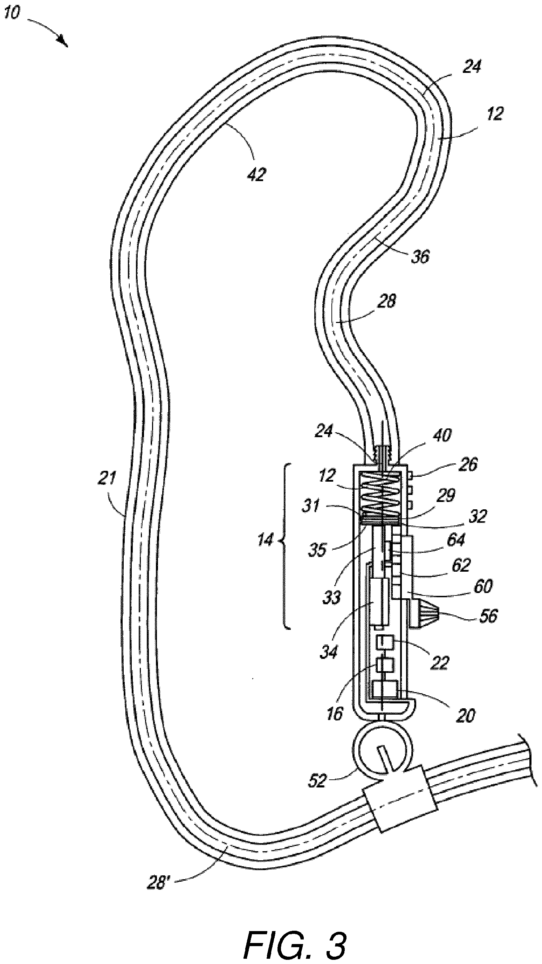

Referring to FIG. 3, an analog indicator 10 of the invention indicates dosage. The indicator 10 includes a reservoir 12, a pump 14, a measuring device 16, a feedback circuit in a controller 20 and a power supply 22'. The reservoir 12 has a longitudinal axis 24 along which a indicia or a scale device 26 is disposed and is adapted for containing a fluid 28 bounded by at least an indicator surface 30. In a preferred embodiment, the pump 14 is made up of the plunger 32 mounted on a screw 33 driven by a micro motor 34. The plunger 32 generally uses an O-ring seal 29 disposed about its circumference, to seal against the fluid 28 passing between the top and bottom surface 31 and 35, respectively, of the plunger. The pump 14 pumps the fluid 28 out of the reservoir 12, and into the catheter 36. In a preferred embodiment, the measuring device 16 is an electronic clock which measures time and communicates a measured value of time to the feedback circuit 20. The feedback circuit 20, powered by the power supply 22, receives a measured time input from the measuring device 16 corresponding to a position on the scale device 26 and, in response thereto, activates the pump 14 to pump or move the fluid 28 out of the reservoir 12, until the surface 30 reaches a desired position in relation to the corresponding position on the indicia 26 (generally calibrated to equal a desired rate of dispensing of the fluid). The power supply 22 powers the pump 14 and feedback circuit 20. As shown, the reservoir 12 communicates the fluid 28 into the catheter 36. A clasp 52 connects ends of the device 10 to create a bracelet 21.

Further, optionally, an optical fiber and an LED light source illuminate the fluid 28 in the reservoir 12 in a known manner.

A potentiometer 56 regulates the voltage setting to a displacement control system 60. The displacement control system 60 includes an incremental position sensor 62, for example, the tracker NSE-5310 (the specification of which is attached as Appendix A to U.S. Provisional Application No. 61/235,725, filed 21 August 2009, incorporated herein by reference hereto) located adjacent the plunger 32. This control system 60 includes encoding for direct digital output, in which a hall element array on the chip 62 is used to derive the incremental position of an external magnetic strip 64 placed adjacent the chip at a distance of approximately 0.3 mm (typically), the magnetic strip 64 being attached to the plunger 32 in order to translate therewith. This sensor array detects the ends of the magnetic strip to provide a zero reference point.

In an alternate embodiment, the power supply 22 can be solar cells, a wound watch spring, movement captured by an oscillating mass (such as used in automatic watches), or a pneumatic system storing compressed air.

To return the fluid 28 to an initial position, such as 6:00 AM, for example, the plunger 32 may be returned by a return spring 40 or a magnetic device (not shown). Other options are conceivable, of course, which include the return line 42, which allows simple reversing of the motor 34 to reset the indicator 10.

A suitable motor 34 is referred to by its trademark SQUIGGLE™, available from New Scale Technologies, Inc. of New York, USA.

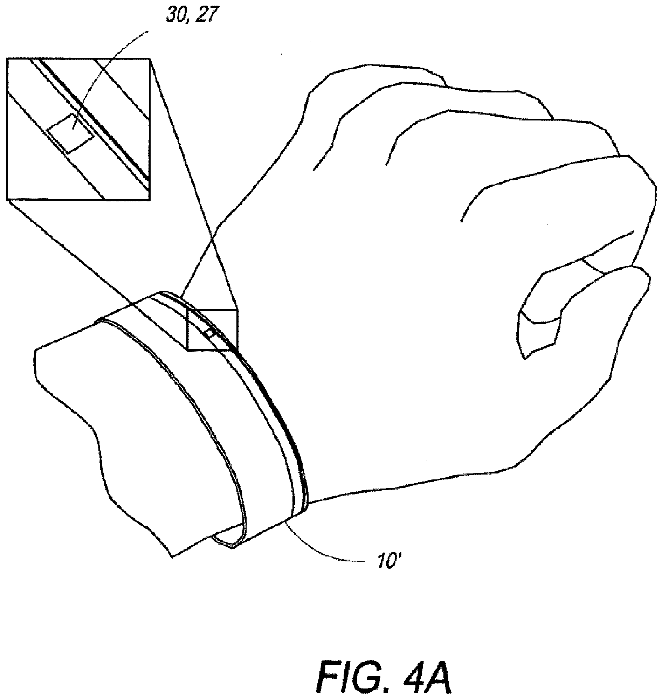

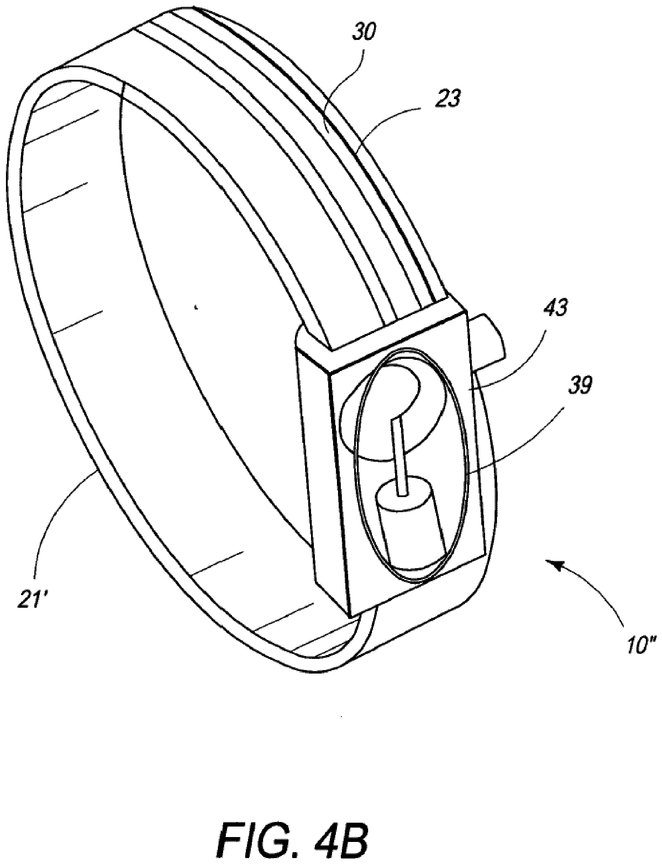

Referring now to **FIGs. 4A** and **4B**, an application of the analog indicator of the invention is a wrist watch or necklace 10 worn around the user's wrist. The reservoir 12' may be made of a transparent or translucent material, or a mixture of transparent and translucent material, formed in any desired shape. It may be made of plastic, rubber, silicon or any suitable material. An elastic material has the advantage that the bracelet 21' may be stretched over the user's wrist. In addition, the fluidic display 23 may be supplemented with a standard watch face 39 on the casing 43.

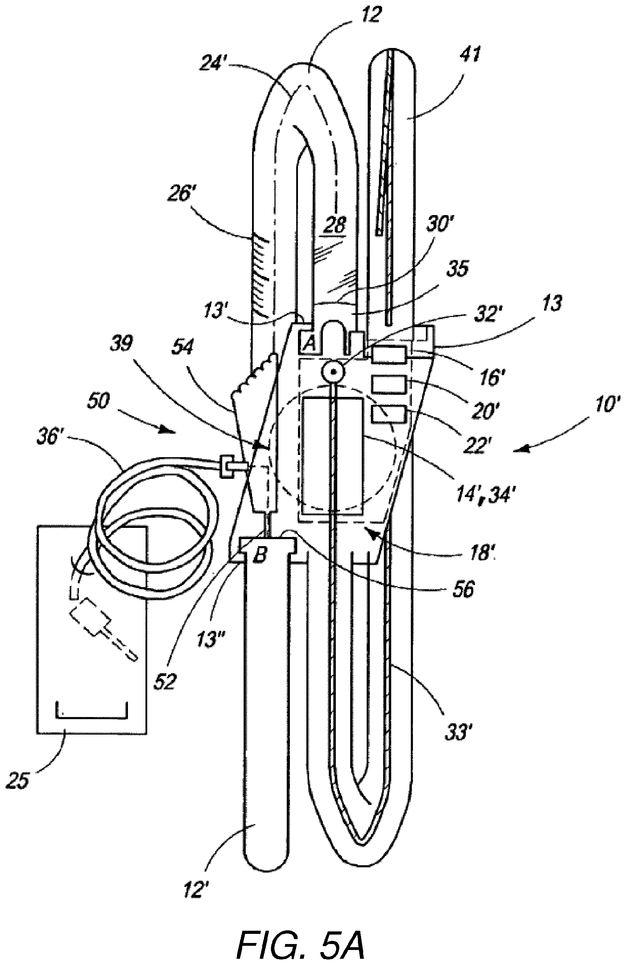

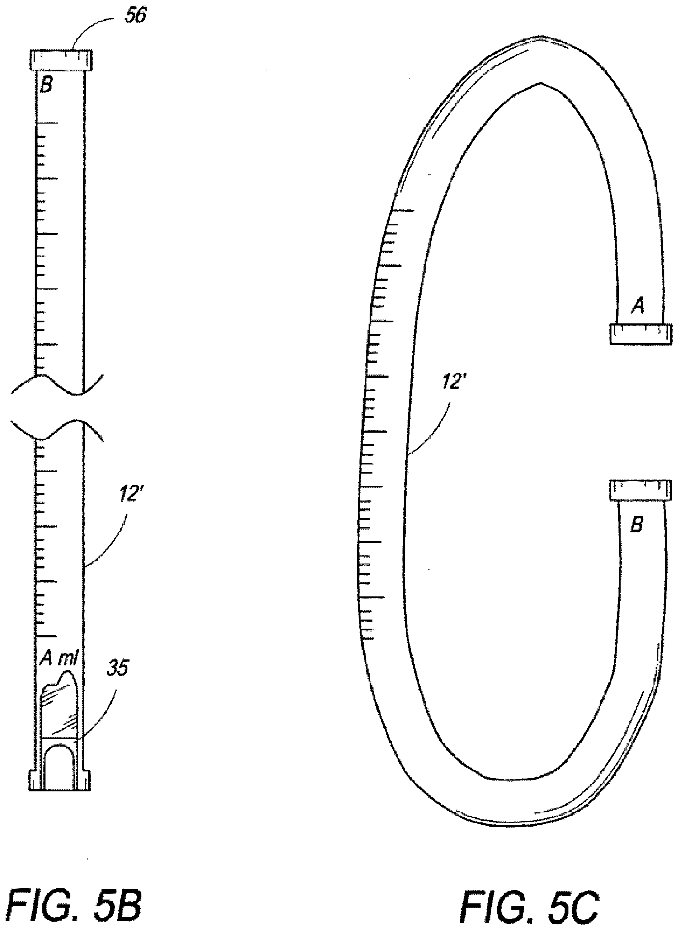

Referring now to **FIGs. 5A** to **5C**, the invention may be configured as a device 10" used to administer doses of liquid drugs 28 such as insulin. In such an embodiment, the flexible tube is a disposable drug reservoir cartridge 12' attached to housing 13 containing a dosage control device 18. The device 10" is carried like a wrist watch, with the flexible cartridge 12' serving as a portion of the band thereof. The indicator 10" includes the reservoir 12', a linear drive 14', an optional feedback circuit 16', a controller 20', and a power supply 22'. The reservoir 12' has a longitudinal axis 24' along which indicia 26' is disposed and is adapted for containing the fluid 28 bounded by at least an indicator surface 30'. In a preferred embodiment, the linear drive 14' drives a spherical plunger 32' mounted on a long flexible threaded shaft 33' which is driven by a micro motor 34'. The shaft 33' is preferably made of a superelastic material such as NITINOL. The linear drive 14' drives the plunger 32' against the piston 35 (preferably made of a flexible material such as rubber) which in turn presses the fluid 28 along the reservoir 12' and ultimately through the cannula tube or catheter 36', which then guides the fluid 28 into the patient's body. The electronics of the device 10" ensures that a programmed dosage of fluid is administered at regular intervals or constantly as prescribed by a physician. Note that optionally, the fluid 28, instead of passing into a wearer's body via a cannula, charges an absorptive patch 25 worn by the patient, for slow diffusion of the drug into the patient's body through the skin. Where a medication is administered via a patch 25, the patch may include an outer layer which is semi-permeable, in order to prevent the medication from evaporating before it has its intended effect (i.e. diffusion into the skin). Further, a perfume may be delivered in a similar manner. Particularly for the perfume dispensing embodiment, the patch may be located partially or entirely under the housing 13, or to the side of the housing and may be affixed thereto using a temporary adhesive rather than directly to the living organism, in order to avoid the need to attach the same to the living organism. Such a patch may be sized to be replaced in a defined area (such as circular area marked 39) against the back or any side of the housing 13, adjacent the living organism, much like a "POST-IT" note, so that replacement patches can readily replace soiled patches.

In a preferred embodiment, the number of turns of the linear drive 14' is recorded and controlled so as to ensure the proper dosage. The electronics are powered by the power supply 22'. Alternatively, the position of the piston 35 can be controlled in the manner as described in the above embodiment shown in **FIG. 3.** The cartridge 12' installs on one side 13' of the housing 13, with its piston 35 adjacent the plunger 32', and on the other side 13", adjacent a piercing mechanism 50 which includes a piercing tube 52 connected to a slidable tab 54. The user may slide the tab 54 to cause the piercing tube 52 to pierce the upper membrane 56 of the cartridge 12', in order to permit the communication of the fluid 28 through the cannula 38 into the patient's body. Where perfume is dispensed, this piercing served to open one end of the cartridge 12' to allow the delivery of perfume into the air, or via a conductive channel (not shown), to, near, or adjacent the skin of the user (for example, directly to and through the patch).

In the embodiment using an external magnetic strip (having a magnetic characteristic where the magnetic field generated thereby increases or decreases along the length of the cartridge) attached to or integrated on the cartridge 12', the computer controller can use this to regulate the dosage administered to the patient.

As with the prior embodiment, the power supply 22' can be a battery, solar power, a wound watch spring, an oscillating mass (such as used in automatic watches), or a pneumatic system storing compressed air,

After a cartridge 12' is fully dispensed, a button (not shown) on the housing 13 can be activated to retract the plunger 32'. The piston 35 remains stationary to prevent any aspiration of fluid from the patient, should the cannula still be connected to the body. Once retracted, the device 10" can be reloaded with a replacement cartridge 12'.

As with the earlier embodiment, a suitable motor 34 is the SQUIGGLE™ motor already described.

Note, that the housing 13 can be fitted with a watch face 39 and corresponding movement (not shown), in order that the drug administration device can also serve as a wrist watch.

Optionally, the threaded rod 33' of the drug administration device 10" is enclosed in a tube 41 which connects on the side 13" of the housing 13' and wraps around the wearer's wrist to reconnect to the side 13' of the housing, giving the visual effect of a two or multi-banded wrist watch.

It is foreseen that the cartridge 12' used in such drug administration device 10" would include a chemical litmus-type indicator which would indicate whether the insulin or other drug is suitable for continued injection. This indication could be expressed by an element of the cartridge 12' changing color, from a color that indicates the fluid is suitable for use, to another color that indicates the fluid is no longer suitable for use.

Still further, the device 10" can be used as a perfume dispenser by replacing the cannula with an aspirating head which can be manually (via a dispenser head or button) or automatically (via the dosage control of the invention) operated.

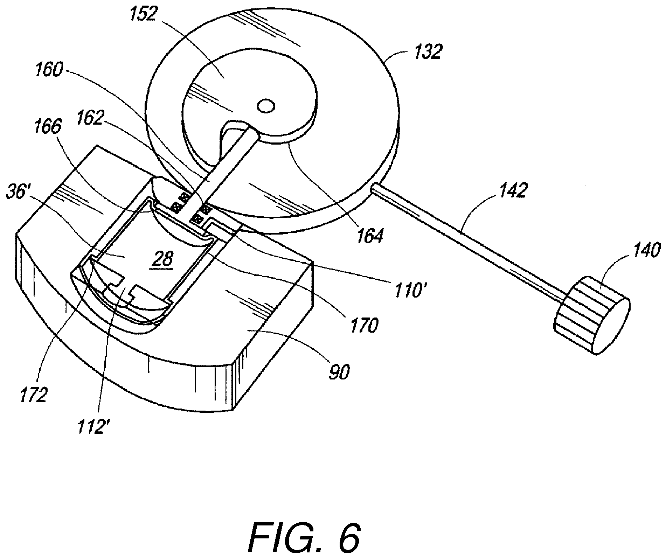

Referring now to **FIG. 6**, in an alternate embodiment, a cam 152 attached to the stem of a watch movement 132, connects to a fluid displacement device 90 via a piston shaft 160, mounted on sealed bearings 162 to axially translate, which is guided in its axial translation by a cam surface 164 thereof. The piston shaft 160 is connected to a piston head 166 which acts against a flexible rolling diaphragm 170 of a reservoir 36' (alternatively, of course the piston may have an O-ring mounted about its periphery or be otherwise sealed, as shown in the embodiment of **FIG. 3**) The rolling diaphragm 170 has a flange 172 which is sealingly fixed at one end so as to effectively separate a fluid 28 from below the piston head 166, from a fluid 28' (which may include air as a fluid gas) above the piston. The reservoir 36' is shown in an extreme position. A passageway 112' leads to the capillary channel 120, and a passageway 110' provides a return passage to the opposite side of the piston head 166.

The cam 152 is formed resembling a nautilus spiral so as to progressively move the piston shaft 160 and therefore the piston head 166 to displace a determined amount of fluid 28 into the capillary channel 120, at a rate which will indicate the time accurately. Of course, a similar determined amount of drug or perfume may be administered to living organism in this manner as well.

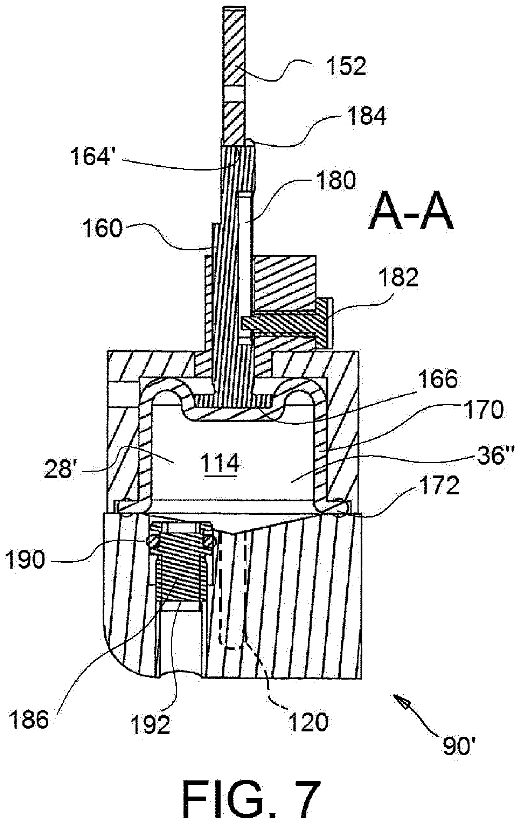

Referring now to **FIG. 7**, again, the alternate fluid displacement device 90 is shown in which the reservoir 36" is in an essentially filled position. A keyway 180 formed on the piston shaft 160 mates with a set screw 182 which screws into the keyway via threads in the fluid display subassembly 90', in order to prevent the piston shaft from rotating on its axis, thereby better maintaining the relationship between the extreme end 184 of the piston shaft and the cam surface 164'. In addition, an adjustment screw 186 having an O-ring seal 190 mounted in a recess therein includes an "ALLEN" or "TORX" interface in an exterior end 192 thereof which allows factory adjustment of the position of the meniscus 30 for calibration purposes. A septum or access port 194 (not shown) or pair thereof, made of an elastic material, may also be used to allow removal and injection of air and fluid 28' and 29' into and out of capillary channel 102 and/or reservoir 36".

It should be noted that the invention 10, 10', 10" may be made exclusive of all electronics (such as would typically be the case where the invention is positioned in the luxury watch market). In such embodiment, the power source 22" may be movement from an oscillating mass, which winds a watch spring, which powers a gear train, for which the rate of rotation is controlled by a pendulum-like regulator or oscillating disk (e.g., a balancier/turbion), which has a characteristic period, as known in the art.

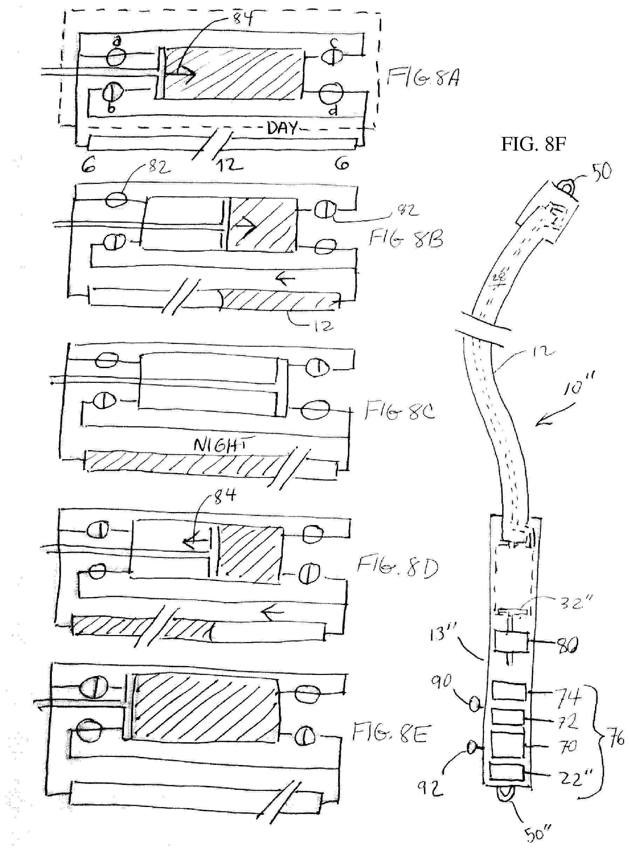

Referring now to **FIG. 8F**, in a further alternate embodiment, the device 10" may be made exclusive of all electronics, such as would typically be the case where the invention is positioned in the luxury watch market. In such embodiment, the power source 22" may be movement from an oscillating mass, which winds a watch spring 70, which powers a gear train 72, for which the rate of rotation is controlled by a pendulum-like regulator or oscillating disk 74 (e.g., a balancier/turbion), which has a characteristic period. The rotational motion created by the mechanism 76 is transformed into linear motion by the screw 80. This screw 80 drives the plunger 32" which drives a fluid 28 as shown in **FIGS. 8A** to **8E**, where valves 82 are opened or closed in order to effect the desired fluid movement in the reservoir 12. The arrows 84 show the direction of movement of the plunger 32". In **FIG. 8A**, the indicator reservoir 12 is empty. As the plunger 32 advances to the right, in the direction of the arrow, the fluid 28 in the indicator advances to the left. Note the lines and positions of the valves 82 that permit this desired fluid flow. **FIGs. 8B** and **8C** show the continued advancement of the fluid in the indicator to the left. **FIGs. 8D** and **8E** show advancement of air to the left, to show day.

In an embodiment without fluid, a threaded rod may be formed as a closed loop and having a surface of which (painted for example) which contrasts with the remaining loop, in order to indicate time on the scale device. A colored reed form, with divots cut at bend points may be actuated along the length of the reservoir so as to resemble a moving liquid.

The reservoir 12' may be made of a transparent or translucent material, or a mixture of transparent and translucent material, formed in any desired shape. It may be made of plastic, rubber, silicon.

In an alternate embodiment, instead of the position sensor 60, a conductive wire (not shown), made of conductive material such as metal, is exposed along at least a portion of its length to fluid in the reservoir 12', as described above.

The conductive wire is therefore in contact with any fluid in the reservoir. The wire may be calibrated using a variable electric resistance along its length as the fluid contacting the wire is pumped in the reservoir, and wherein the fluid is pumped until the electric resistance measured in the wire matches that which corresponds to the measured value, as calibrated. Calibration of the indicator 10 is performed by comparing variable resistance measures with locations along the length of the reservoir, the locations marked with a scale to indicate the corresponding measured value.

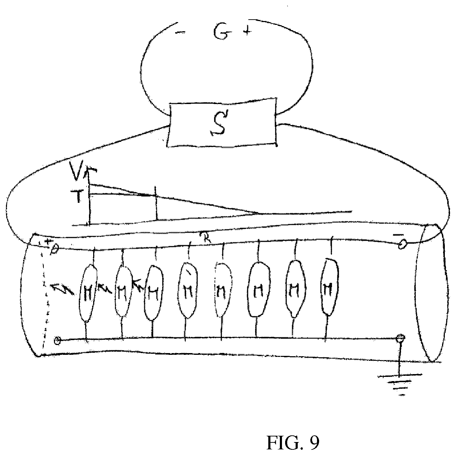

Referring now to **FIG. 9**, a textile application of the invention is shown. The goal of this application is to provide a device of the invention which can be sewn in material. A workable embodiment includes:

- a molecular chain or fluorescent micro LEDs are included in the reservoir;
- a reservoir made of an insulating material;
- module or micro LEDs placed along the length of the reservoir at a distance which permits placing at least 12 x 60 = 720, for the time piece embodiment;
- a connected at the source R and to ground is made;
- the LEDs emit light (fluorescence or phosphorescence, shiny glass type) when R attains a voltage of T;
- voltage R is provided by an electric power source S;
- the electric source S maintains a voltage level of R depending on the electrical resistance of R, but independent of the consumption of the molecules or florescent micro LEDs M;
- the florescent molecules M have an infinite resistance as long as the voltage applied is less than T and they become fluorescent as soon as a set voltage level is applied; and
- the voltage delivered by the source S to R varies as a function of the measured value G.

What remains flexible is the chain of LEDs, which light up and turn off together or via waves, but not for indicating a measured value. It may be as fine and flexible as a thread which may be integrated into a textile item (because it has a small diameter on the order of a millimeter), water resistant, washable, etc.

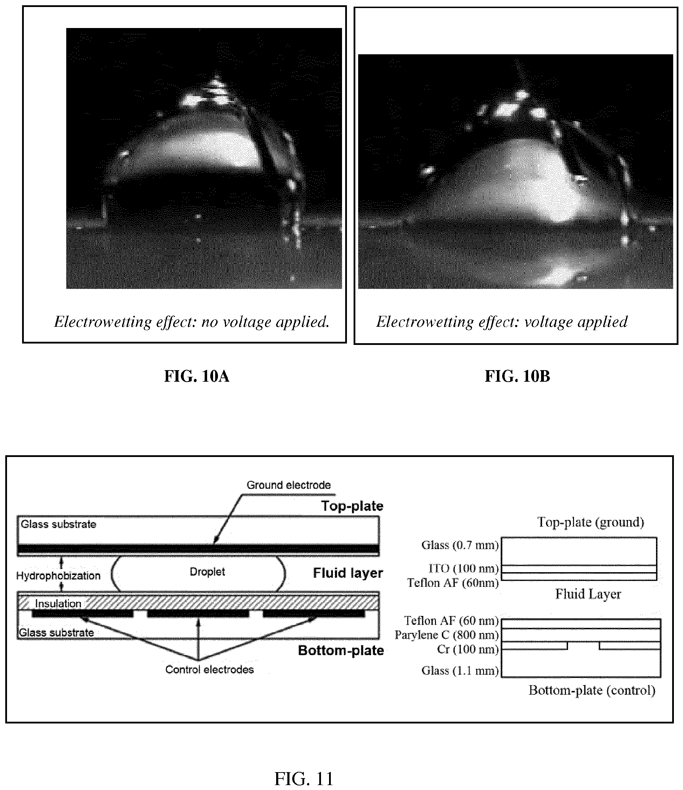

In another embodiment, fluid may be displaced within a display by a process called electrowetting. Electrowetting is a phenomenon where a normally hydrophobic surfaces loses its properties and becomes hydrophilic as represented in **FIG. 10A** and **FIG. 10B**. **FIG. 10A** shows the droplet without voltage applied to an electrode. **FIG. 10B** shows the droplet with voltage applied to an electrode.

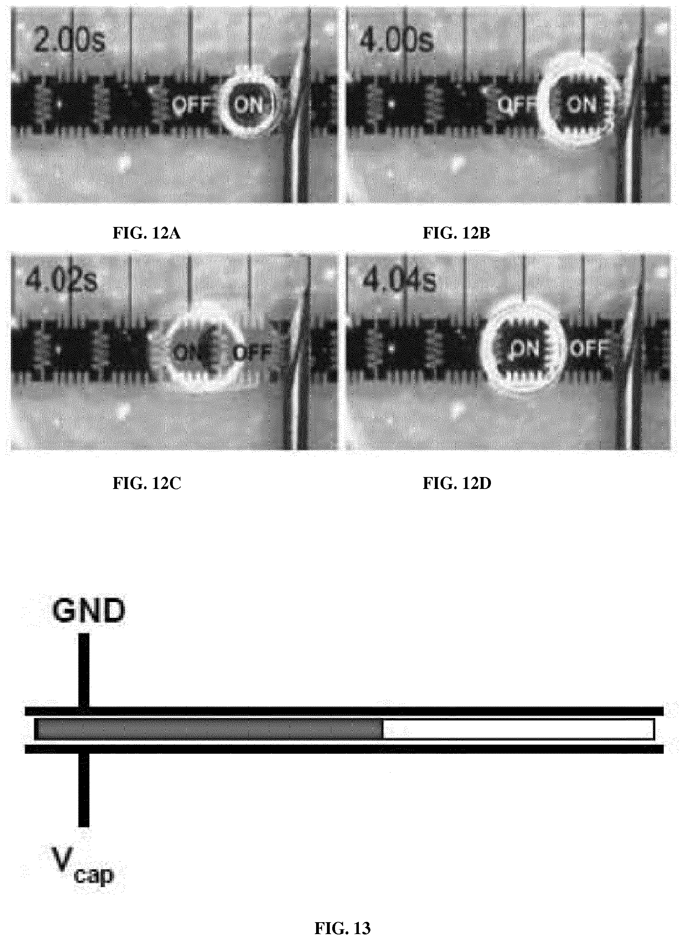

A schematic representation of an electrowetting display is shown in **FIG. 11** along with a detailed schematic of the different layers used to make the actuator. **FIG. 12A** - **FIG. 12D** show pictures from a test involving the displacement of a droplet of water in silicone oil with Electrode pitch: 1 [mm], height: 400 [$\mu$m].

Still further, two embodiments apply the electrowetting phenomenon using a capacitive sensor.

Referring to **FIG. 13**, in a first capacitive sensor embodiment, a single electrode is used, where the liquid level is inferred from the analogical value of capacitance measured across the whole display. This embodiment allows the use of a simpler electronic circuit. However, it is more difficult to calibrate given the influence of environmental parameters.

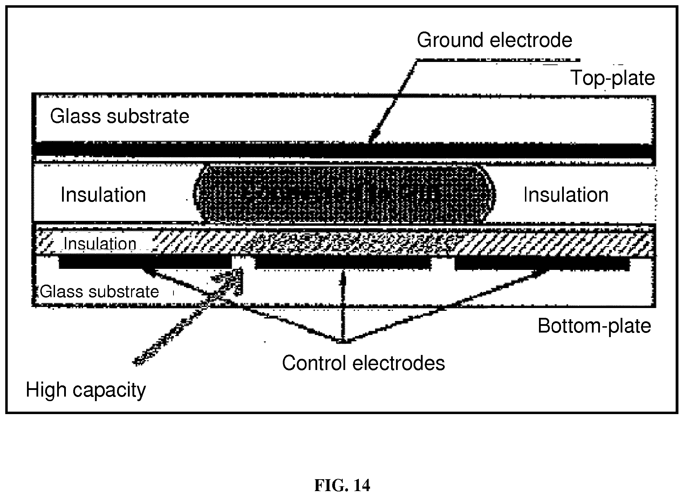

Referring to **FIG. 14**, in a second capacitive sensor embodiment, the liquid level is determined as a digital value, using for example, one hundred and forty-four electrodes, one for each time step.

The above solution is extremely robust, not being influenced by environmental parameters as in the first capacitive sensor embodiment. The device shall fulfil the general watch requirements ISO 764, ISO 1413 and ISO 2281.

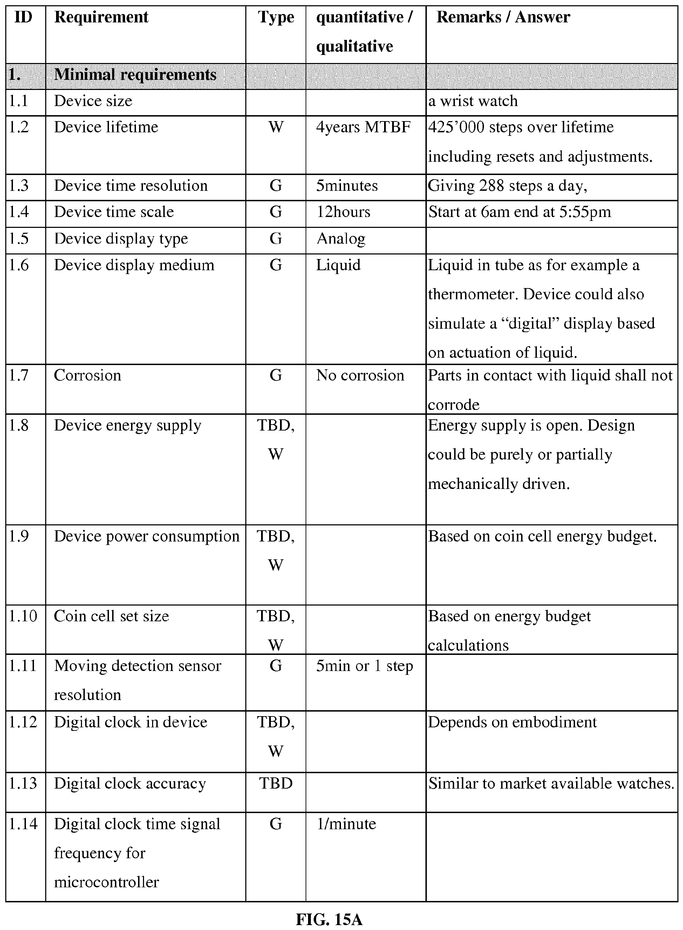

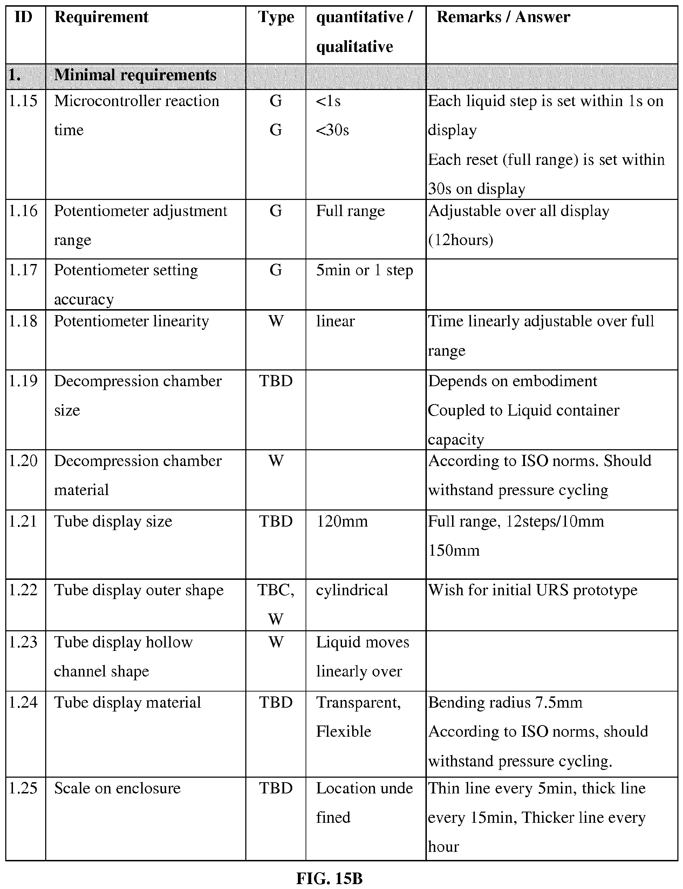

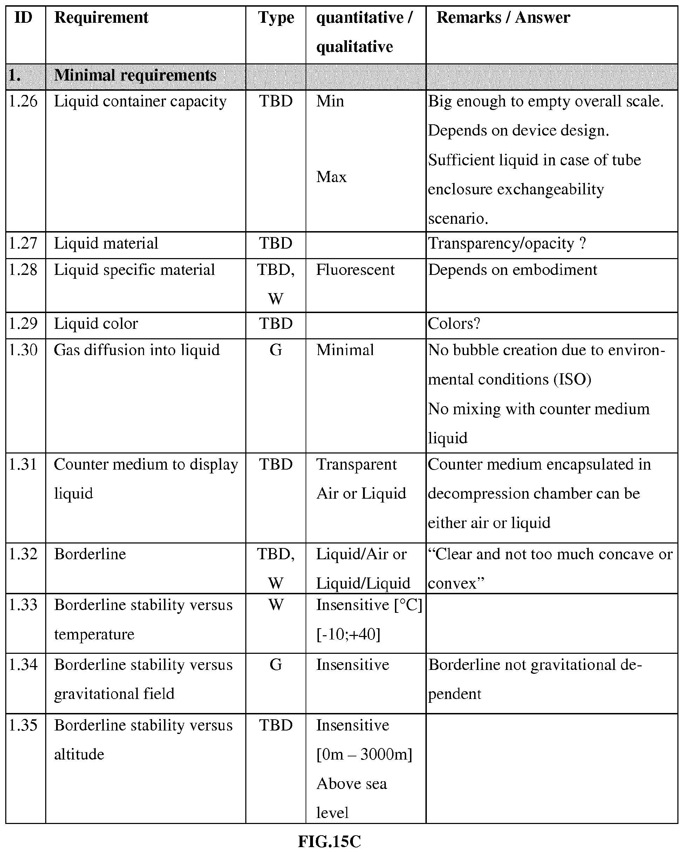

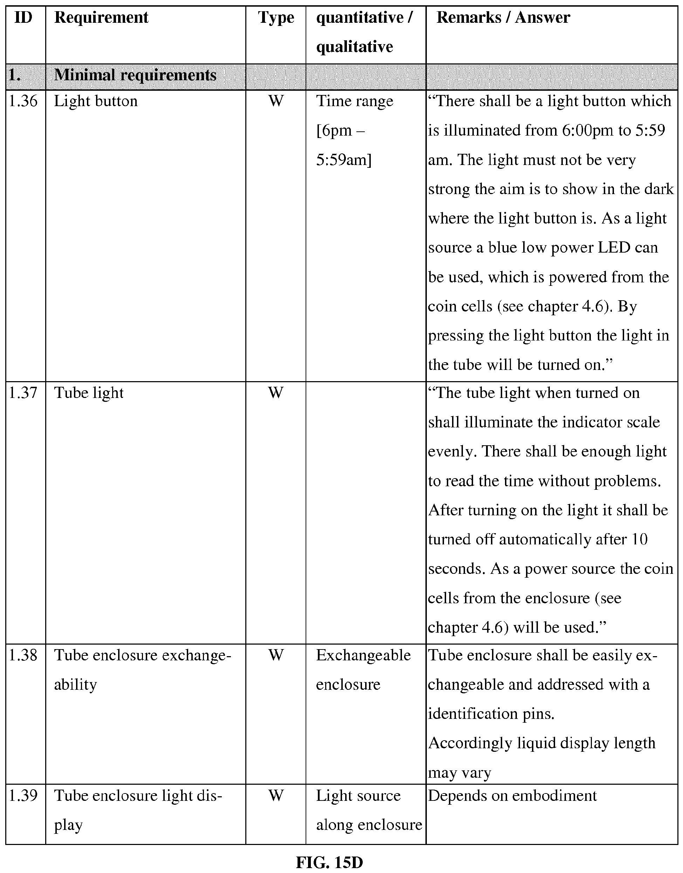

**FIG. 15A** to **FIG. 15D** are tables showing considerations of requirements of elements of the invention.

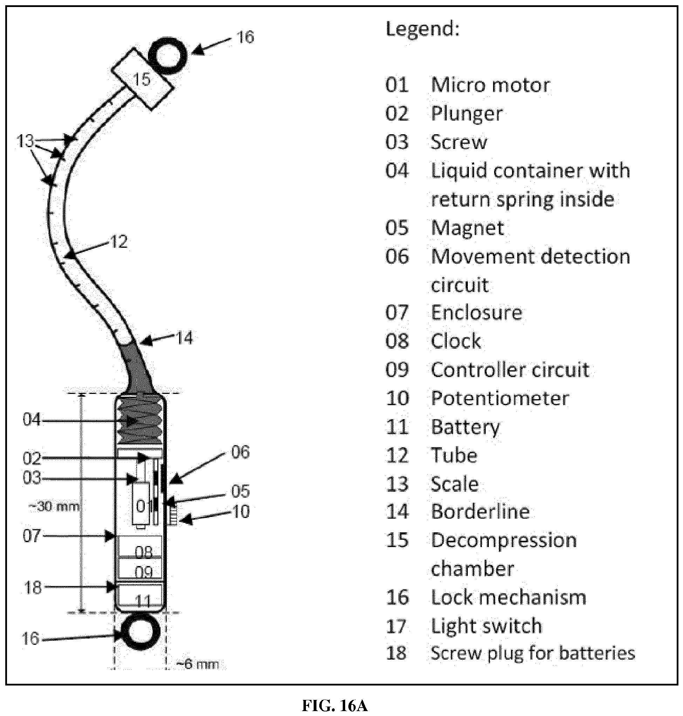

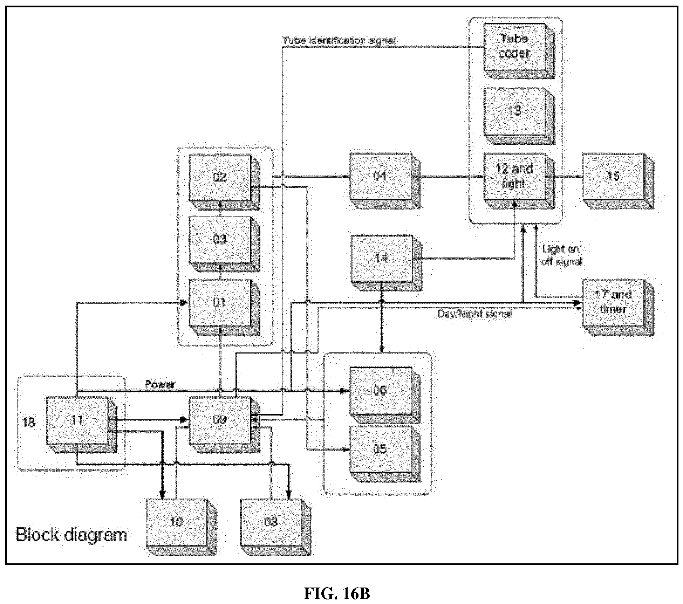

**FIG. 16A** shows a Prototype as after URS (cf. **FIG. 3**) and **FIG. 16B** shows a related Black Box diagram.

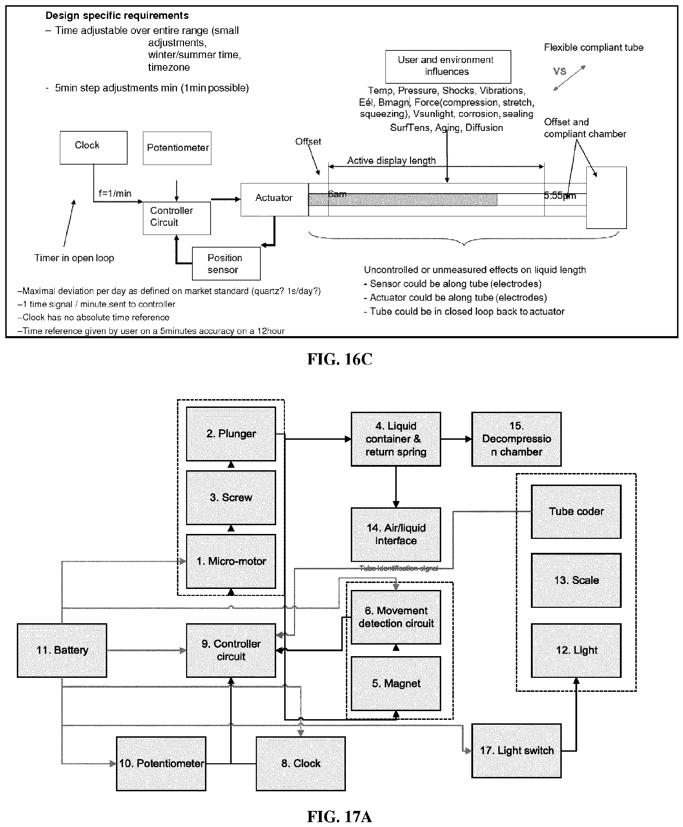

**FIG. 16C** shows Design specific requirements of the invention for Phase I.

The original block diagram of the project is presented in **FIG. 17A**. Some of its parts are oriented specifically towards the application of the Squiggle drive.

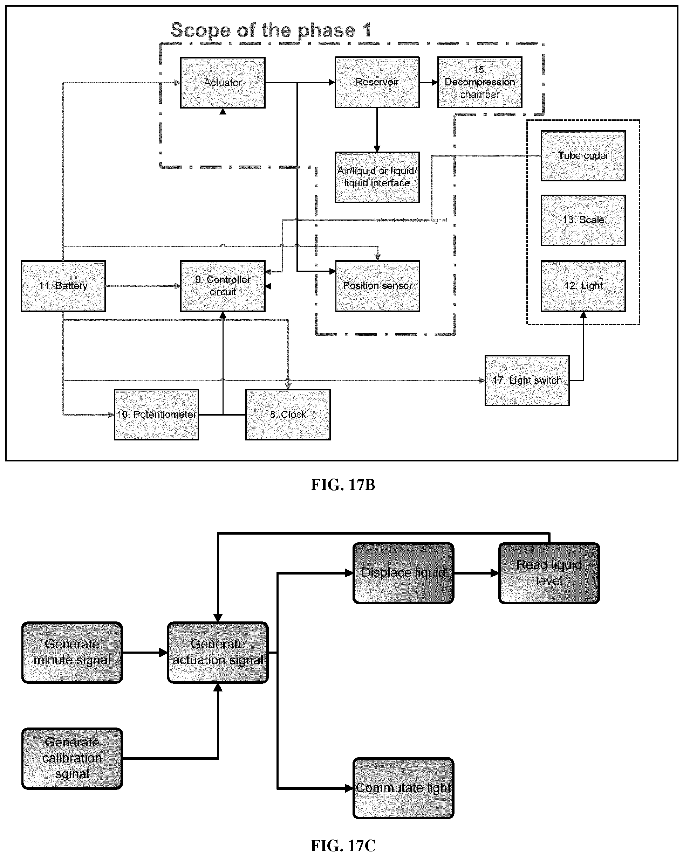

The generalized block diagram is presented in **FIG. 17B**. In the same figure, the scope of the first phase of this project is outlined. The goal is to develop the actuator with its direct dependencies, which are the reservoir and the decompression chamber.

As the sensor plays a major role in the design and the control of the actuator, it is also in the scope of this first phase.

A succinct function analysis of the device is presented in **FIG. 17C**. In this figure, the functions in blue will be treated in the first phase of the project.

In this section, solutions for the phases interface, displacement of the liquid and detection of the liquid position functions will be treated:

The phases interface is not a function, strictly speaking. Nevertheless, as it has a major impact on the design of the actuator.

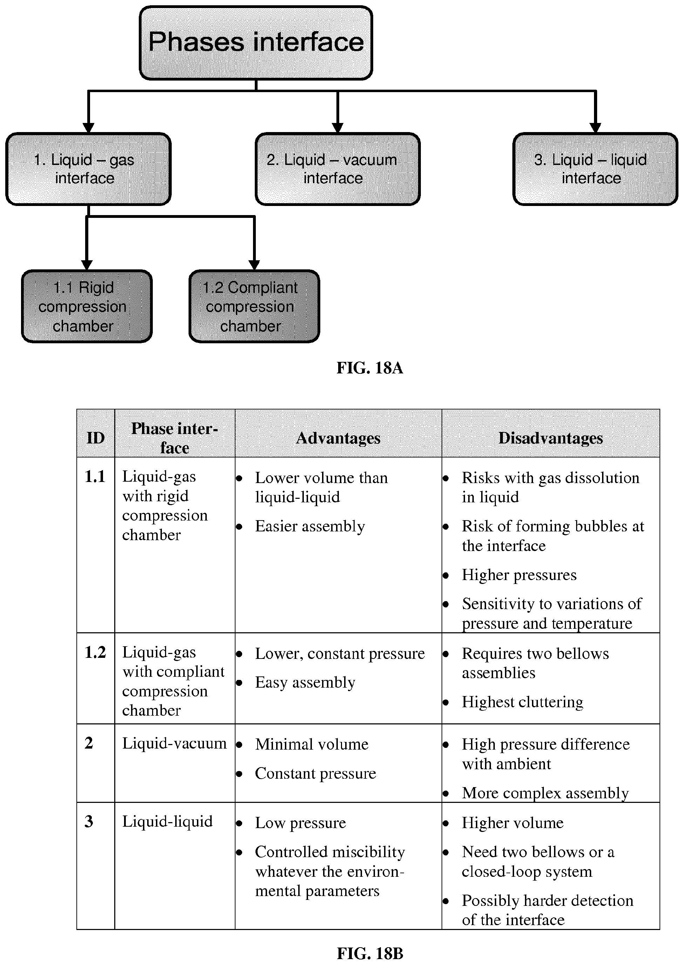

The tree of solutions for the phases interface is presented in **FIG. 18A.**

These solutions are discussed in **FIG. 18B.**

**Here we describe the liquids for liquid-vacuum (liquid-vapor) phase interface.**

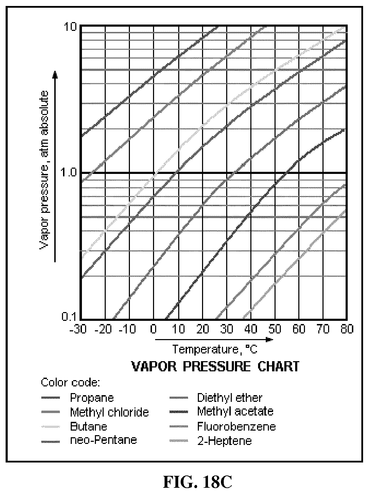

The so-called liquid-vacuum phases interface would in fact be a liquid-vapor interface, the "empty" space being instead filled with vaporized liquid, at its vapor pressure. The vapor pressure as a function of the temperature, for different liquids, is presented in **FIG. 18C**. It is clear that this value has a large variation with respect to the temperature. For instance:

- In order to have a positive pressure at -10 [°C], with methyl chloride, the pressure would reach 8 [bar] at 40 [°C]
- The pressure of propane would jump to even higher levels

This means that the actuator would have to be dimensioned for the pressure it would face of 40 [°C]. It would therefore be over-dimensioned over most of its operational range, and a risk of failure would exist should the device be temporarily heated to superior temperatures.

We conclude that:

For these reasons, the liquid-vapor pressure should be avoided.

Out of the two remaining interfaces, the liquid-liquid interface is preferable, as:

The liquid has a lesser sensitivity to dilatation, the risk of making bubbles in the case of a shock is reduced, the advance of the meniscus is more regular, and in case of rapid changes of temperature and pressure, bubbles risk to be formed in a liquid-gas interface.

**Here we describe the solutions for the displacement of the liquids:**

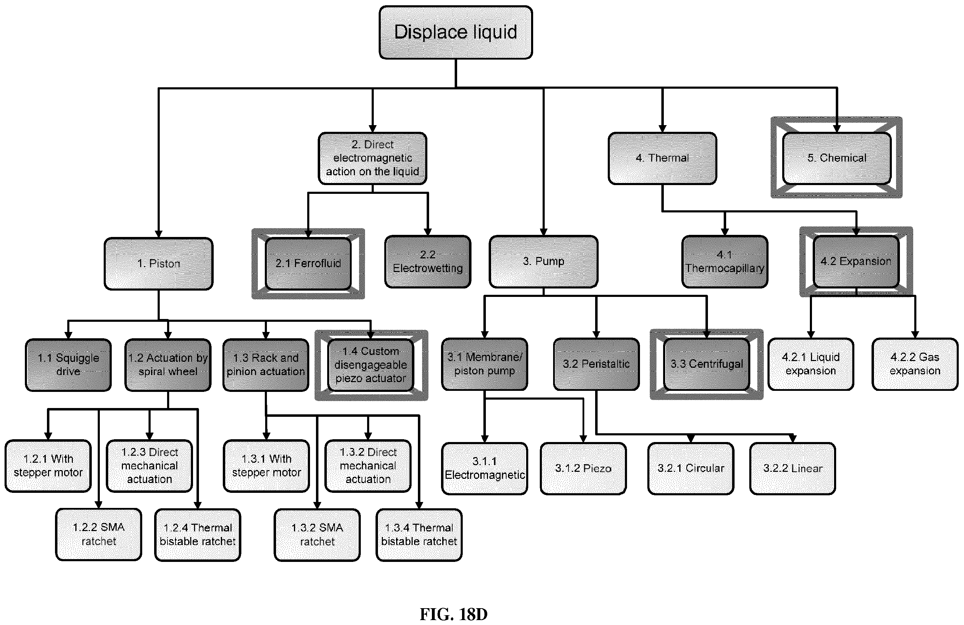

The tree of solutions for the displacement of the liquid is presented in **FIG. 18D**. The solutions are clustered in five main categories:

1. **Piston systems:** where a piston compresses liquid contained in a bellows reservoir
2. **Direct electromagnetic action on the liquid:** an electromagnetic action on the fluid itself moves it, without an actuator
3. **Pump systems:** liquid from a bellows reservoir is pumped into the display tube
4. **Thermal systems:** a thermal effect induces the displacement of the liquid
5. **Chemical:** the liquid is displaced by a chemical reaction

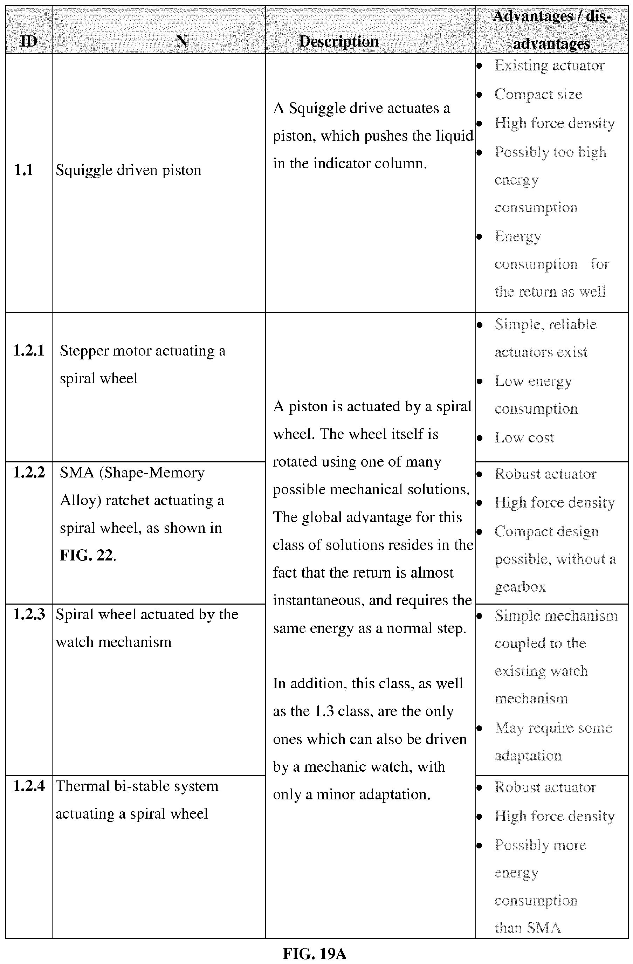

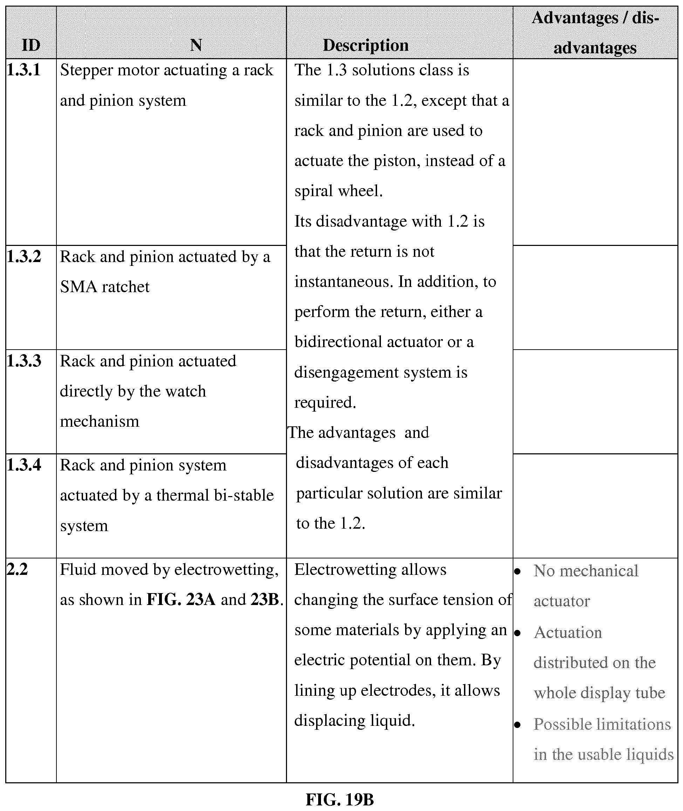

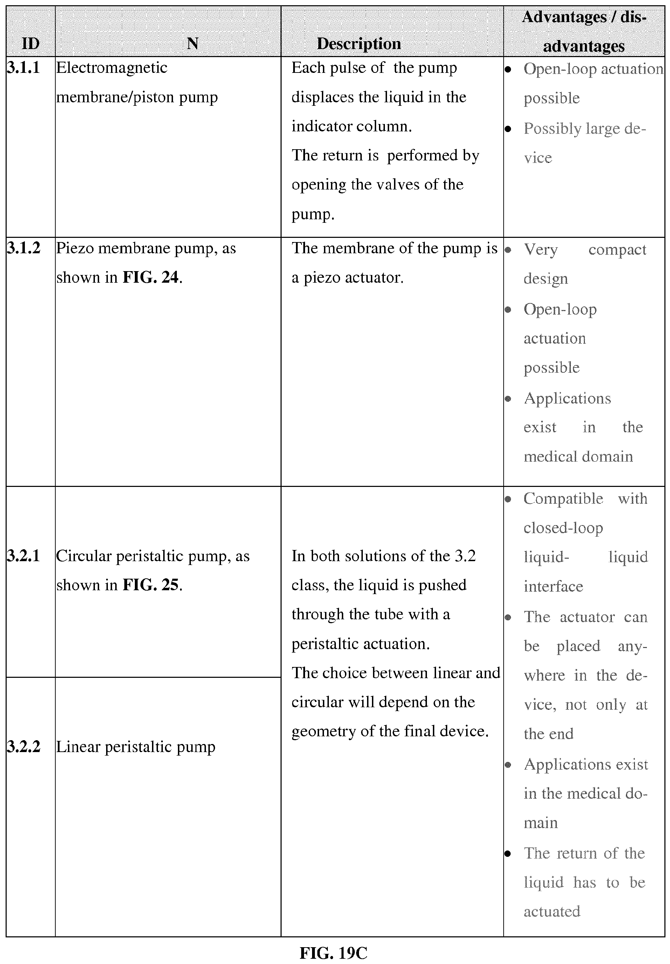

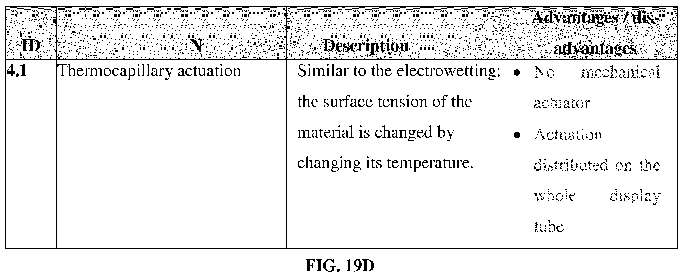

The categories 1 to 4 are discussed in **FIG. 19A** to **19D.**

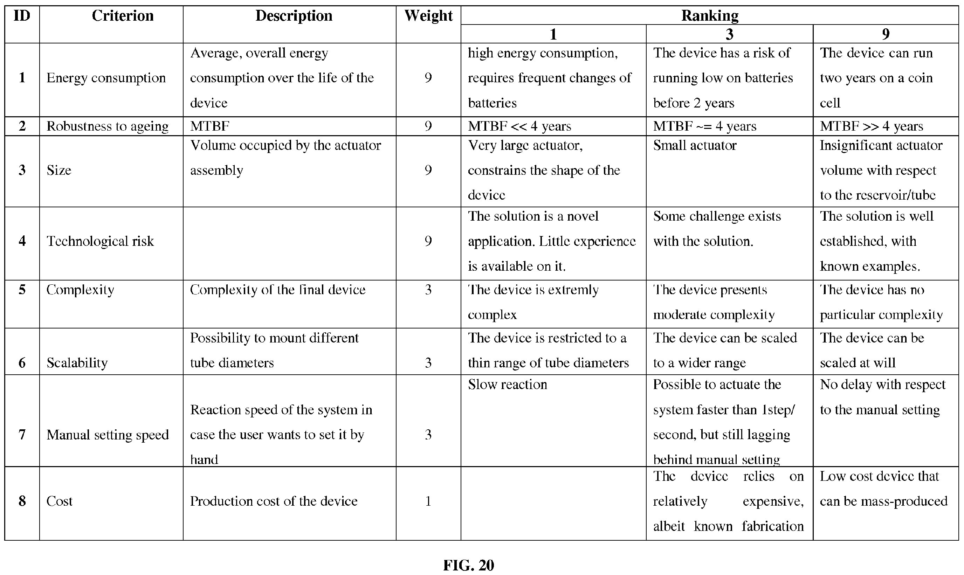

The evaluation criteria for the liquid displacement systems are shown in **FIG. 20.**

The ranking criteria are presented in **FIG. 20**. The ranking is done using the 1-3-9 method in which every solution is assigned a grade of 1, 3 or 9 for each considered ranking criterion. The ranking criteria themselves have a weight, also 1, 3 or 9. This way, any contribution can bring a value between 1 and 81 to the total grade of the solution.

The robustness to the environmental parameters is not displayed here, as it will be defined by a conjunction of the actuation, the type of interface, and the sensing.

Following criteria were a priori given weightings below the maximal of 9:

**The complexity:** due to the anticipated high-end segment to which the product is designed for, the complexity is not considered to be a criterion of the utmost importance.

**The scalability:** the product is for the moment for watch displays. Although possible further applications could require scaling to other dimensions, it is not for the moment a key criterion.

**The manual setting speed:** Some solutions do not allow to set the display manually at any speed. This might prove problematic as the user would not have an immediate feedback on his action on the display. This criterion is given a weighting of 3 for the moment, but could have to be increased.

**The cost:** once again, due to the high-end segment for which the product is designed, the cost does not appear to be a criterion of the highest importance. A highly costly and complex device may even attract interest of the watch customers.

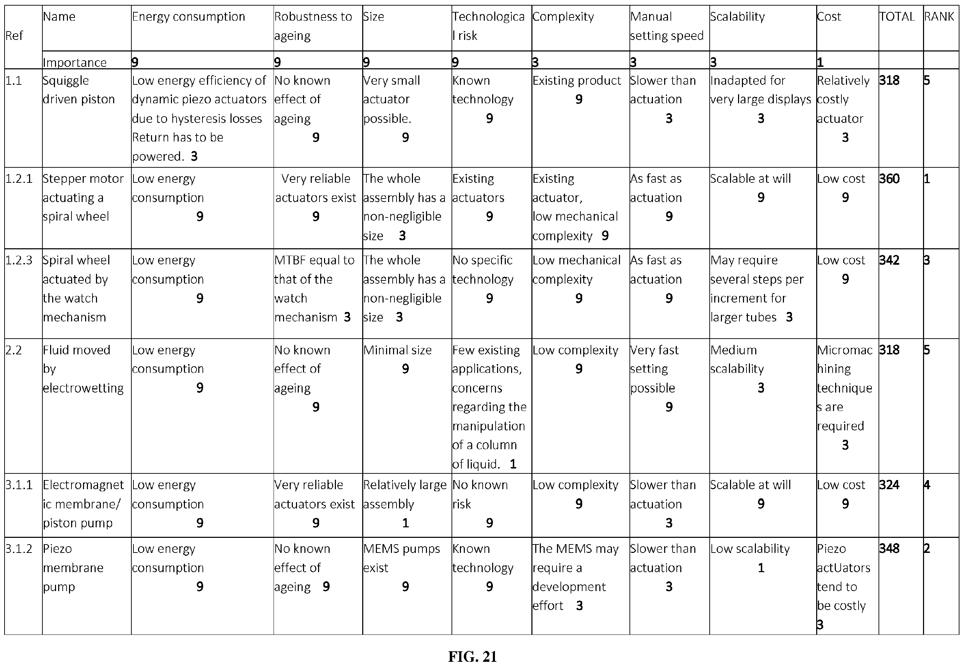

The ranking of all the considered solutions, with the aforementioned ranking criteria, is shown in **FIG. 21**.
The five leading solutions are:

**1. The stepper motor actuating a spiral wheel** comes first in this ranking. It is a very simple solution, relying on a relatively simple mechanism and known actuators. In addition, the manual setting of the indicator can be done very quickly, using a mechanical clutch to disengage the spiral wheel from its gear train. It is only handicapped by its relatively larger size.

**2. The piezo membrane pump** is second. It has a good ranking due to its low size, robust design and known technology. It is handicapped by a relatively low scalability, possibly higher cost than some other solutions.

**3. The spiral wheel actuated by the watch mechanism** is third. Note that this solution is displayed indicatively, and will not be pursued here, as it is not the objective of the first phase of the project to develop such a solution. It is however to be noted that the winning solution can also be easily converted in a fully mechanical display.

**4. The electromagnetic membrane/piston pump** is in fourth position. It has the advantages of the piezo membrane pump, at the cost of a higher size.

**5. The electrowetting** is in fifth position. This solution is highly seductive for its total lack of mechanical actuation, the possibility of using the same displacement electrodes for a full closed-loop regulation, and compact size. It also allows a very rapid manual setting.

**5. The Squiggle driven piston drive** is tied for the fifth position. This solution is handicapped by a higher energy consumption, due to its high-frequency piezo actuators and to the necessity to power the return. In addition, such piezo actuators tend to be costly, and it is not fully scalable. Finally, unless a second actuator is implemented the manual setting is bound to be slow with this method.

**The leading solutions are presented in details in the following.**

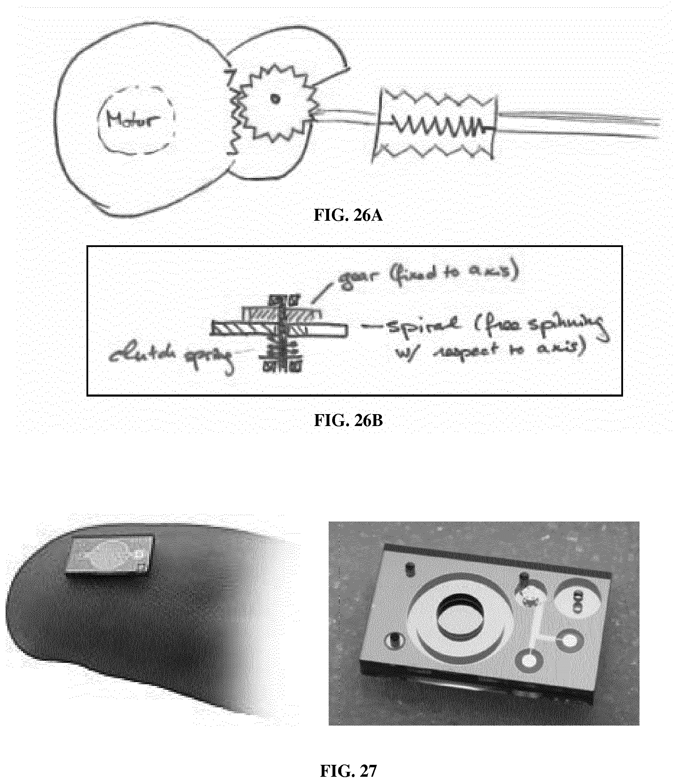

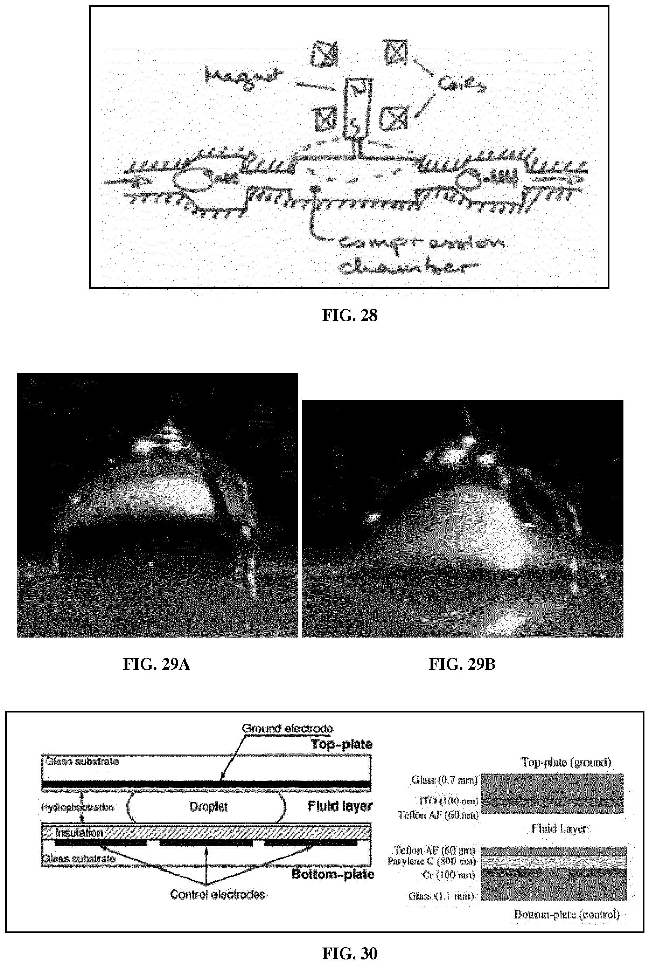

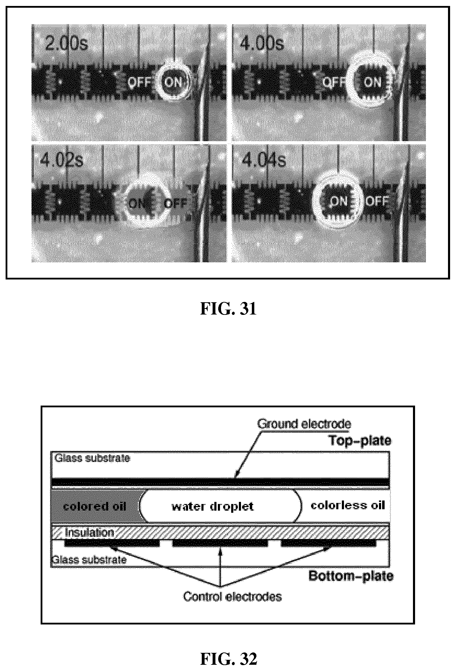

Here we describe the stepper motor actuating a spiral wheel. A schematic representation of this solution is presented in **FIG. 26A** (a top view), as well as in **FIG. 26B** (a side view) presenting a solution to do the setting quickly. In order to do the setting, the user manipulated button would disengage the spiral wheel from its gear train, and allow an unpowered and quick setting. All the components are simple and well-known, including the stepper motor.
Here we describe the piezo membrane pump. In **FIG. 27** is presented the Nanopump/piezo membrane pump, a device designed by Debiotech for insulin infusion purposes. This particular device has a 200 [nl] dispense per pulse. It is entirely micro- machined on Silicon On Insulator (SOI) wafers, which grants a high repeatability.
In addition, as the device is self-priming, it would allow for open-loop regulation: at the end of one 12 hours cycle, the liquid can be pulled back in the reservoir by opening the return valves. Then, the pump can be activated until the liquid is detected by a single capacitive sensor placed on its outlet. After this point, the pump can be trusted to provide regular steps during the next 12 hours period.
Note that the capacitive sensor could theoretically be integrated in the device.
Some devices as the Nanopump exist on the market, or are in development.
Here we describe the electromagnetic membrane/piston pump. A schematic of such an electromagnetic membrane/piston pump device is presented in **FIG. 28**, for the case of a membrane pump. It is noteworthy that the piston configuration is also implementable. However, both solutions are for the moment considered together, as the function of both devices is massively similar.
In both cases, the volume of a compression chamber is varied, and two check valves ensure that the flow generated by this variation goes in the desired direction.
One of the main advantages of such pumps is that they generate a volumetric flow; the advance of the liquid in the indicator could therefore be controlled by an open-loop system, provided that the system is recalibrated after each 12 hours cycle.
Here we describe the electrowetting. The electrowetting is a phenomenon where a normally hydrophobic surface loses its properties and becomes hydrophilic. This is presented in **FIG. 29A** and **29B.** This way, with several electrodes lined up, it is possible to control the displacement of a droplet of water in a display.
A schematic of such a display is presented in **FIG. 30**, as well as a detailed schematic of the different layers used to make the actuator. Using a droplet slightly larger than the electrode, the droplet moves to the adjoining electrode when activated.
Pictures from a test involving the displacement of a droplet of water in silicone oil are presented in **FIG. 31.** It is visible that the displacement is extremely quick. In addition, the power involved is very low as the electrodes act as capacitors: no conduction of current takes place in the system.

Most of the published work, as yet, involves the displacement of droplets of water, and not of bulk, as would be required to displace a column of liquid in the case of the liquid display. However, the display behavior can also be achieved by the displacement of a single droplet, such as presented in **FIG. 32**, in which a water droplet moves a colored and colorless oil adjacent thereto in the display. The droplet in this case is used to make the separation between a colored and a colorless oil, the colored oil being the indication medium.

This data intends to display some of the so far demonstrated capabilities of the electrowetting. This technology promises to yield a very rapid and low-consumption device.

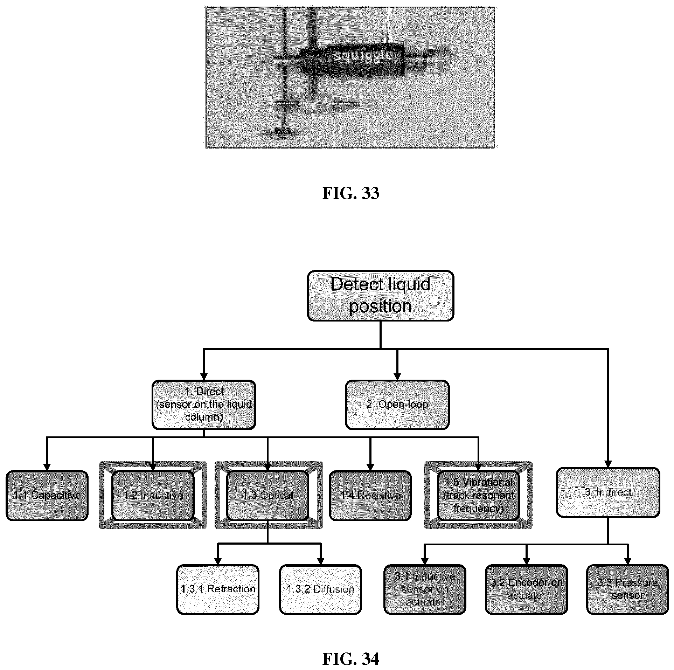

Here we describe the squiggle driven piston. **FIG. 33** shows the Squiggle driven piston variant. This solution relies on an existing product, such an actuator could be adapted to a spiral wheel system, for instance.

Here we describe the detect liquid position solutions. The tree of solutions for the detection of the liquid position is presented in **FIG. 34**. The three large groups are first - the **'direct sensing'**, where the sensor is integrated on the indicator tube, and detects directly the position of the liquid; second - the **'open-loop'**, where no sensor is used and the system is reset every twelve hours in order to prevent accumulation of errors; and third - the **'indirect sensing'**, where the position of the actuator is tracked, and the position of the liquid column is inferred. In addition, it is to be noted that a compensation for the temperature may have to be done if an indirect sensor is used with a liquid-gas interface.

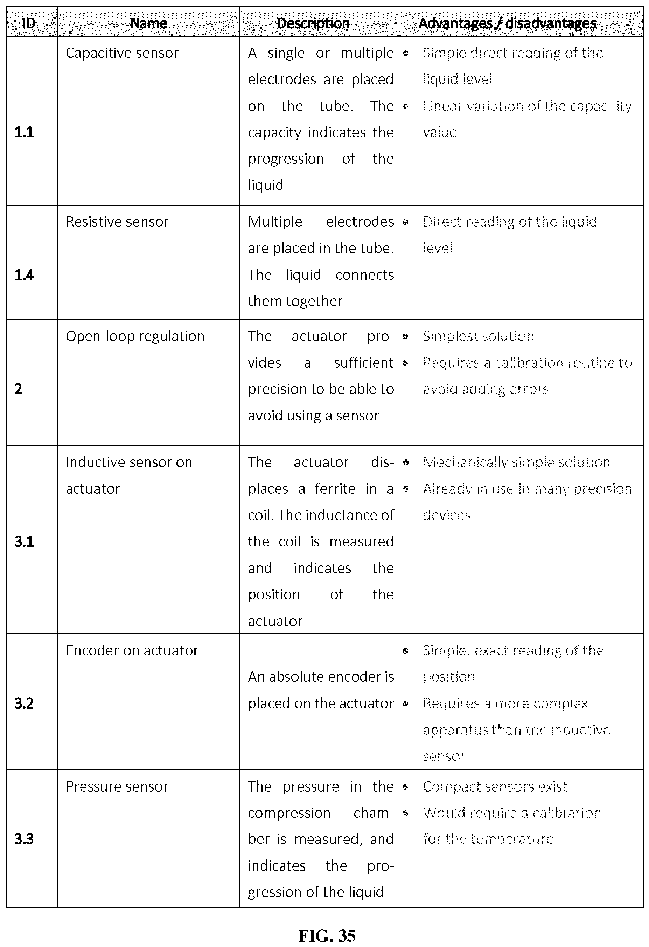

Solutions for the detection of the liquid position are discussed in **FIG. 35.**

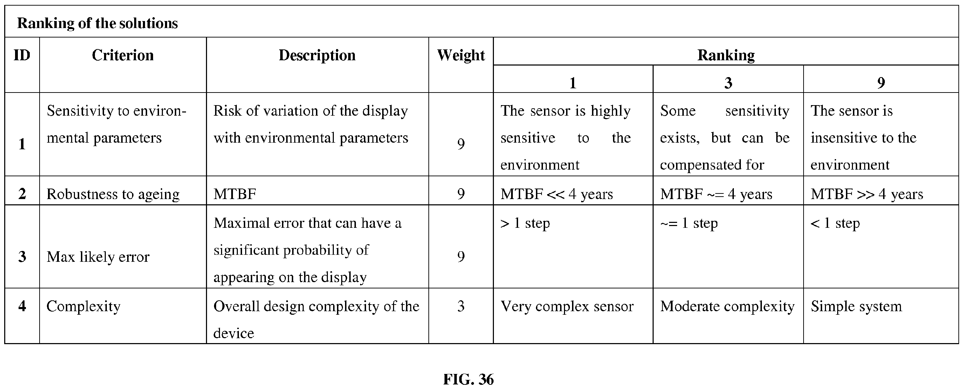

Evaluation criteria for the liquid sensing methods are discussed in **FIG. 36.**

A sensitivity to environmental parameters is specified only if the sensing method is inherently sensitive, with no possibility of avoid this sensitivity by selecting an appropriate interface, for instance.

For all the considered indirect sensors, as well as for the open-loop regulation, it is considered that the actuator that displaces the liquid is volumetric, i.e. that certain position of the actuator corresponds to a position of the liquid column. This is taken as assumption as no pressure generators made it past the selection of the actuators.

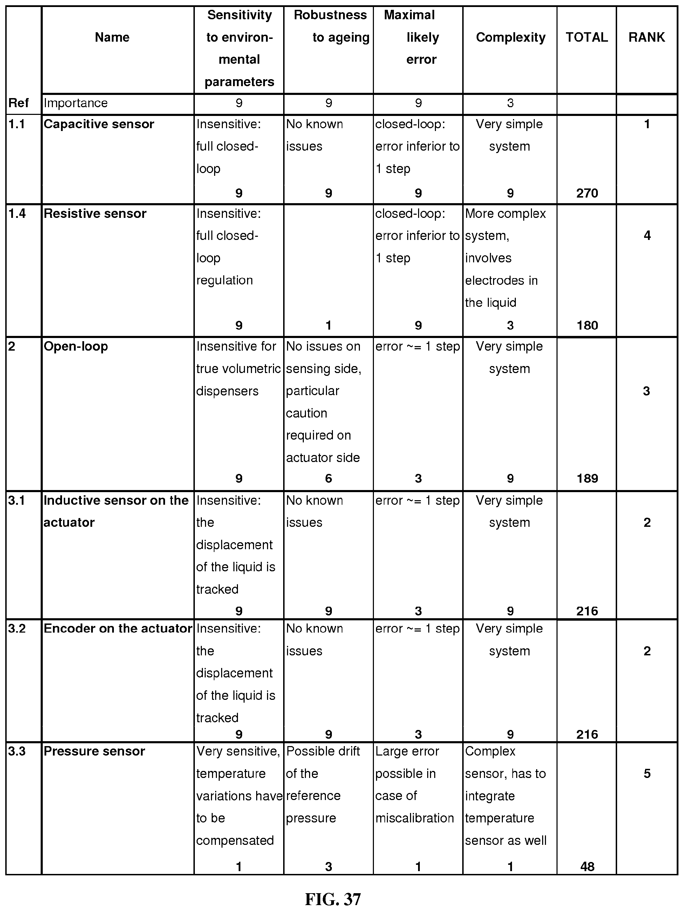

The ranking of the selected solutions of the liquid level sensors is presented in **FIG. 37.**

The results are the following:

- The capacitive sensor is the preferred solution, as it allows for a reliable closed-loop control of the position of the liquid column, while relying on a relatively simple technology.

- The indirect sensing methods come in second position. Both are simple, but may lead to slightly higher errors, as no closed-loop regulation is done.

- The open-loop regulation comes in third position. It may present an error, and particular caution has to be taken so that the dispense per step of the actuator does not change with the time. However, its simplicity is a great advantage.

These three first solution groups will be presented in detail in the next section. The resistive sensor will not, as it has similar performances, while it has a significantly more complex design.

**Detailed presentation of the leading solutions**

Here we describe the capacitive sensor. Two implementations of the capacitive sensor are possible. One implementation solution is a single-electrode sensor, where the liquid level is inferred from the analogical value of capacity measured across the whole tube. Another implementation solution is a multi-electrode sensor, where the liquid level is determined as a digital value, using 144 electrodes, for all the time steps.

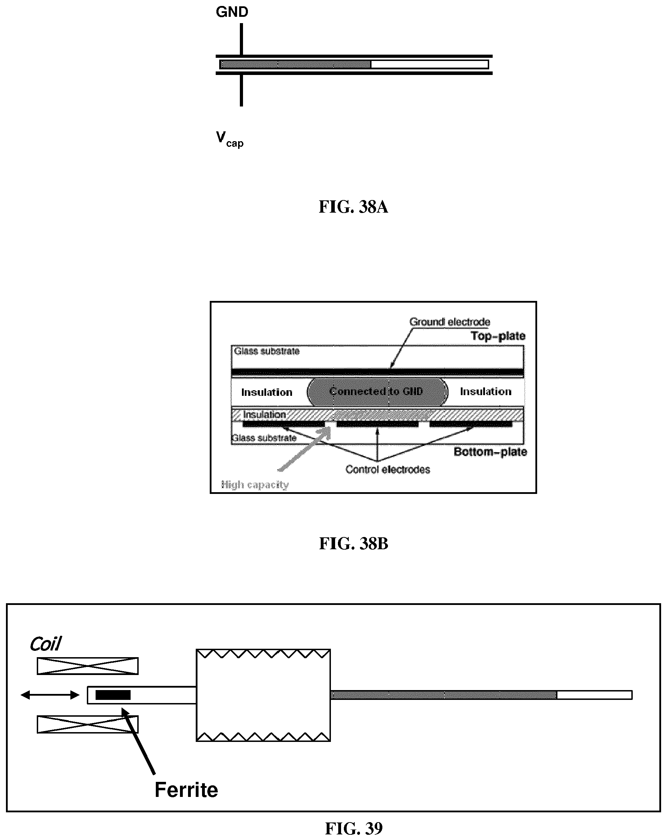

The first solution would allow using a simpler electronics circuit, but might prove challenging to calibrate due to the sensitivity of the analog circuit to the environmental parameters. The second, however, would be an extremely robust solution. Both solutions are presented in **FIG. 38A** and **FIG. 38B.**

The robustness of the second implementation, as well as its compatibility with the electrowetting solution, makes it a preferred one.

Here we describe the inductive sensor on the actuator. The inductive sensor placed on the actuator measures the position of a ferrite in a coil, by measuring the inductance of this coil. It is presented schematically in **FIG. 39.** Such sensors are already widely used and provide very reliable results.

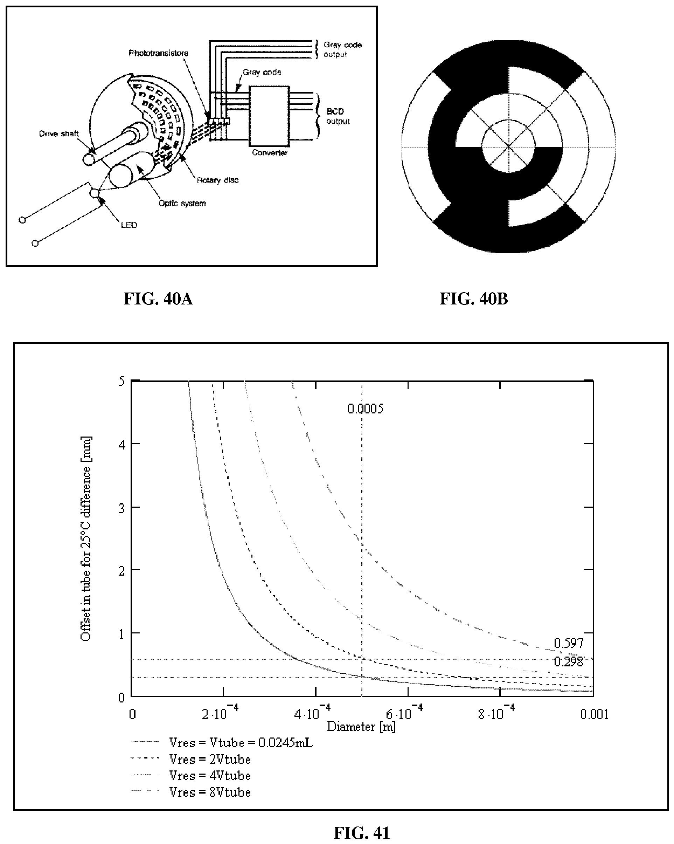

Here we describe the encoder on the actuator. An encoder is a simple system that provides the absolute position, or the displacement, of a rotating actuator. A schematic of such a system, as well as an encoder wheel for an absolute positioning, are presented in **FIG. 40A** and **FIG. 40B** respectively. This system can be realised with virtually any desired accuracy, depending on the application.

The encoder and the inductive sensor have similar performances. The former is more adapted to rotating applications, and the latter to linear translation. The main direction of displacement of the actuator should be the rationale for the discrimination between those two sensors.

Here we describe the thermal expansion calculation, in particular the thermal expansion of materials. Ambient temperature is an external parameter that directly acts on the system and on liquid in the display tube and therefore on its accuracy for time display. Its effect is increased for a bigger reservoir volume attached to a small display capillary. Parts such as liquid container, display tube and the liquid itself must be considered along with the 2nd liquid container for a liquid-liquid scenario. Applicable temperature range: °C [-10; +40].

Typical thermal linear expansion coefficients of materials and liquids $\alpha$ [$K^{-1}$]

- Invar: $2 \times 10^{-6}$

- Glass: $10\text{-}70 \times 10^{-6}$

- PMMA, PC: $50\text{-}100 \times 10^{-6}$

- PUR: $50\text{-}80 \times 10^{-6}$

- PP: $100\text{-}150 \times 10^{-6}$

- LDPE: $280 \times 10^{-6}$

- PVC: $60 \times 10^{-6}$

Typical volume expansion coefficients of liquids $\gamma$ [$K^{-1}$]

- Quicksilver: $180 \times 10^{-6}$

- Water: $207 \times 10^{-6}$ at 20°C (anomalous expansion)

- Ethanol: $750 \times 10^{-6}$

- Ether: $1700 \times 10^{-6}$

- Glycerol $500 \times 10^{-6}$

- Gasoline: $900 \times 10^{-6}$

- Silicone Oil: $1170 \times 10^{-6}$

Liquids volume expansion coefficient is more or less 3 times greater than $\alpha$, however water for example is highly non-linear.

Matching of materials and liquids will be defined later depending on the selected design embodiments. Criteria such as viscosity (versus a pumping device), surface tension, miscibility, freezing temperature and stability over the indicated temperature range are considered.

Calculations will show effect of a liquid with a $\gamma$ coefficient of $500 \times 10^{-6}$ [$K^{-1}$] a reservoir in PP ($\alpha$ $125 \times 10^{-6}$ [$K^{-1}$] (or $3 \times \alpha = 375 \times 10^{-6}$ [$K^{-1}$])) and display tube in PVC ($60 \times 10^{-6}$ [$K^{-1}$]). Mismatch is of about $125 \times 10^{-6}$ [$K^{-1}$].

Here we describe the calculations for a PP reservoir. The graph in **FIG. 41** shows liquid increase length in indicator tube for a 25°C temperature change. Temperature applied to the whole system. Reservoir material PP, Tube material PVC, Liquid with volume dilatation coefficient of $500 \times 10^{-6}$ [$K^{-1}$].

Vtube is the maximal liquid volume in display tube (length 120m, diameter 0.5mm giving 0.024mL).

Curves confirm that for a relative bigger reservoir volume, temperature coefficients mismatch between casing and liquid, induces a bigger inaccuracy. Effect is widely increased for a capillary display tube.

Reservoir volume is linearly scaled to the tube volume. If tube diameter is big, reservoir is scaled up to match volume. Therefore, offset in tube due to temperature does not depend on tube diameter. Following equation expresses the offset length versus a reservoir volume de- pending on display volume. P is the parameter starting from 1 (minimum liquid volume for display tube) to 5 (Reservoir contains up to 5 times the display volume) and Ltube: 120mm.

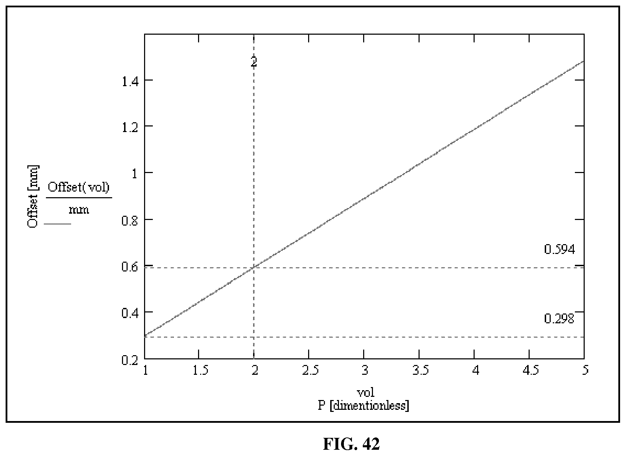

And curves are displayed on the graph in **FIG. 42.**

$$\text{Offset} = \frac{\Delta V}{\text{Tube\_area}} = \frac{\text{P} \cdot \text{Tube\_area} \cdot \text{L}_{tube} \cdot (1 + \Gamma \Delta T)}{\text{Tube\_area}} = \frac{\text{P} \cdot \text{L}_{tube} \left[ (1 + \alpha pp \cdot \Delta T)^3 - (1 + \gamma liq \cdot \Delta T) \right]}{(1 + \alpha pvc \cdot \Delta T)^2}$$

As liquids and solids are considered as incompressible, gases are compressed following the ideal gas law.

We conclude that:

• offset in display due to temperature is linear to the Volume and corresponding channel diameter;

• volume must be minimized while tube diameter must be maximized, ideally, liquid volume matches required display volume (120mm long channel and reading comfort);

• a compliant chamber is required in case of liquid/air (linear channel) or a double liquid/liquid interface (close-looped channel); and

• reservoir's material thermal expansion coefficient could match with liquid's thermal expansion coefficient.

Here we describe the pressure calculations. In the case of a display with a liquid/gas interface, and a rigid decompression chamber, the pressure will augment linearly while the liquid advances, as the gas gets compressed in the compression chamber. The final pressure will depend on two parameters:

• The section of the tube, that defines the amount of gas that has to be compressed

• The volume of the decompression chamber

The final pressure can therefore be calculated as:

$$P_{final} = P_{initial} \cdot \frac{V_{chamber} + V_{tube}}{V_{tube}}$$

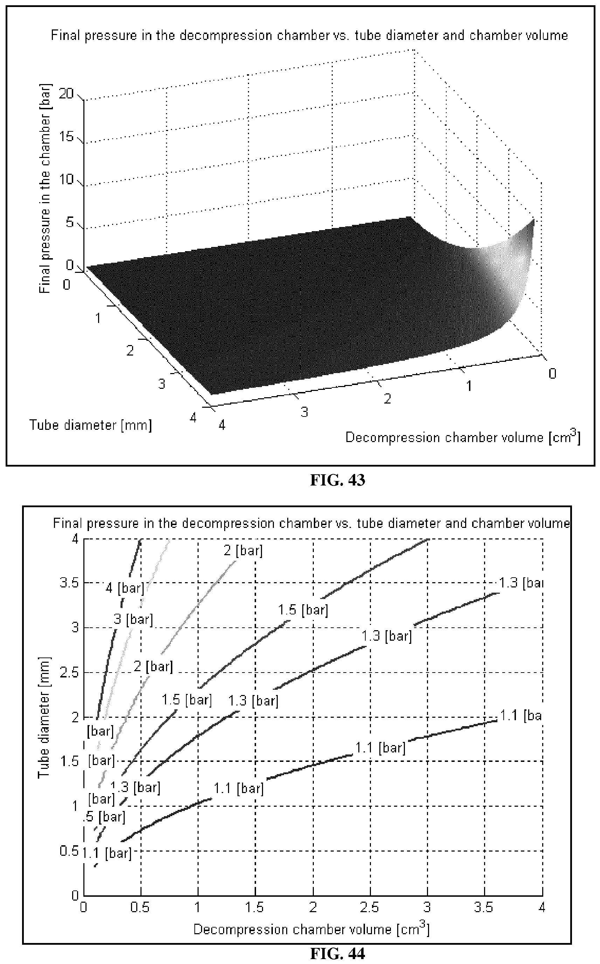

The final pressure in the compression chamber as a function of these parameters is presented in **FIG. 43.** The same values are represented as a contour plot in **FIG. 44.**

As it is visible in these figures, large pressures can easily be reached. This would both lead to higher energy consumption in the actuator, and higher mechanical requirements for the indicator. Actions that can be taken to limit these constraints are the following. The decompression chamber volume can be maximized, which involves an increase of the overall size. Also, the tube section can be minimized, which may, however, affect the visibility. Further, a liquid-liquid interface may be used, which requires either one compliant reservoir at each end of the tube, or, as an alternative, a tube making a loop, which would not require any kind of reservoir space.

Here we describe the piston with rigid decompression chamber force calculations, namely the piston reaction force. For a system with a piston, and a liquid-gas interface, the force acting on the piston will vary linearly with the progression of the liquid in the indicator. This, in turn, will be converted to a force that depends on the section of the piston, which can be written:

$$F_{final} = P_{initial} \cdot \frac{V_{chamber} + V_{tube}}{V_{chamber}} \cdot S_{piston}$$

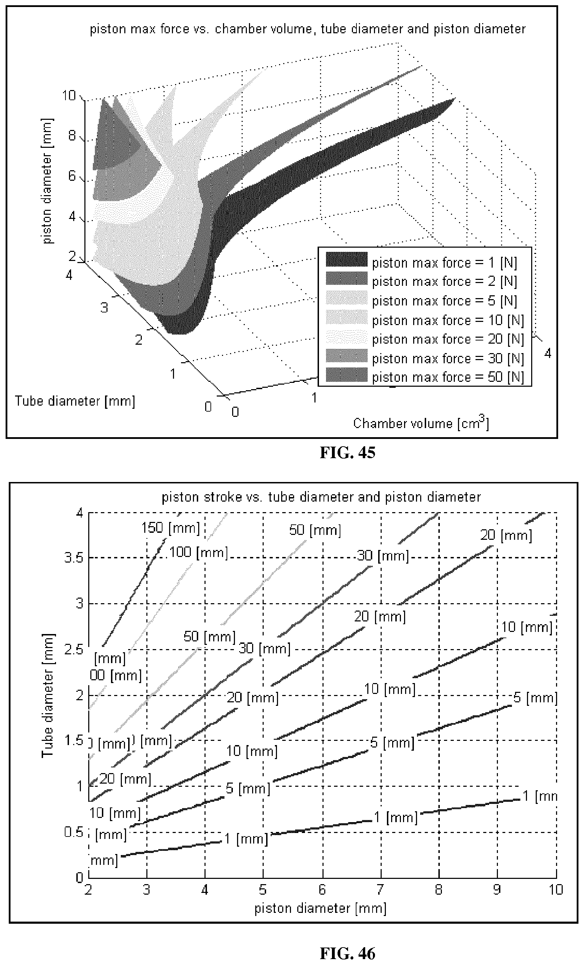

The maximal force acting on the piston, as a function of the tube diameter, chamber volume and piston diameter, is presented in **FIG. 45.**

It is visible that on a large part of the graph, the maximal force does not exceed 1 [N].

The piston stroke, as a function of the piston diameter and tube diameter, is presented in **FIG. 46.** It will have to be set depending on the dimensional constraints of the device, but will also affect the pump energy consumption, for a larger piston will require more force to be actuated.

The mechanical power is defined as:

$$P_{mechanical} = \frac{F_{piston} \cdot d_{stroke}}{t_{stroke}}$$

$$P_{mechanical\_average} = \frac{F_{piston\_average} \cdot d_{stroke}}{t_{stroke}}$$

$$P_{mechanical\_average} = \frac{F_{piston\_max}/2 \cdot d_{overall\_stroke}/144}{t_{stroke}}$$

With $d_{stroke}$ the distance that has to be provided by the piston for one 5 minutes increment, $d_{overall\_stroke}$ the previously computed overall stroke length of the piston, and $t_{stroke}$ the stroke duration defined as 1 [s]. As the actuator force rises linearly with the progression of the display, half of the maximal calculated force is considered to be the average required force.

The required electrical power can then be computed as:

$$P_{electrical\_average} = \frac{P_{mechanical\_average}}{\eta_{total}}$$

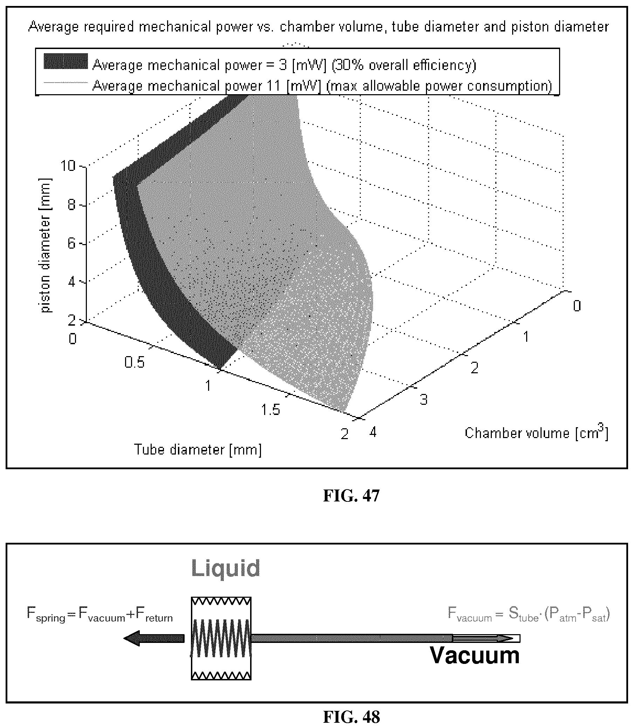

With $\eta_{total}$ the overall efficiency of the system, considering both electrical and mechanical power losses. Isosurfaces of power consumption can then be drawn, such as presented in **FIG. 47.** The overall allowable power consumption is estimated to be 11 [mW], such as that which one coin cell can supply the system during two years of continuous operation.

Considering an overall efficiency under 30% to set a reasonable limit for the average energy consumption, one reaches a value of 3 [mW].

For the calculation of the maximal allowable power consumption, the assumption is taken that the return is done using the pressure generated during the forward motion, i.e. that the actuator does not have to be activated for the return.

It is visible that the trend for the power consumption is not the same as for the force. This is due to the fact that, while larger pistons require more force, their stroke distance is greatly reduced.

Here we describe the piston return time vs. return force. A schematic representation of a liquid-vacuum system is presented in **FIG. 48.** In this system, the force exerted by the vacuum has to be compensated by the force of the return spring, so that the system is in equilibrium. In addition, a force has to be added so that the return is done sufficiently quickly.

Note that the situation is the same in a liquid-gas interface with compliant compression chamber, or in a liquid-liquid system, except that the suction generated by the vacuum is not present, which lowers the overall forces.

The flow in a tube, under a certain pressure differential, and assuming that the flow is laminar, is calculated as:

$$Q_{liquid} = \Delta P \cdot \frac{1}{R_{tube}}$$

Where $R_{tube}$ is the fluidic resistance of the tube to the advance of the liquid. It can be calculated by Poiseuille's law as:

$$Q_{liquid} = \Delta P \cdot \frac{\pi \cdot r_{tube}^4}{l_{tube} \cdot v_{liquid} \cdot 8}$$

$$Q_{liquid} = V_{liquid} \cdot S_{tube}, \; \Delta P = \frac{F_{return}}{S_{piston}}, \; S_{tube} = \pi \cdot r_{tube}^2, \; S_{piston} = \pi \cdot r_{piston}^2$$

$$V_{liquid} = \frac{F_{return} \cdot r_{tube}^2}{8 \cdot \pi \cdot l_{tube} \cdot v_{liquid} \cdot r_{piston}^2}$$

If we consider the complete return of the liquid, from the completely filled display, the fluidic resistance will drop steadily with the advance of the liquid. The average fluidic resistance will be equal to that of a tube half the total length of the tube. However, if the interface is a liquid- liquid one, the fluidic resistance will not change with the advance of the liquid. Following speeds are therefore calculated for both cases:

$$\overline{V}_{liquid, \, liquid-gas \, display} = \frac{F_{return} \cdot r_{tube}^2}{8 \cdot \pi \cdot \frac{l_{tube}}{2} \cdot v_{liquid} \cdot r_{piston}^2} \qquad \overline{V}_{liquid, \, liquid-liquid \, display} = \frac{F_{return} \cdot r_{tube}^2}{8 \cdot \pi \cdot l_{tube} \cdot v_{liquid} \cdot r_{piston}^2}$$

- The return spring force

- The tube radius

- The viscosity of the liquid

- The piston radius

The maximal specified time for the return is of 30 [s].

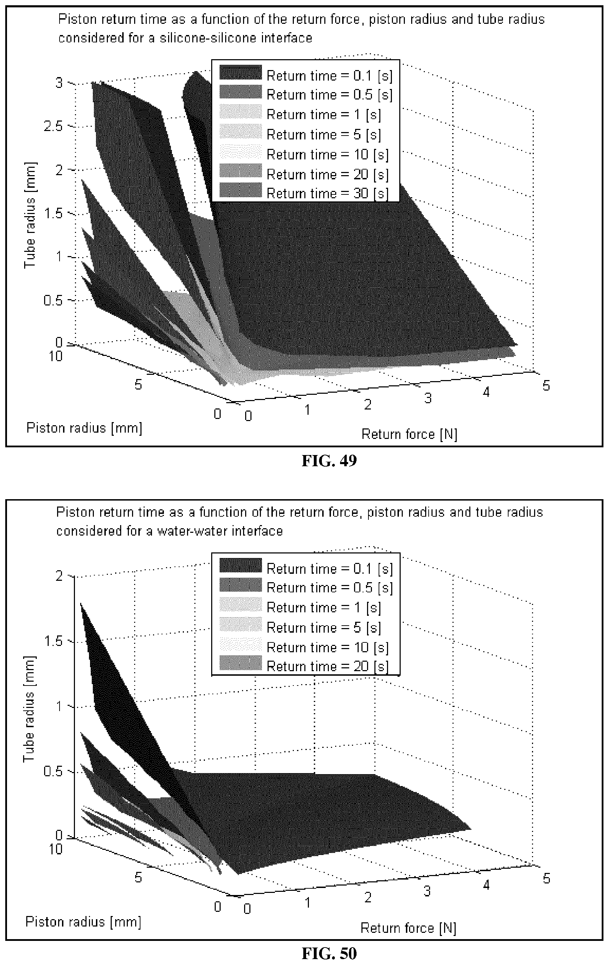

Isosurfaces of return times as a function of the tube and piston radius, and of the return force, are presented in **FIG. 49** for a silicone-silicone interface, and in **FIG. 50** for a water-water interface. It is visible that in both cases, the situation where the return takes 30 seconds or more is exceptional. However, if a much quicker return is required, particular care should be taken on the choice of the dimensions.

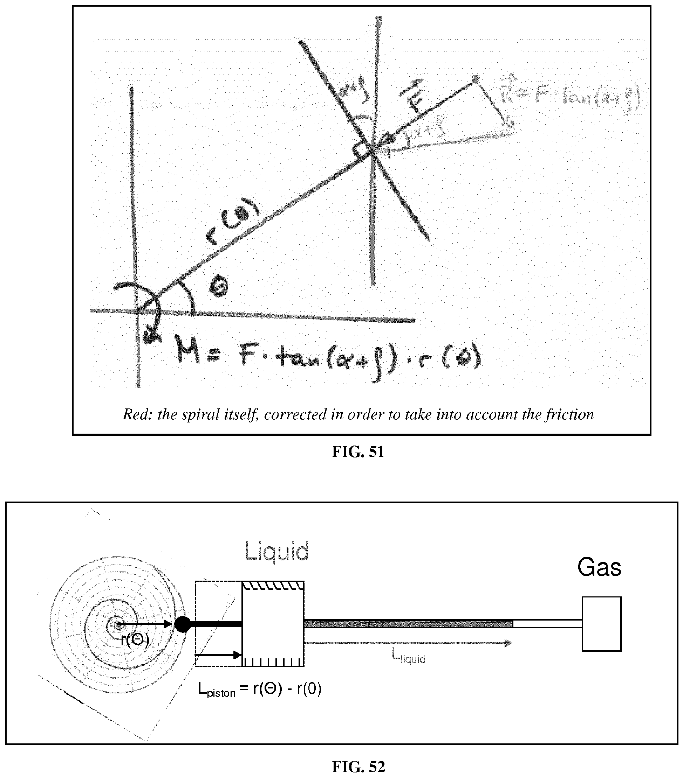

Here we describe the spiral wheel torque calculations and the general spiral formulae. The forces acting on the spiral at any given time are presented in **FIG. 51**. The variables in presence are the force (F) of the piston, the equivalent perpendicular force (R) generating the torque on the spiral, the angle ($\alpha$) between the tangent and the spiral and the tangent of a circle passing by this point (calculation follows), the added angle ($\rho$) due to the friction, calculated as $\rho = \alpha \tan(\mu)$, where $\mu$ is the friction, and the torque (M) required to turn the spiral wheel.

$\alpha$ is calculated at any point as:

$$\alpha = a\tan\left(\frac{1}{r(\Theta)} \cdot \frac{dr}{d\Theta}\right)$$

The required torque is therefore written as:

$$M = F \cdot \tan(\alpha + \rho) \cdot r(\Theta)$$

Here we describe the constant torque spiral calculation. If a rigid compression chamber is to be used, the spiral shape has to be adapted accordingly, in order to keep the torque on the drive constant. If a logarithmic spiral were used in this case, the torque would augment while the display advances, which would require implementing a drive that would be overdimensioned over most of the stroke distance, in order to be capable of providing enough torque at the end of the stroke.

The generalized spiral system is presented with some of its key values in **FIG. 52**.

The pressure in the compression chamber can be written as:

$$P_{chamber}(L_{liquid}) = P_{initial} \cdot \frac{V_{chamber} + V_{tube}}{V_{chamber} + V_{tube} - L_{liquid} \cdot S_{tube}}$$

known:

$$L_{liquid} = L_{piston} \cdot \frac{S_{piston}}{S_{tube}}, \ L_{piston} = r(\Theta) - r(0), \ F_{piston}(\Theta) = \left(P_{chamber}(\Theta) - P_{atmosphere}\right) \cdot S_{piston}$$

then:

$$F_{piston}(\Theta) = \left[P_{initial} \cdot \frac{V_{chamber} + V_{tube}}{V_{chamber} + V_{tube} - \left(r(\Theta) - r(0)\right) \cdot S_{piston}} - P_{atmosphere}\right] \cdot S_{piston}$$

We want in this calculation to have a constant torque on the drive. As seen in the previous section, the torque is calculated as:

$$M(\Theta) = F(\Theta) \cdot \tan(\alpha(\Theta) + \rho) \cdot r(\Theta)$$

In order to solve this equation, we assume that, the contribution of the friction is null. We know that $\alpha$, the angle of the spiral, can be calculated as:

$$\alpha = a\tan\left(\frac{1}{r(\Theta)} \cdot \frac{dr}{d\Theta}\right)$$

Therefore, the torque can be calculated approximately as:

$$M(\Theta) = F(\Theta) \cdot \frac{dr(\Theta)}{d\Theta}$$

A constant torque means that we want the derivative of the torque as a function of the angular position of the spiral to be zero. Therefore:

$$\frac{dM(\Theta)}{d\Theta} = \frac{dF(\Theta)}{d\Theta} \cdot \frac{dr(\Theta)}{d\Theta} + F(\Theta) \cdot \frac{d^2 r(\Theta)}{d\Theta^2} \overset{!}{=} 0$$

As the force depends on the angle, this leads to a complex second order differential equation. Should a solution involving a spiral wheel, and a liquid-gas interface be chosen, the shape of the spiral is computed numerically.

Note that, if any shape of spiral but an Archimedean spiral is used, the step size to be performed by the motor will not be constant along the movement of the piston, for the distance increment of the spiral will not be constant with the angle.

Here we describe the archimedean spiral calculations. The Archimedean spiral is one of the simplest shapes, with as equation:

$$r(\Theta) = a + b \cdot \Theta$$

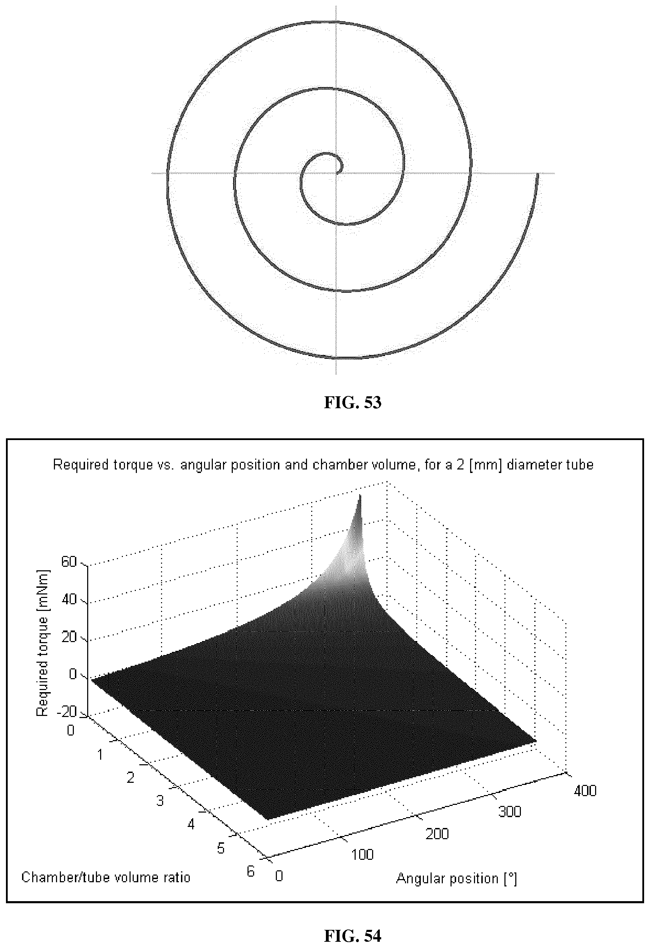

One such spiral is presented in **FIG. 53.** It has the particularity that, for a given rotation of the spiral, the linear displacement of the piston pressed against it is always constant, whatever the angle. This situation is not true for other spiral shapes. Should any other spiral but an Archimedean spiral be used, the rotation speed of the motor would not be constant, in order to achieve a constant displacement of the indicator.

The Archimedean spiral has the property that the spiral slope $\alpha$ decreases with the progression of the angular position $\Theta$, which in turn diminishes the required torque. It is hereafter presented as a possible solution for the situations where gas has to be compressed in a rigid chamber.

As calculated in the precedent chapter, the torque to be provided by the actuator for a general spiral compressing gas is:

$$M(\Theta) = F(\Theta) \cdot \tan(\alpha(\Theta) + \rho) \cdot r(\Theta)$$

In an Archimedean spiral, the spiral slope angle is calculated as:

$$\alpha = a\tan\left( \frac{1}{r(\Theta)} \cdot \frac{dr}{d\Theta} \right)$$

$$\alpha = a\tan\left( \frac{b}{a + b \cdot \Theta} \right)$$

While the torque is:

$$M(\Theta) = F(\Theta) \cdot \tan(\alpha(\Theta) + \rho) \cdot r(\Theta)$$

Neglecting the effect of the friction, it is possible to write:

$$M(\Theta) = F(\Theta) \cdot \tan(\alpha(\Theta)) \cdot r(\Theta)$$

$$M(\Theta) = F(\Theta) \cdot \frac{b}{a + b \cdot \Theta} \cdot r(\Theta)$$

$$M(\Theta) = F(\Theta) \cdot b$$

Here we describe torque calculation for a liquid-gas interface, with the force calculations established in the section above, describing the constant torque spiral calculation. We can write:

$$M(\Theta) = \left[ \frac{P_{initial} \cdot (V_{chamber} + V_{tube})}{V_{chamber} + V_{tube} - b \cdot \Theta \cdot S_{piston}} - P_{atmosphere} \right] \cdot S_{piston} \cdot b$$

In our specific case, the spiral will have only one turn. The parameters of the spiral therefore be defined as a is the minimal radius of the spiral, which has only a design importance.

$$b = \frac{d_{overall\_stroke}}{2 \cdot \pi}$$

Therefore, as:

$$d_{overall\_stroke} \cdot S_{piston} = V_{tube}$$

$$M(\Theta) = \frac{P_{initial} \cdot (V_{chamber} + V_{tube}) \cdot \frac{V_{tube}}{2 \cdot pi}}{V_{chamber} + V_{tube} \left(1 - \frac{\Theta}{2 \cdot \pi}\right)} - P_{atmosphere} \cdot \frac{V_{tube}}{2 \cdot \pi}$$

It is remarkable that for an Archimedean spiral, if the friction is neglected, the torque characteristic does not depend on the geometry of the spiral. This can be explained as follows: if the spiral has a high slope, the stroke of the piston will be longer, meaning that its surface will be lower. This in turn will lead to a lower pressure being applied on the piston surface, which compensates for the high slope.

Note that the volume of the reservoir does not have a role in the calculation, as it is by definition equal to the volume of the tube. The reservoir will merely have to be scaled according to the tube dimensions.

The last equation can be simplified by presenting the chamber volume as a function of the tube volume, such as:

$$V_{chamber} = V_{tube} \cdot \kappa$$

$$M(\Theta) = \frac{P_{initial} \cdot (V_{tube} \cdot \kappa + V_{tube}) \cdot \frac{V_{tube}}{2 \cdot \pi}}{V_{tube} \cdot \kappa + V_{tube} \left(1 - \frac{\Theta}{2 \cdot \pi}\right)} - P_{atmosphere} \cdot \frac{V_{tube}}{2 \cdot \pi}$$

$$M(\Theta) = \frac{P_{initial} \cdot V_{tube} \cdot (\kappa+1) \cdot {}^{V_{tube}}\!\!/_{2 \cdot \pi}}{V_{tube} \cdot \left(1+\kappa - {}^{\Theta}\!\!/_{2 \cdot \pi}\right)} - P_{atmosphere} \cdot \frac{V_{tube}}{2 \cdot \pi}$$

$$M(\Theta) = \frac{P_{initial} \cdot (\kappa+1) \cdot {}^{V_{tube}}\!\!/_{2 \cdot \pi}}{1+\kappa - {}^{\Theta}\!\!/_{2 \cdot \pi}} - P_{atmosphere} \cdot \frac{V_{tube}}{2 \cdot \pi}$$

It is noteworthy that the torque still depends on the absolute value of the tube diameter. However, the ratio alone is important regarding the variation of the torque with the angular position. **FIG. 54** presents the curve of the torque as a function of the chamber volume to tube volume ratio, and to the angular position, for a 2 [mm] tube diameter.

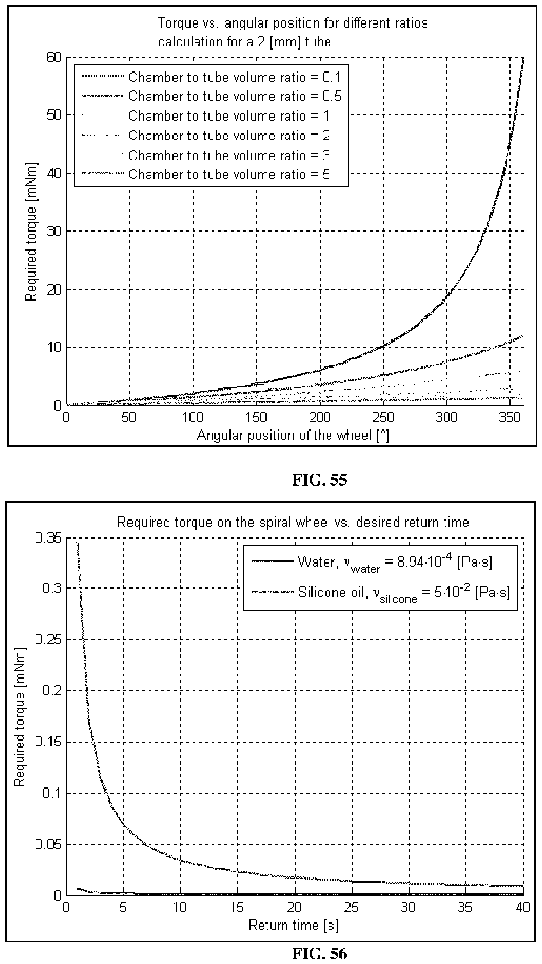

The same curve is represented as cuts for different ratios in **FIG. 55.** It is visible that the torque can be kept relatively stable if ratios above 2 are used for the chamber volume.

As it is visible, those approximate calculations neglect the friction, which leads to a null torque at the beginning of the rotation of the wheel. The friction must of course be considered to accurate results.

We conclude that using an Archimedean spiral would simplify the motor control, as each motor position increment would correspond to a constant liquid level increment. However, this spiral geometry would require a variable torque, depending on its angular position. Only if the compression chamber has more than twice the volume of the tube is it possible to keep the torque stable with an Archimedean spiral. Should the device be more compact, a constant torque spiral should be used. Alternatively, using a liquid-liquid or liquid-vacuum interface allows circumventing this issue.

Here we describe the torque calculation for a liquid-liquid interface. In the case of a liquid-liquid or liquid-vacuum interface, the force acting on the piston is considered constant. The torque can in this case be calculated as:

$$M(\Theta) = F(\Theta) \cdot b$$

$$M = F \cdot b$$

$$M = F \cdot \frac{d_{overall\_stroke}}{2 \cdot \pi}$$

It is visible that the torque is constant, and depends only on the overall stroke of the spiral. The force will be determined as the minimal force ensuring a rapid enough return of the liquid in the reservoir, with the calculations established in 5.8. As was then written, the return spring force can be calculated as a function of the desired return time as:

$$\overline{V}_{liquid, liquid-liquid\ display} = \frac{F_{return} \cdot r_{tube}^2}{8 \cdot \pi \cdot l_{tube} \cdot v_{liquid} \cdot r_{piston}^2}$$

$$\overline{V}_{liquid} = \frac{l_{tube}}{t_{return}}$$

$$F_{return} = \frac{8 \cdot \pi \cdot l_{tube}^2 \cdot v_{liquid} \cdot r_{piston}^2}{t_{return} \cdot r_{tube}^2}$$

Therefore, the torque can be calculated as:

$$M = \frac{8 \cdot \pi \cdot l_{tube}^2 \cdot v_{liquid} \cdot r_{piston}^2}{t_{return} \cdot r_{tube}^2} \cdot \frac{d_{overall\_stroke}}{2 \cdot \pi}$$

$$\pi \cdot r_{piston}^2 \cdot d_{overall\_stroke} = V_{tube}$$

$$M = \frac{4 \cdot l_{tube}^2 \cdot v_{liquid}}{t_{return} \cdot r_{tube}^2} \cdot \frac{V_{tube}}{\pi}$$

$$\frac{V_{tube}}{\pi \cdot r_{tube}^2} = l_{tube}$$

$$M = \frac{4 \cdot l_{tube}^3 \cdot v_{liquid}}{t_{return}}$$

This is a truly remarkable result. The required torque in this situation depends only on the viscosity of the considered fluid, and on the desired return time, the tube length being given.

For a liquid-vacuum interface, this torque would be divided by two, as is the average fluidic resistance of the tube during the return of the liquid in such a case.

It is visible that the required torque depends directly on the viscosity of the liquid. The resulting required torque for water and silicone oil is presented in **FIG. 56.** It is visible that, due to the difference in viscosity between water and silicone oil, the torque requirements are ultimately significantly different. However, in both cases, the torques are maintained within reasonable limits.

It is noteworthy that, in this first approximation where the friction is neglected, the torques are typically an order of magnitude inferior for a liquid-liquid or liquid-vacuum interface than for a liquid-gas interface

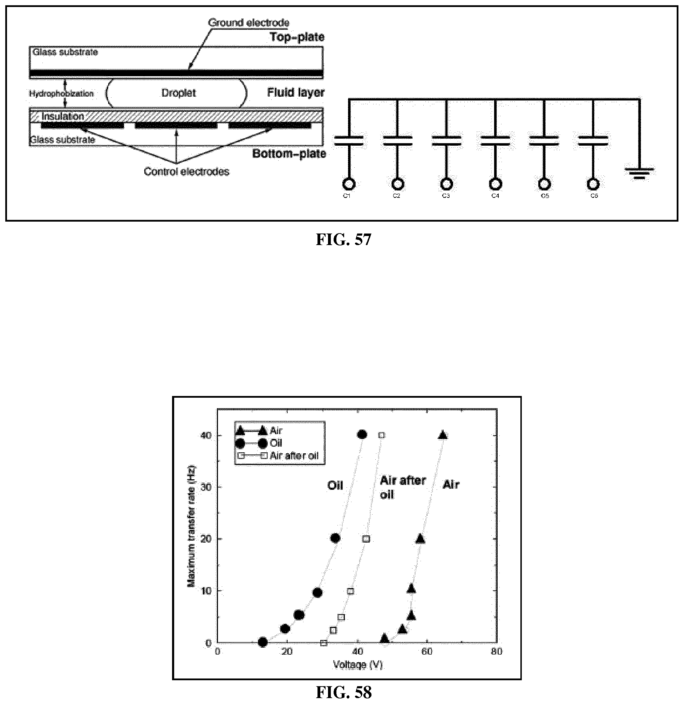

Here we describe electrowetting and power consumption. The schematic of the electrowetting principle is presented in **FIG. 57.** As presented in the right side of the figure, an electrowetting display can be represented as an array of capacitors. When the droplet has to be displaced, the electrode next to it is activated, which diminishes the surface tension on this spot, dragging the droplet. The electrode activated constitutes a capacitor whose dielectric is constituted by the insulation and hydrophobization layers, and whose ground electrode is the water droplet, itself in electrical contact with the ground electrode.

The value of a planar capacitor is calculated as:

$$C = \varepsilon_0 \cdot \varepsilon_r \cdot \frac{S_{capacitor}}{d}$$

In our case, the electrodes are rectangular, and the capacitor is constituted of two consecutive layers (insulation and hydrophobization). The hydrophobization layer, however, is too thin to provide an electrical insulation. The properties of the insulation layer are:

| Layer | Material | Thickness | Dielectric constant |
|-------|----------|-----------|---------------------|
| Insulation | Parylene C | 800 [nm] | $3.15^2$ |

The size of the electrodes can be determined as follows:

- Length = 0.833 [mm] $\rightarrow$ 120 [mm] divided in 144 electrodes

- Width = 1 [mm], assumption

The capacitor value is then approximately calculated at C = 29 [pF]. This value corresponds to the typical values in the literature, and is also a value easily measurable by the ordinary capacitive sensing chips.

A first assumption of the power consumption for one step increment, assuming that the capacitor gets completely charged in the process, can then be done with the following:

$$Q_{capacitor} = C \cdot U = I \cdot t$$

$$P = U \cdot I = \frac{C \cdot U^2}{t}$$

The goal is to do the displacement with the minimal possible voltage. If one considers the results presented in **FIG. 58**, it appears possible to move the droplet with a 20 [V] voltage, at 3 [Hz]. The displacement time is therefore of 0.3 [s], and the power required is of 0.038 [$\mu$W].

We conclude that the value of power calculated before should be taken as an indicator of order of magnitude. Refined calculations and tests should be done to confirm this value. The power appears to be extremely low. This order of magnitude is confirmed in the literature. To this consumption should be added the consumption of the electronics.

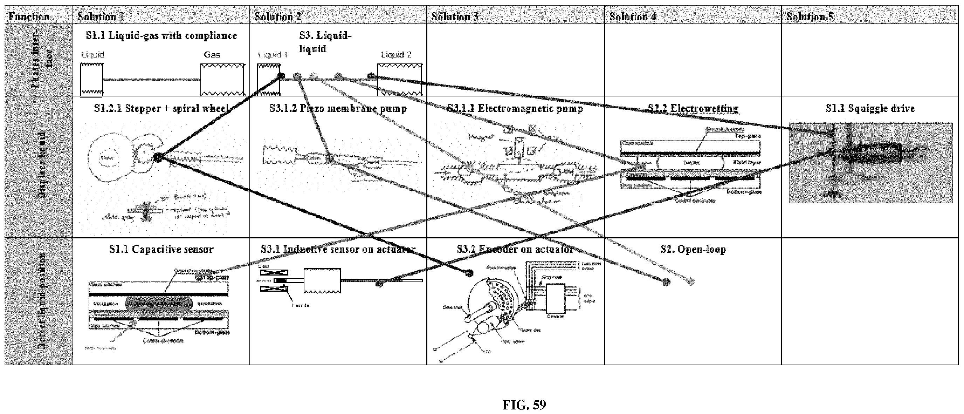

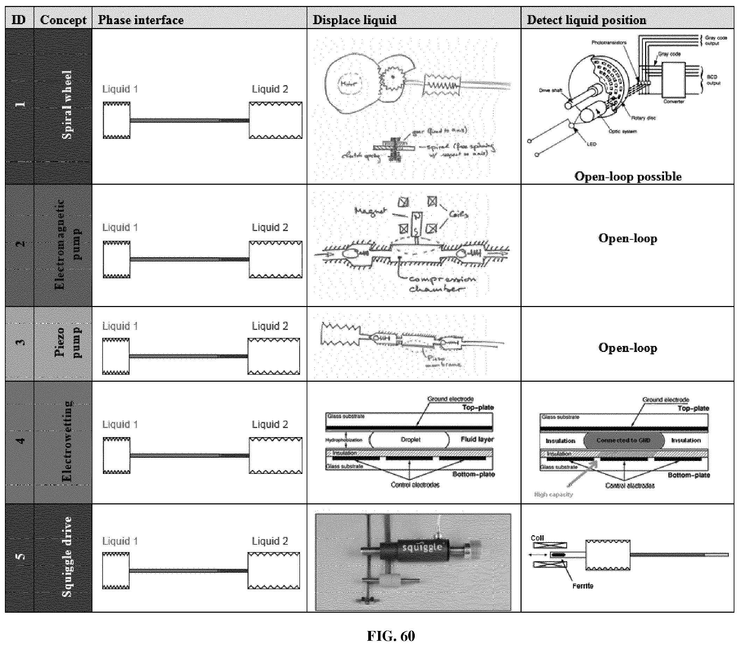

Now we describe embodiments representation and ranking. The morphological boxes method aims to combine solutions presented for the different functions of the device, in order to generate complete concepts. A summary of the retained solutions, as well as the global combinations, are presented in **FIG. 59.** As it is visible, one concept was designed per actuation method, as this function is at the core of the device. Five different concepts are therefore presented hereafter. Note that, while the liquid column sensing is quite dependent to the chosen actuation method, it is not so for the interface. The proposed interface can still be changed, for some of the proposed concepts. Five different concepts are presented in **FIG. 60.** This section presents preliminary designs of the two solutions presented in the latter section. Part 'Assumptions' presents the parameters that are assumed, for practical reasons or to simplify the calculation. Part 'Preliminary design selections' presents the calculations that lead to the other parameters.

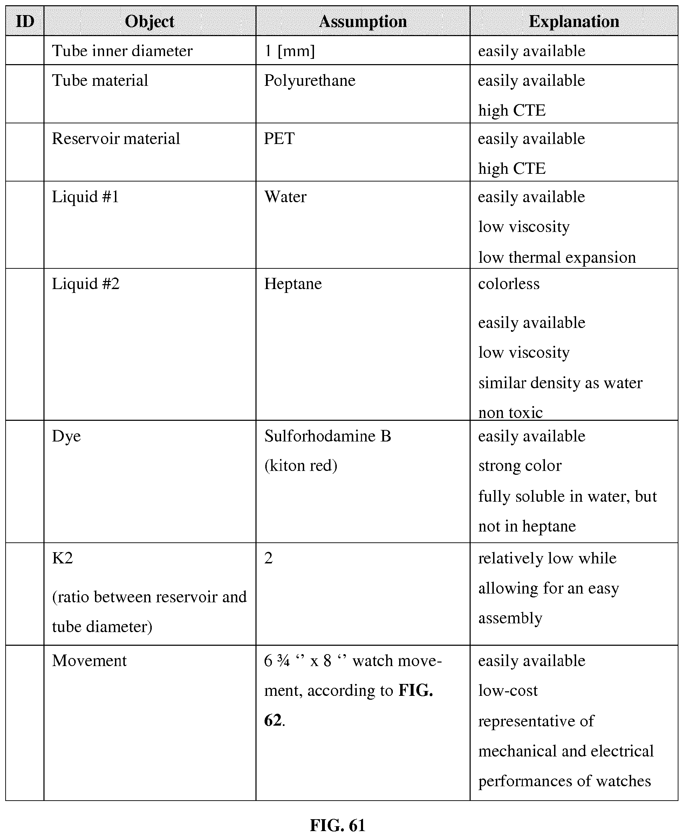

Here we describe the embodiment 1 - spiral cam. As no full optimization will be done in this phase, some parameters will be assumed. They are presented in **FIG. 61.**

Here we describe off the shelf movements. Stepper motors are widely used in the watchmaker industry such as the "Lavet" motor named after its inventor name. Several off the shelf watch movements are available on the market with following main characteristics:

Torque: 5-18$\mu$Nm on second shaft to max 1-3mNm on hour wheel after gear train reduction;

Nominal voltage: 1.5V;

Typical consumption: 2$\mu$A (no load);

Designed with a battery silver oxide $\sim$28mAh, expected lifetime: <2years;

Price per Mio parts/year: 0.45 (plastic) to 2.25 USD (metallic).

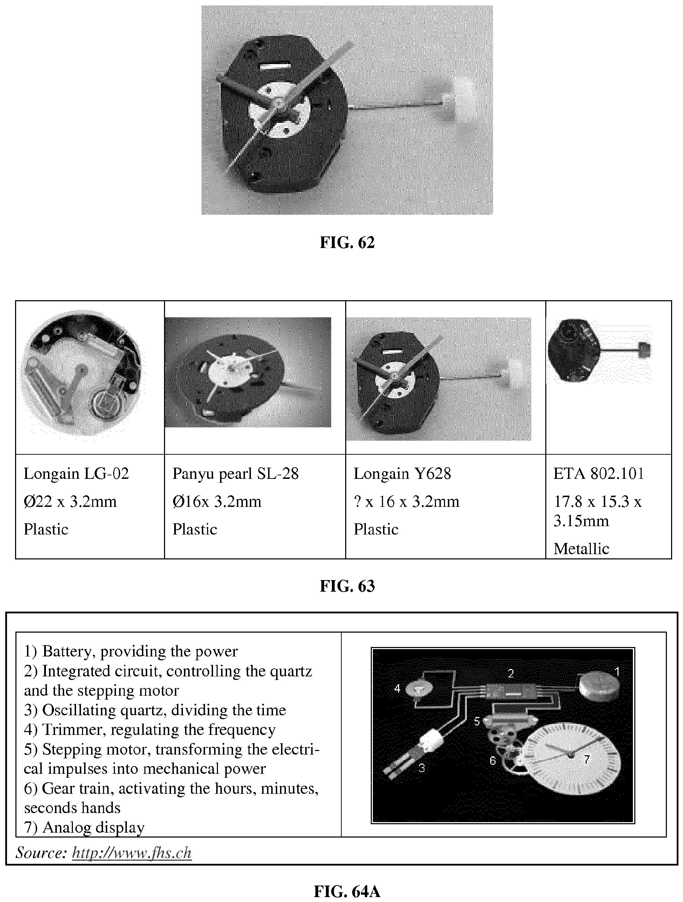

Movements cannot address display tubes of variable lengths. Examples are shown in **FIG. 63.**

Low cost plastic and metallic watch movement typically have a gear train that is addressing the seconds wheel, the minute wheel (optional) and hour wheel. Design is also sometimes including a friction clutch allowing to adjust time (hours and minutes) with help of setting stem without turning the motor.

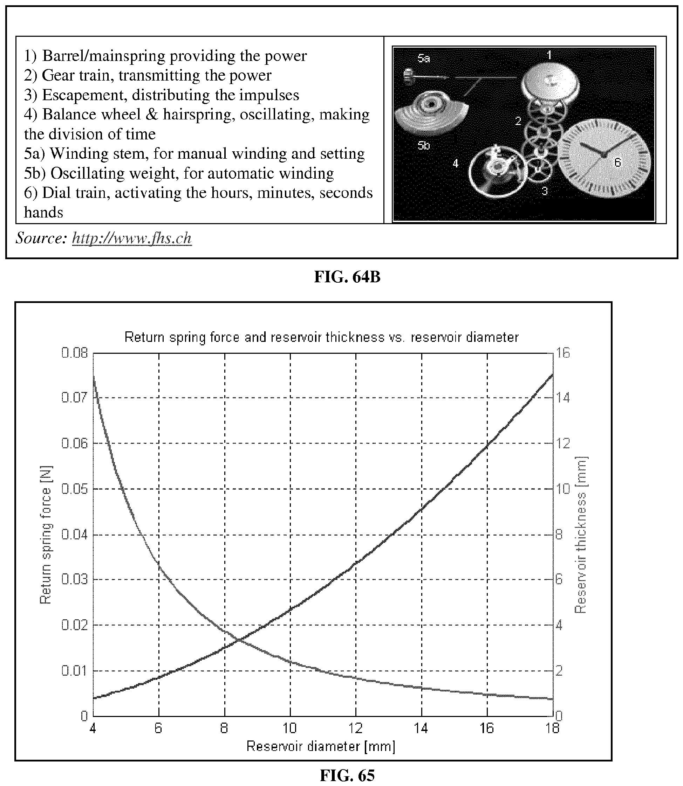

Design of watch movement is as illustrated for a digital quartz watch in **FIG. 64A** and for a mechanical watch in **FIG. 64B.**

Low cost plastic and metallic watch movement typically have a gear train that is addressing the seconds wheel, the minute wheel (optional) and hour wheel. Design is also sometimes including a friction clutch allowing to adjust time (hours and minutes) with help of setting stem without turning the motor.

The hour wheel is of interest as it is on the top of movement assembly and can directly be connected to the spiral cam for the device. Movement has already a dimension of 24 hours/day and can be easily adapted for a demonstrator design.

Here we describe time adjustments with an OEM watch movement. For a market available watch, stepper motor continuously increments time giving a minute resolution of 6°/seconds, 6°/minutes and 15°/hour for 24 hour cycles. In case of adjustments time is relatively adapted to new time by acting on hour and minutes gear train in a 12 hour time resolution. Stepper motor will then increment time with new relative time indication.

For the analog liquid watch embodiment following considerations must be regarded.

Time is relatively adjustable in a 24 hour time range (12 hours for display, 24 hours for button LED indicator).

Time increments are not in open loop as every 12 hours a reset occurs and must match the 6am or 6pm value. In this regard, coupling over liquid display and relative hour wheel must perfectly match (open loop time display).

Liquid display cannot be scaled according to variable channel length unless piston size and reservoir are adapted during device assembly.

Considerations are identical in case of a fully mechanical watch movement (ETA, lemania, ...) integration. Energy budget to be confirmed. Preliminary design focuses on a low cost plastic watch movement.

Here we describe preliminary design selections. The reservoir is the most critical part of our system. The key criterion is the linearity of the display with the advance of the piston in the reservoir; this linearity would be perfect with a piston running in a straight cylinder, but is challenging to achieve even with bellows reservoir. In addition, as we are bound to run with relatively low forces, the reservoir itself should not have a spring rate.

For this reason, the choice is on a design with a piston, and a sealing done with a rolling diaphragm. This way, the linearity is kept at its maximum with the piston actuation, while the sealing is granted by the rolling diaphragm.

The return force required for the reservoir spring depends on

the desired return time, and

the capillary force, due to the surface tension at the interface of the two liquids.

Forces for certain return times were calculated in earlier part. The effect of the capillarity is here integrated. The capillary force is calculated as:

$$F_{capillary} = 2 \cdot \pi \cdot r_{tube} \cdot \gamma_{water-heptane} \cdot \cos\left(\Theta_{contact}\right)$$

Following values are taken for the unknown parameters in this equation:

- $\gamma_{water-heptane} = 51\ [\mathrm{mN.m^{-1}}]^5$

- $\Theta_{contact} = 45° \rightarrow$ assumed value

The capillary force in a 1 [mm] diameter tube is therefore of 94 [μN]. This force is negligible with respect to the other contributions.

If we consider a cylindrical reservoir, the return spring force and reservoir height, as a function of the reservoir diameter, are presented in **FIG. 65.** It is visible that in all cases, the return spring force is relatively low. The reservoir is therefore dimensioned in order to be practical to manipulate.

Three different designs of this embodiment are developed:

11 [mm] reservoir diameter, 1 [mm] stroke, and round display;

5 [mm] reservoir diameter, 4.5 [mm] stroke, and round display; and

5 [mm] reservoir diameter, 4.5 [mm] stroke, and lineardisplay.

These two reservoir designs are developed because, regarding the cluttering aspect, a flat reservoir seems to be more appropriate. However, a flat reservoir means a short stroke, which imposes high tolerances on the cam wheel. For instance, the first design, with a 1 [mm] stroke, has a 6.9 [$\mu$m] vertical displacement of the piston per time step. This is critical regarding the tolerances of the wheel.

Note that, for all the cases, an average return spring force of 50 [mN] will be considered. This is superior to the requirement, but it would be difficult to reliably control the force of a spring with a nominal force of 10 [mN].

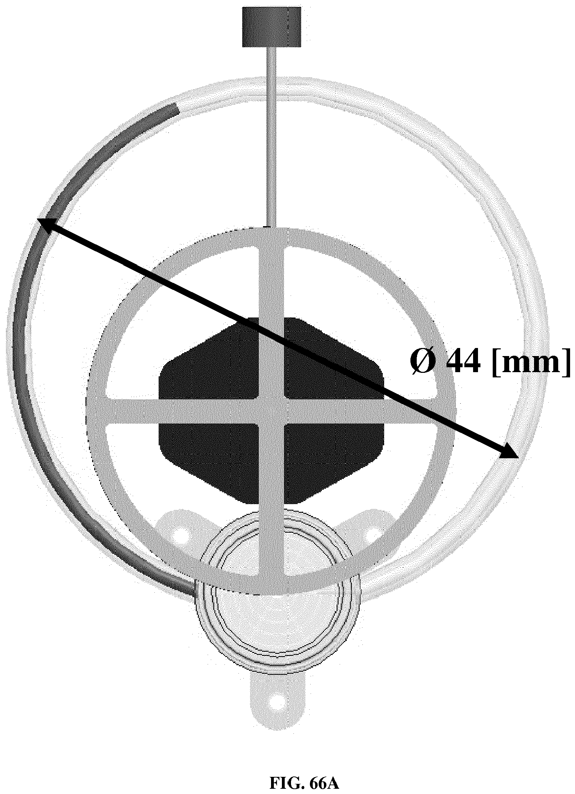

Here we describe embodiment 1, as a flat version. The embodiment 1, flat version is presented in **FIG. 66A**, with the movement and the indicator tube. It is clear that, with this design, the reservoir occupies only a fraction of the total volume.

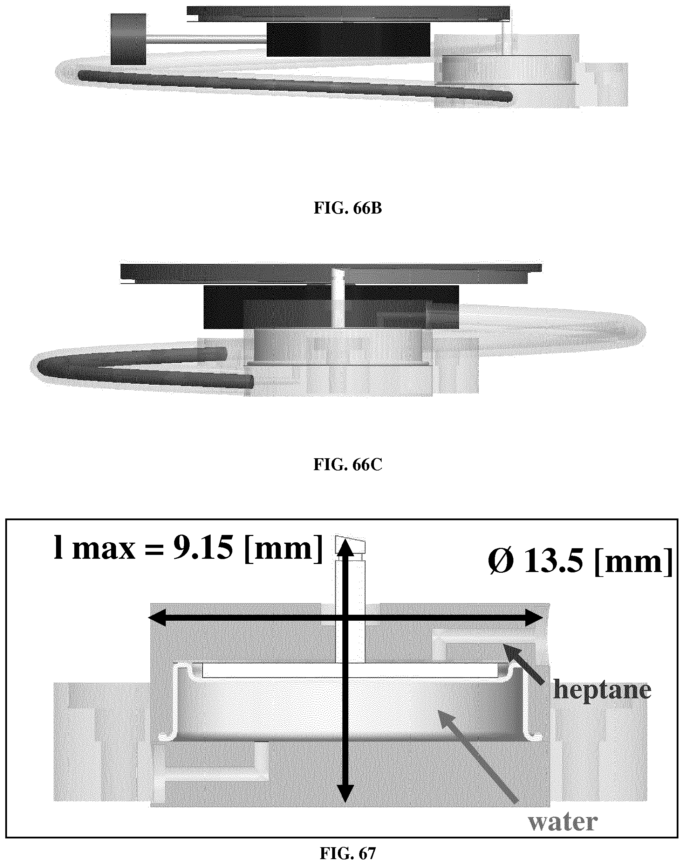

A side view of the assembly is presented in **FIG. 66B**, and a front view in **FIG. 66C.** Note that a significant optimization of the total size is still possible. Note also that, in this preliminary design, the setting wheel is in the watch. The cam wheel can be seen in the front view: it is at the "zero" position. As the watch mechanism rotates the cam wheel, it presses on the piston, which actuates the liquid.

A cross section of the reservoir is presented in **FIG. 67**. The rolling diaphragm is represented in green, and the piston is outlined in red. In this configuration, the reservoir is in its "zero" position, where the indicator liquid is entirely in the reservoir, and the vast majority of the other liquid is in the tube. As the piston advances, it pushes the water out, and frees space for the heptane behind the membrane.

Once again, the design is made so as to be easily machined. It does not represent an optimum.

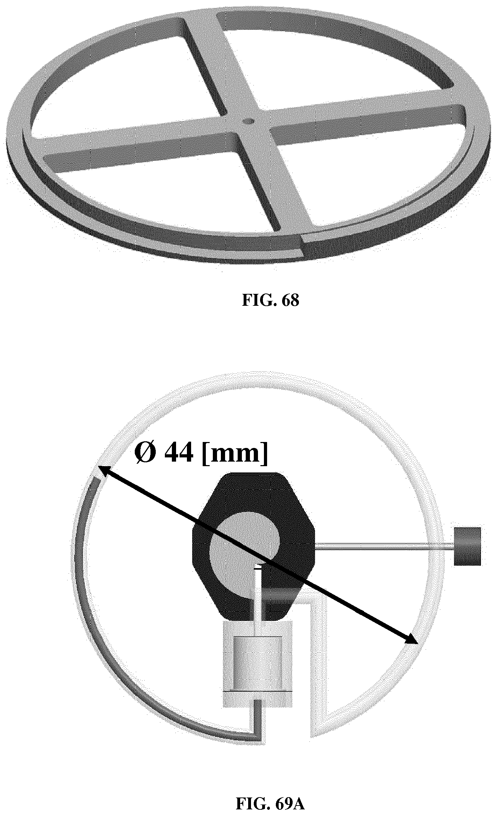

A view of the cam wheel alone is presented in **FIG. 68.** The wheel is designed such as to provide a 1 [mm] stroke over one rotation.

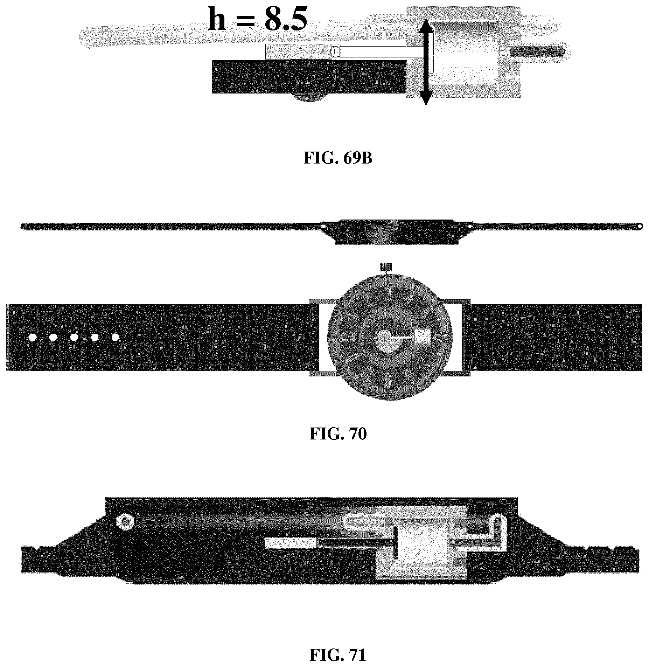

Here we describe embodiment 1, as a long, circular version. A top view of the embodiment 1 with long reservoir is presented in **FIG. 69A.** A side view, with a cut through the reservoir area, is presented in **FIG. 69B**. The reservoir design is identical to the case with a flat reservoir.

It is noteworthy that the configuration with a long reservoir allows for a more compact overall packaging, which was unexpected. All the components of the display are integrated within the 44 [mm] diameter of the display, and the assembly has an overall lesser thickness, even in this unoptimized case. In addition, no added volume has to be granted to allow for the stroke of the piston.

**FIG. 70** presents the embodiment 1, with the previously presented mechanism, packaged in a watch. In this embodiment, the front of the watch is a flat, opaque panel, with twelve glasses indicating the twelve hours. A cross section of the mechanism is presented in **FIG. 71.** Note that the casing is roomy for the current design of the mechanism. The overall size and cluttering of the watch could be reduced by making an oval display, instead of a round one, for instance.

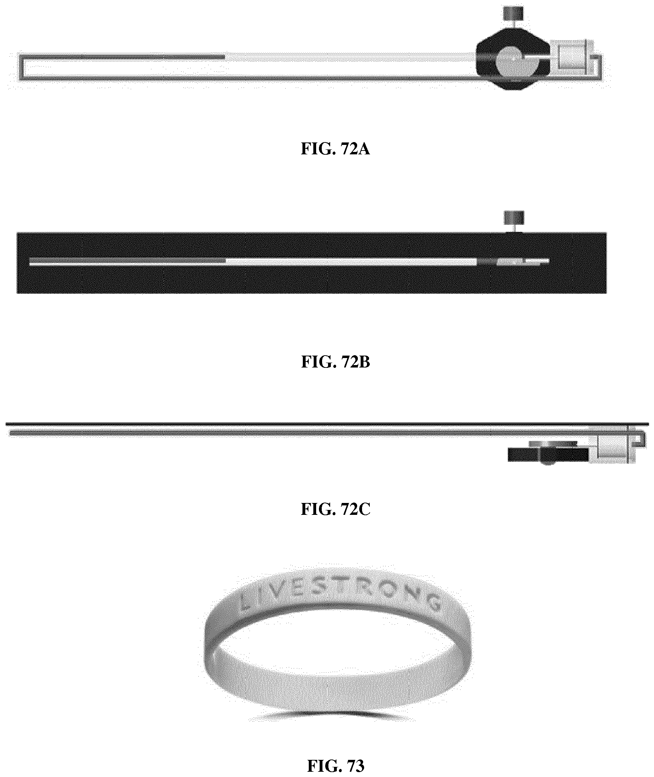

Here we describe embodiment 1, as a long, linear version in a first variant. The linear display of the embodiment 1 is presented in **FIG. 72A** and **FIG. 72B** in a top view, and in **FIG. 72C** in a side view. In this embodiment, the tube has twice the length required for the display. Alternatively, the system could be built with a slave reservoir at the end of the tube. However, this approach is not presented here because the width of the system is constrained by the actuator, leaving space for a loop of tube.

In the case of a band watch, it might be better to concentrate the thick part on one end. The total volume occupied by the system is lower in this case.

Here we describe embodiment 1, as a long, linear version in a second variant. Another was to implement the linear version of the embodiment 1 in a low-cost watch, while circumventing the limitations imposed by the need to close the bracelet, would be to build it into a flexible bracelet watch, such as the one presented in **FIG. 73.**

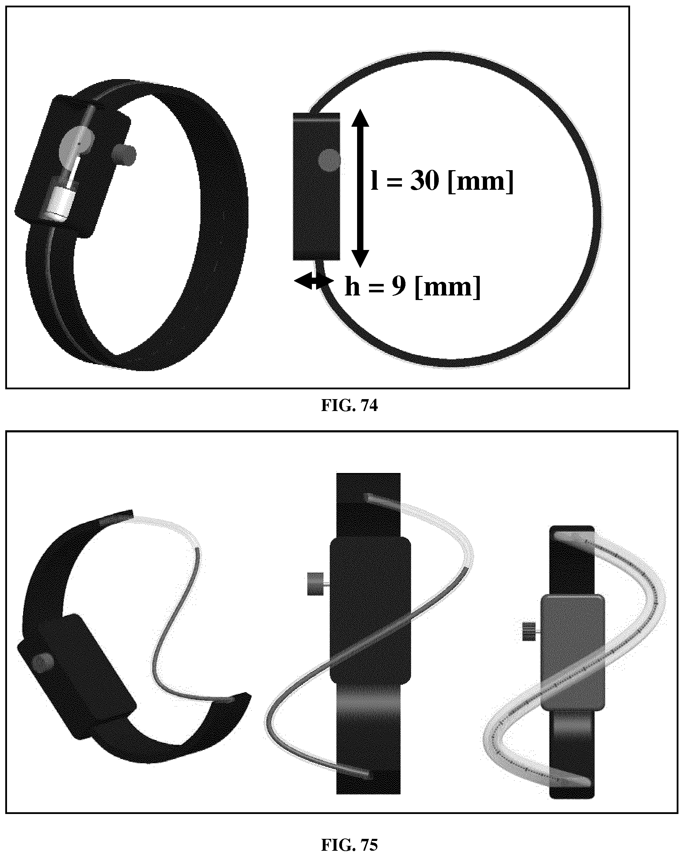

An implementation of the spiral cam mechanism in this design is presented in **FIG. 74.** It is visible that the mechanism itself can be integrated in a relatively small capsule, that would in a final device be shaped as an outgrowth of the bracelet itself. The surface of this capsule can be opaque, optionally bearing the logo of the manufacturer.

Here we describe embodiment 1, as a 'S' shaped variant. Based on the latter, bracelet design, a variation with a S shaped display is presented in **FIG. 75.** In this design, a flexible tube is fully embedded in a flexible bracelet, allowing the watch to fit on the wrist. The mechanism rests below the wrist, as it is too large to be placed on either end of the S shape.

The display itself should be of a stiffer material so as to keep its shape. Note that this could also be achieved by embedding the flexible tube in a harder display casing, that could also bear the time marks.

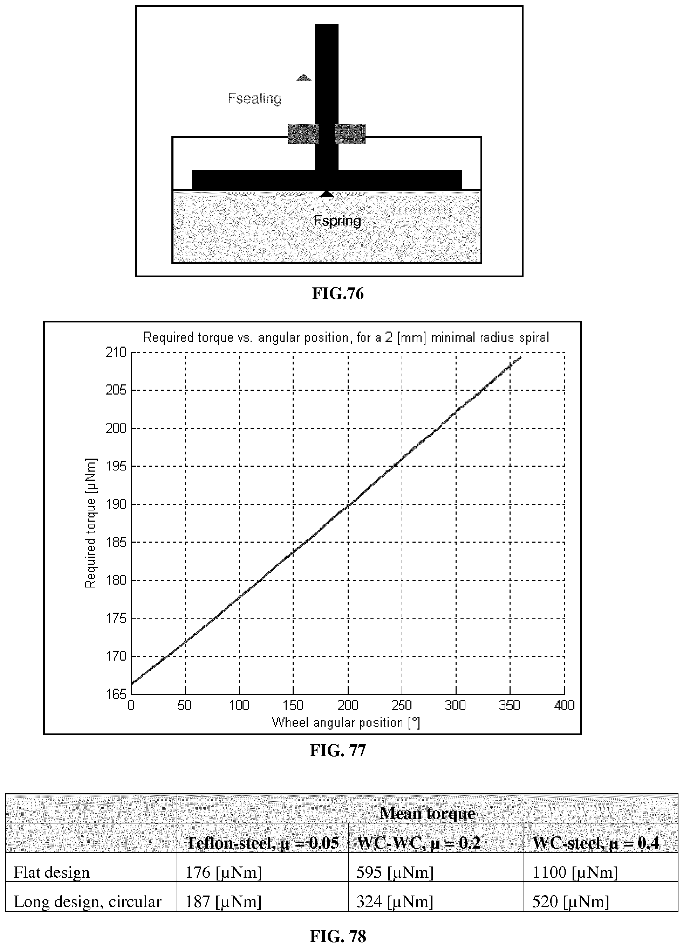

Here we describe forces acting on the system of embodiment 1. In a generalized piston case, the forces acting on the piston are presented in **FIG. 76.** The total force is equal to the sum of the force of the spring, and of the friction force applied by the sealing ring.

The force of the spring is defined as 50 [mN].

The force of the sealing has to be estimated. Considering that the pressure at the interface between the sealing and the piston is of 0.5 [bar], in order to grant a sufficient sealing, and considering that the sealing has a 1 [mm] inner diameter, and a 1 [mm] height, the radial force applied on the piston is of 0.157 [N]. Taking a worst-case friction coefficient between the rubber of the sealing and the Teflon of the piston of 1, this leads to 157 [mN] of additional force on the piston.

Here we describe the torque calculation for the system of embodiment 1. As presented in part of the general spiral formulae, the torque on a spiral is calculated as:

$$M = F \cdot \tan(\alpha + \rho) \cdot r(\Theta)$$

$$\rho = a\tan(\mu)$$

$$\alpha = a\tan\left(\frac{h_{stroke}}{2 \cdot \pi \cdot r}\right) \text{ (axial cam wheel design)}, \quad \alpha = a\tan\left(\frac{1}{r(\Theta)} \cdot \frac{dr}{d\Theta}\right) \text{ (spiral cam wheel)}$$

Flat design torque requirements.

In our case, following values can be used:

- F = 200 [mN], considering the spring and the friction

- $h_{stroke}$ = 1 [mm]

- r = 14.5 [mm]

- $\mu$ = 0.05, considering a steel cam wheel, and a Teflon piston

This leads to a required torque of: M = 176 [$\mu$Nm].

Long design torque requirements.

Following parameters are to be used for the long design:

- F = 200 [mN], considering the spring and the friction

- $h_{stroke}$ = 4.5 [mm]

- Spiral equation: r(theta) = 2 [mm] + 4.5 [mm] . theta/(2·$\pi$)

- $\mu$ = 0.05, considering a steel cam wheel, and a Teflon piston

The torque as a function of the angular position of the wheel is therefore presented in **FIG. 77**. The mean torque is of 187 [$\mu$Nm].

The long design, with the linear display, should have a twice higher spring force, as the tube is twice as long. However, the spring force is overestimated in the case with a circular display, therefore the same force can be applied to the linear display as well.

The torques for both embodiments are presented in **FIG. 78**.

It is noteworthy that the flat design requires a lower torque than the long design for the lowest friction, but a higher torque with the other friction coefficients. This can be explained as follows; the torque M is calculated as:

$$M = F \cdot \tan(\alpha + \rho) \cdot r(\Theta)$$

In addition, the term tan($\alpha$ + $\rho$) can be decomposed as:

$$\tan(\alpha + \rho) = \frac{\tan(\alpha) + \tan(\rho)}{1 - \tan(\alpha) \cdot \tan(\rho)}$$

Therefore, if the angle is larger, as it is the case with the long design, an increase of the friction angle $\rho$ will have a lesser impact on the overall result.

However, the torque values are reasonable for both embodiments, and both considered friction coefficients. As a comparison, the ETA 802.001, 6 ¾" x 8" watch movement, has a typical torque on the minute shaft of 250 [$\mu$Nm]. The torque on the hour shaft, not considering the friction, should be 12 times that. A large margin therefore exists.

The same movement has a typical current consumption of 0.95 [$\mu$A]. Therefore, a 16.6 [mAh] capacity of battery is required to power the movement for two years (not considering the energy consumption of other elements, such as the LED).

Calculations were done considering tungsten carbide (WC) as the Teflon piston would risk to wear off over the life of the device, especially considering a high- end device that should have a high durability. A sapphire-sapphire interface would also have a low friction, but machining the cams out of sapphire would be challenging. Tungsten carbide, however, is almost as hard as sapphire, and its machining is known, as many drill bits are machined out of this material.

**Here we describe embodiment 2 - electrowetting.**

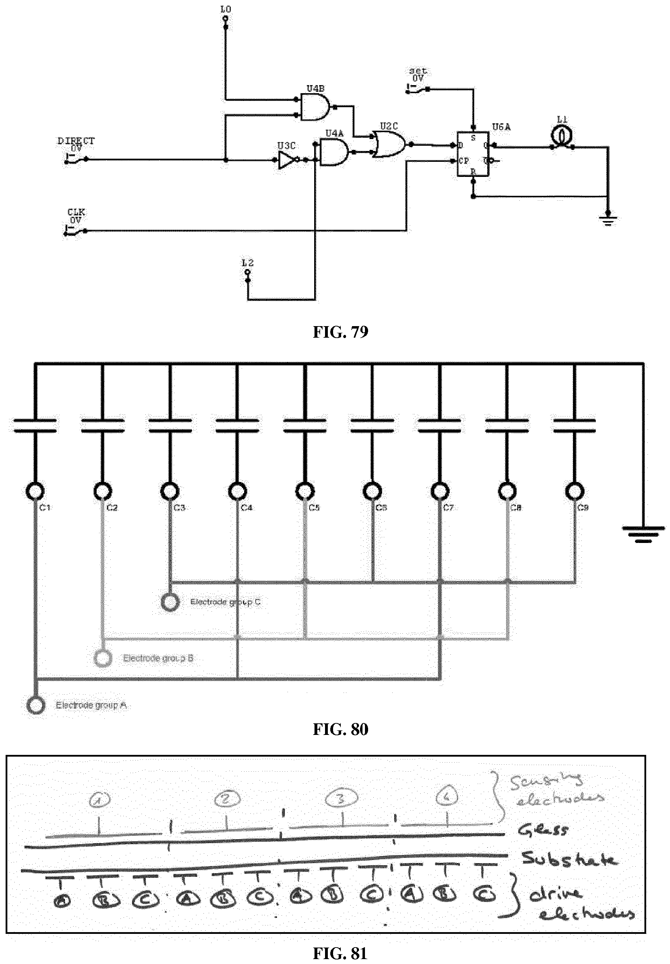

In another embodiment, a schematic representation of a simplified driver circuit for the electrowetting display is presented in **FIG. 79.** Following elements are visible in this figure:

the light bulb L1 corresponds to the state of an electrode;

the supply L0 corresponds to the state of the preceding electrode; and

the supply L2 correspond to the state of the next electrode.

This system requires only two parameters to work, namely the clock signal CLK, that indicates when to switch, and the direction DIRECT, that indicates in which direction the droplet should be moved.

The electrodes themselves would be connected as it is schematically represented in **FIG. 80.** This way, the actuation could be achieved by addressing three groups of electrodes, instead of addressing each electrode separately.

Two more components should be mounted downstream of this circuit, for each electrode group.

One gate mounted as an astable, in order to generate a finite length pulse, and one relay to apply the driving voltage on the electrodes.

Here we describe a simplified sensing circuit. A full sensing on all the electrodes wouldn't allow having the electrodes connected to a simplified driving circuit such as presented in the preceding chapter. In addition, it would require using 144 electrodes, which would make the whole system electrically very complex. For this reason, the assembly presented in **FIG. 81** is proposed. In this system, in order to drive the droplet, all the sensing electrodes are connected to the ground, and the drive signal is applied to the driving electrodes. In order to detect the position of the droplet, the driving electrodes are connected to the ground, and the position is read on the sensing electrodes.

This system allows detecting an approximate position only. It can therefore be used only in the case where the droplet can safely be assumed to remain in its position over a 15 minutes time lapse.

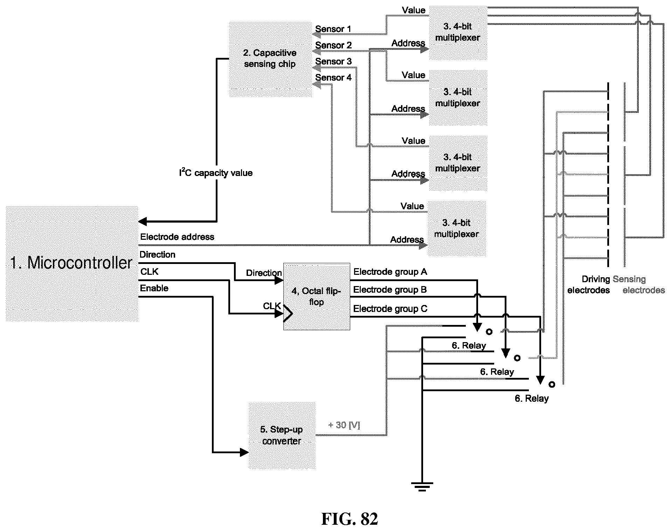

The schematic of the full driving electronics is presented schematically in **FIG. 82.** This representation integrates the principal components and some electrodes. Note that not all the wires are represented, and neither are the passive components required to have the system running.

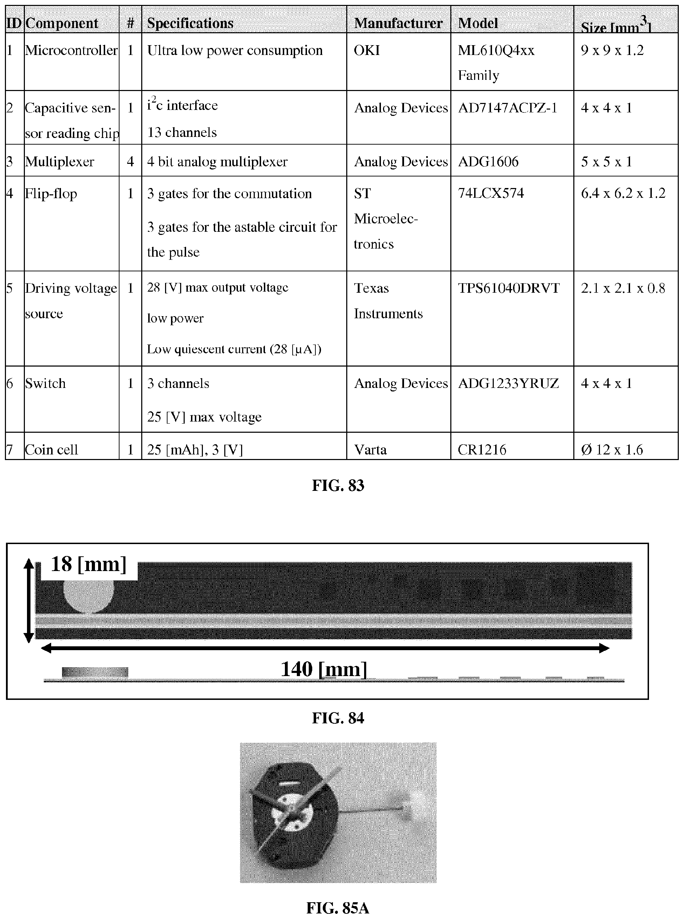

**FIG. 83** is a list of all the components required to drive the system. Note that if this product were to be mass-produced, the size and the cost could be reduced by developing a custom IC. In addition, the components presented in the latter table are a tentative list for purposes of rough design, not an optimized solution.

The top and side view of an implementation of the electrowetting display are presented in **FIG. 84,** with the aforementioned components. It is noteworthy that the size limitations for the length of the display, and the width of the coin cell, allow for a large amount of space for the electronics.

Note in addition that, although represented as a flat device in this figure, the substrate could be a flex print circuit, allowing it to be wrapped around the wrist.

A rough estimate of the power consumption of the aforementioned assembly is presented in the following table:

| ID | Element | Mean consumption |
|---|---|---|
| 1 | Capacitive sensing chip | 5 [nA] |
| 2 | Microcontroller | 0.5 [$\mu$A] |
| 3 | Step-up | 7[$\mu$A] |

(continued)

| ID | Element | Mean consumption |
|---|---|---|
| 4 | Display | Negligible |
| | **TOTAL** | **0.5 [μA]** |

With this calculation, we can conclude that a 10 [mAh] battery set is required to have the system functioning for two years without change of batteries. This is possible to achieve with standard coin cells. However, although the power consumption of the display itself is very limited, it is clear that the consumption of the different components makes this solution more energy consuming that a simple mechanical solution. Note that the power consumption would be further reduced, should a custom IC be used for this application.

**Design concepts**

Below, fluidic concepts based on a watch movement are presented. Concept 1 is a circular fluidic channel in a standard wrist watch casing, concept 2 is an elastic linear fluidic channel incorporated in a flexible bracelet, and concept 3 is a fluidic channel in a shaped "S" display. Also presented below is the electro wetting concept. Concept 4 is the electro wetting design.

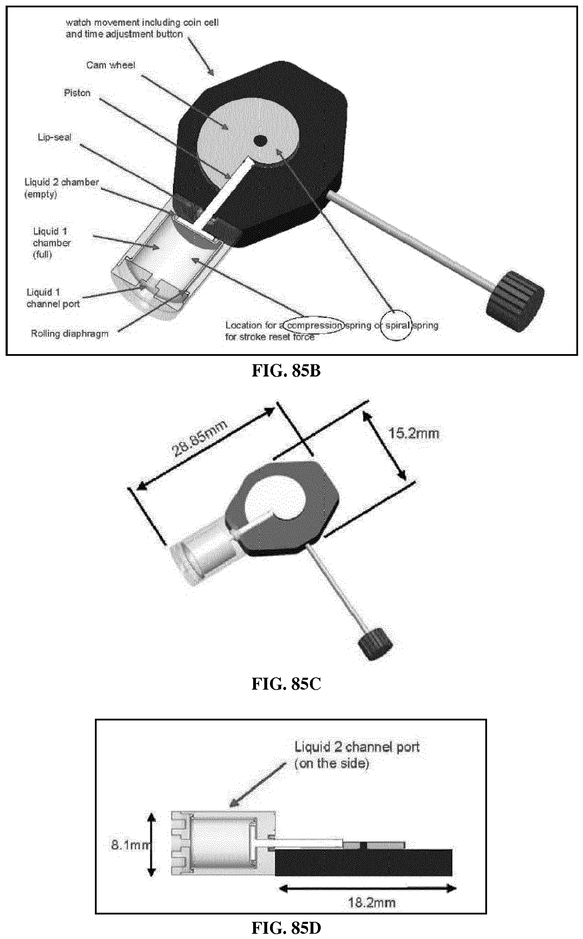

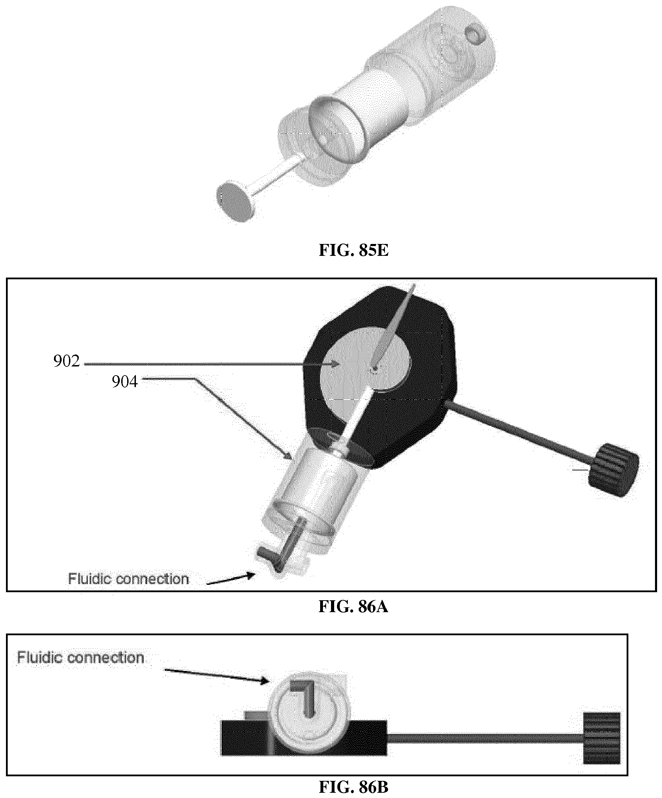

Here we describe the Fluidic concept / Watch movement coupling. The fluidic concept is based on a watch movement. Integration of a low cost electrical or high-end mechanical movement are feasible. This is shown in **FIG. 85A** to **FIG. 85E.**

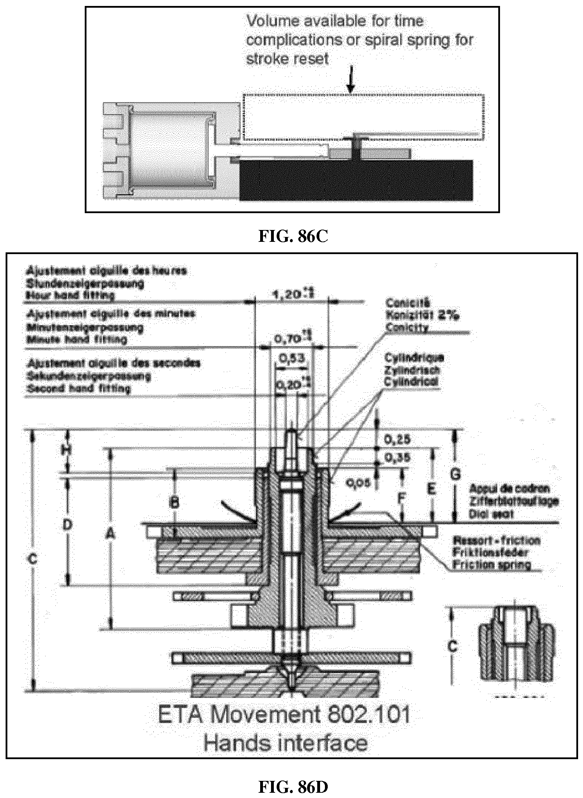

Here we describe the Fluidic concept / Assembly. Cam wheel is assembled on movement's hour fitting 902. The assembly of fluidic channel is coupled to reservoir and filling 904. The assembly is placed in the watch casing with mechanical references as for OEM movements. Second hands can be disposed on corresponding fittings or additional movement-based time complications. This is shown in **FIG. 86A** to **FIG. 86D.**

Here we describe the Concept 1. A circular fluidic channel in a standard wrist watch casing. The design can be close to a "normal" wrist watch. Channel circular is in a casing. Fluidic design is protected from the outside. A variable channel shape is possible under, above or inside of the display window. A display of fluidic/mechanic assembly is possible. A display of higher-end watch mechanism or complications is possible.

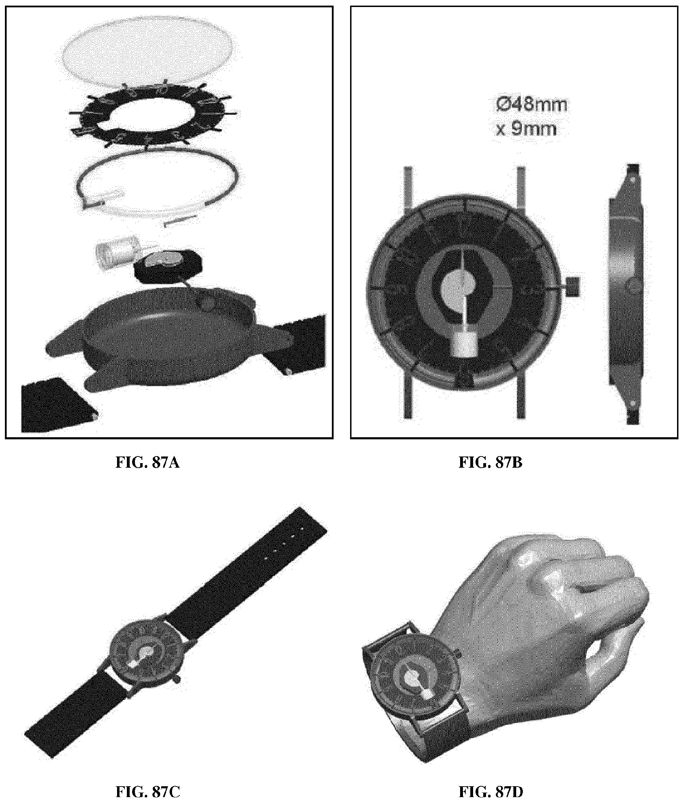

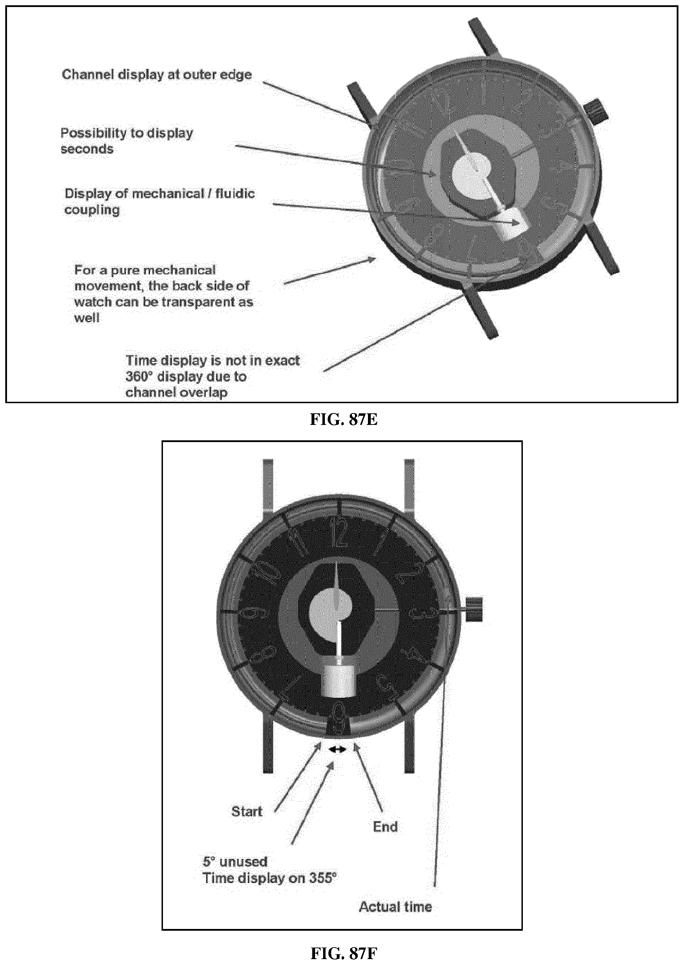

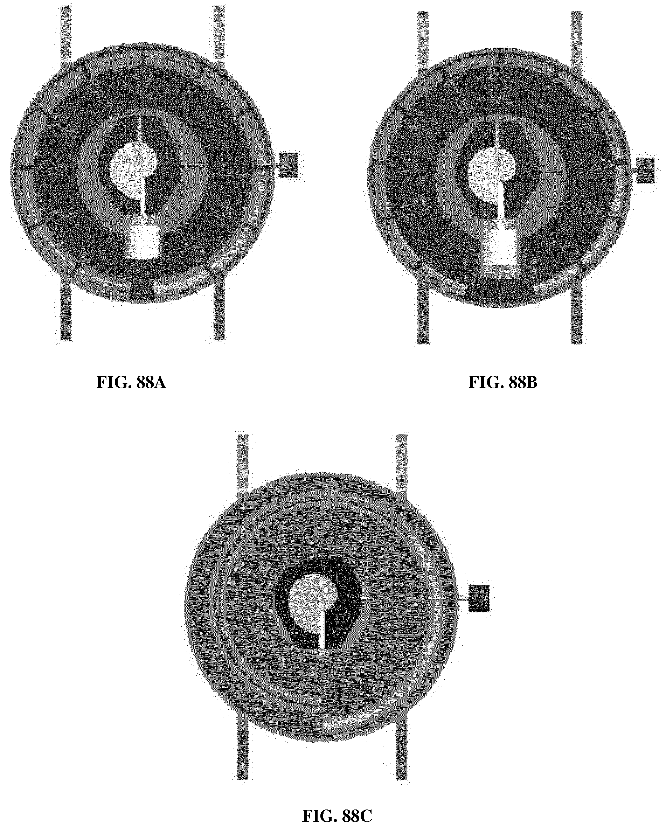

Integration of concept 1in a watch is shown in **FIG. 87A** to **FIG. 87F. FIG. 88A** shows a 355° display variant 6am/pm centered. **FIG. 88B** shows a 330° display variant Fluidic mechanism centered. **FIG. 88C** shows a 360° display variant /!\ hour length increases as channel expands along radius.

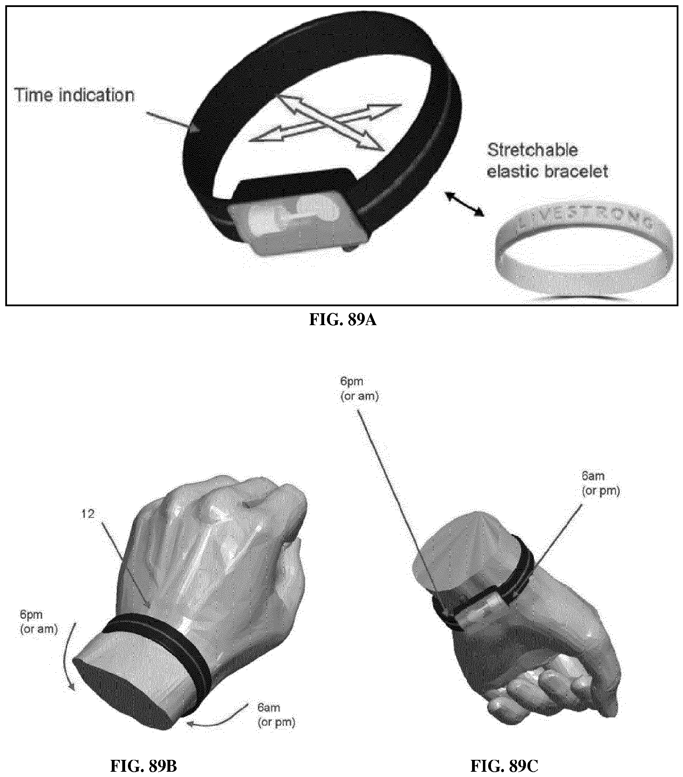

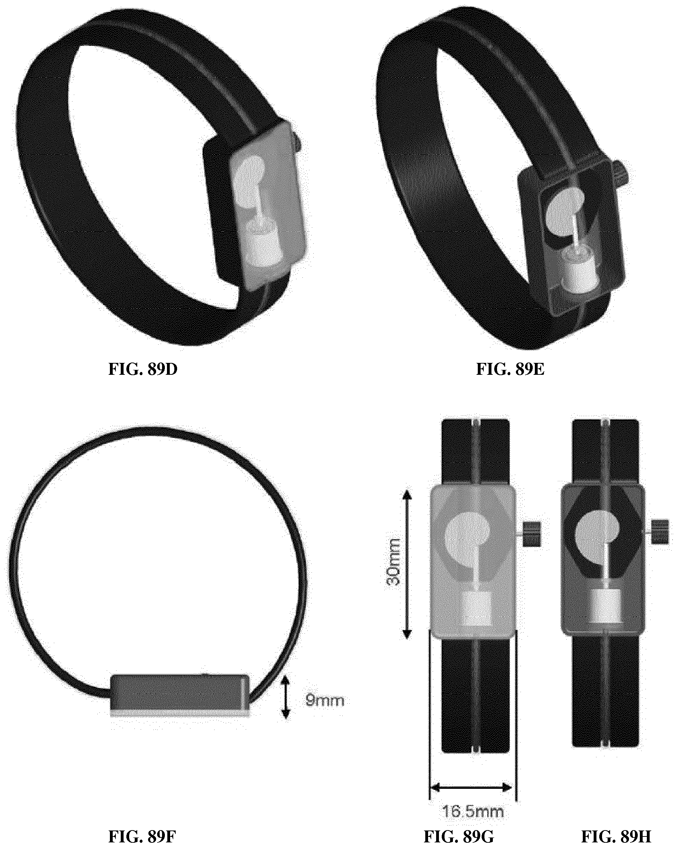

The integration of concept 2 is shown in **FIG. 89A** to **FIG. 89H.** An elastic linear fluidic channel incorporated in a flexible bracelet. This design is "reversed" compared to a watch, the casing is worn bellow the wrist. The channel is in the bracelet, both bracelet and channel are elastic. The bracelet cannot be opened. The bracelet has no fixation clips. The user has to stretch bracelet over fingers and palm to fit his wrist. The channel is circular around wrist, and could be double winded or of different shape. Fluidic design is not protected from outside. It must resist multiple stretching cycles. The mechanism could be damaged if user applies pressure on the channel. The front and backside of casing could be transparent to show the mechanism.

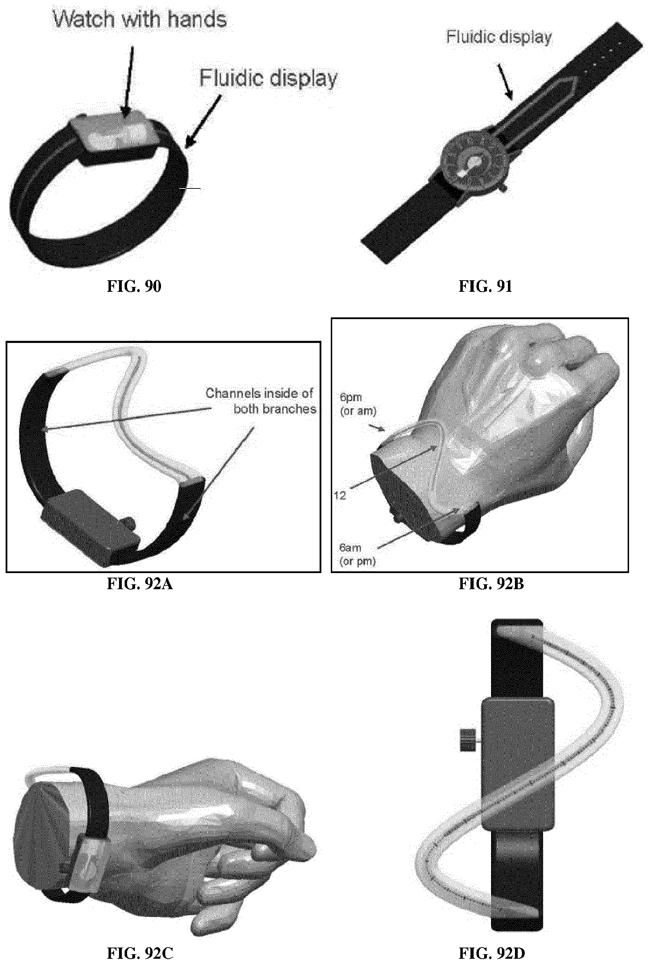

Here we describe Concept 2 in an elastic linear concept variant. The mechanism could also include seconds and minutes hand inside of casing. The cam wheel (hour hand) could integrate an indicator (hour hand). The watch would have a fluidic time display in the bracelet and a hands display in the casing bellow the wrist. This concept is shown in **FIG. 90.**

Referring now to FIG. 91, we describe Concept 2B. This watch can be worn as Concept 1 with fluidic channel in a close looped bracelet.

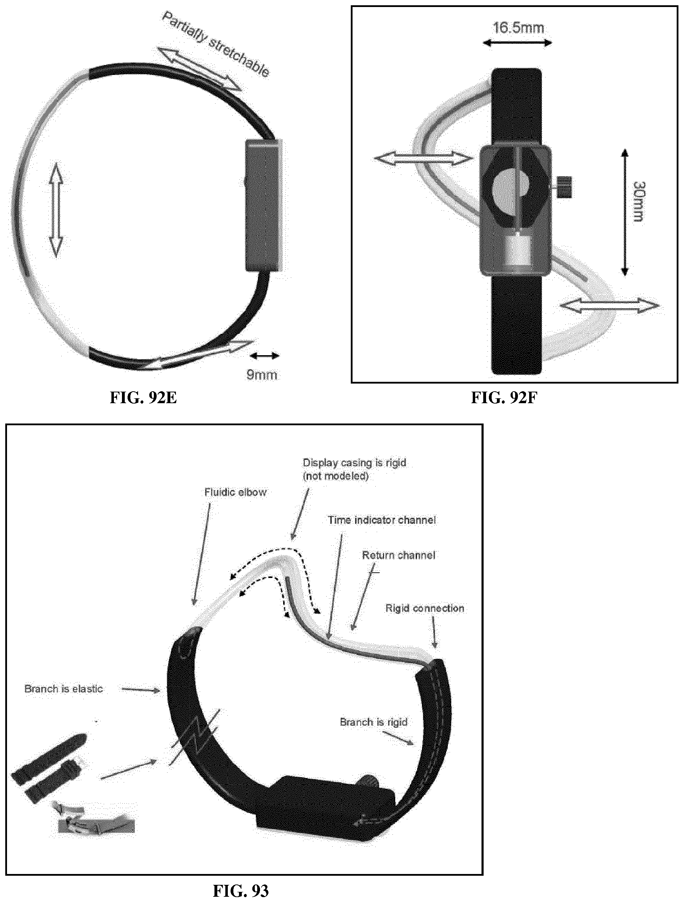

Referring now to **FIG. 92A** to **FIG. 92F**, we describe Concept 3A. Fluidic channel in a shaped "S" display. The design is "reversed" compared to a watch, the casing is worn bellow the wrist. The channel is in a bracelet, both bracelet and channel are semi-elastic. The bracelet cannot be opened. The bracelet has no fixation clips. A user puts it on by stretching the bracelet and display over the fingers and palm. The channel glass is around wrist. The fluidic design is not protected from outside. It must resist to multiple stretching cycles. The mechanism could be damaged if user applies pressure on the channel. The front and backside of the casing could be transparent to show the mechanism.

Referring to FIG. 93, we describe Concept 3B. The channel is in the "S" display with an opening system. The channel is doubled and has a return branch back to the decompression chamber in the casing. The bracelet can be opened on the other branch. The channel is partially around wrist. The fluidic design is not protected from outside. It must resist to multiple stretching cycles. The mechanism could be damaged if user applies pressure on the channel. The front and backside of the casing could be transparent to show the mechanism. Concept 3B allows display casing exchangeability (high end), which is not possible for concept 3A.

Here we describe the Electrowetting concept. This is here named concept 4. The fluidic concept is based on electrowetting. It uses capacitive sensing, and actuation with the same electrodes. The design is based on a rectangular channel. The assembly is layered, allowing bending.

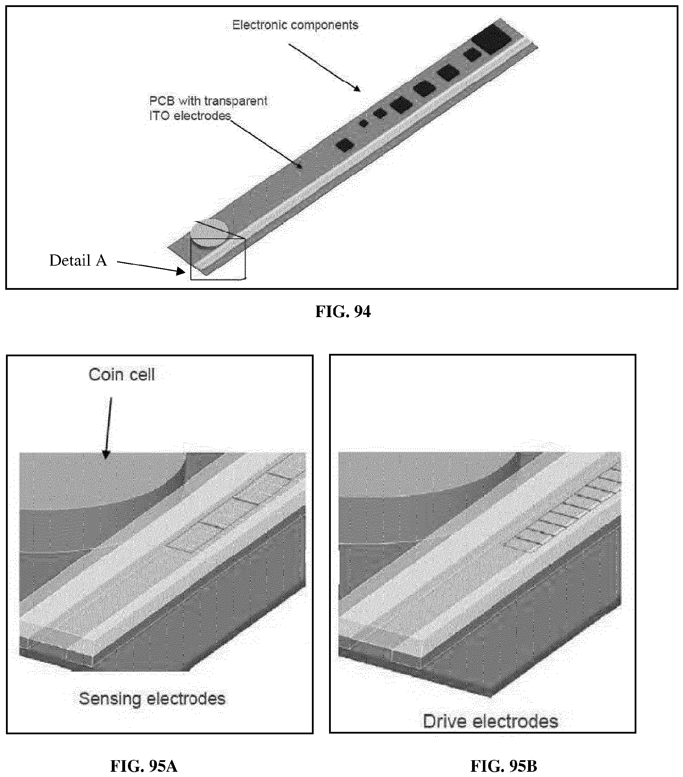

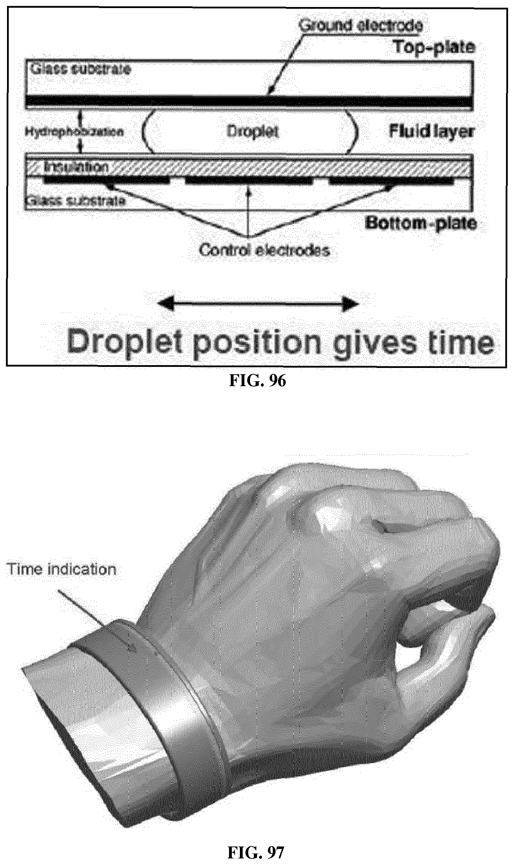

**FIG. 94** shows a PCB with transparent ITO electrodes and electronic components. **FIG. 95A** and **FIG. 95B** show the detail A of **FIG. 94**. In **FIG. 95A** the sensing electrodes are highlighted. In **FIG. 95B** the drive electrodes are highlighted. **FIG. 96** shows a schematic of the electrowetting, as provided e.g. in **FIG. 30**, **FIG. 32**, **FIG. 57.** Reference is made to **FIG. 82**, showing a full schematic of the driving electronics.

Concept 4 could have following variants. In concept 4A, timeline around the wrist is similar to concept 2. This design cannot be stretched. Therefore, the watch has a conventional clipping bracelet. In concept 4B, time is displayed in a standard watch casing. 3 droplets are moved around in different channels. Each channel has a scaling and represents seconds, minutes and hour on three concentric circles.

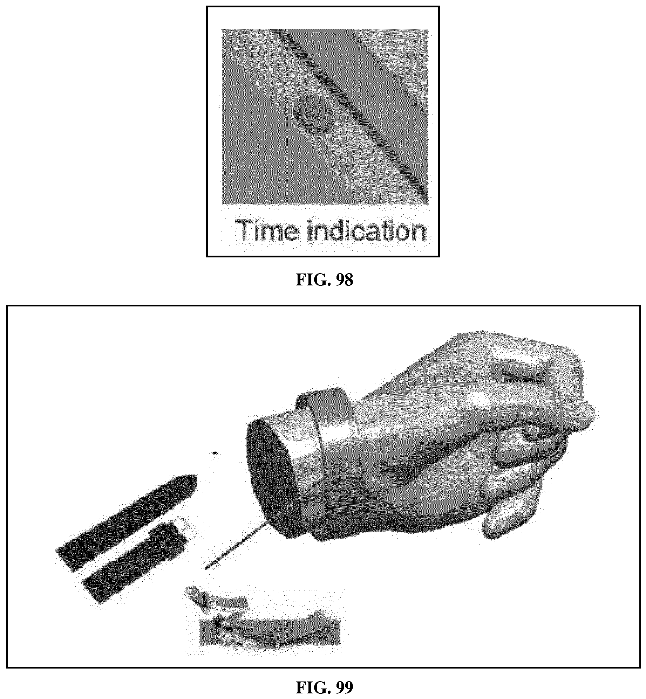

**FIG. 97** shows the indication of time on a bracelet based electrowetting. **FIG. 98** shows the time indication of **FIG. 97** in detail. **FIG. 99** shows closing devices for the bracelet.

The invention(s) may be summarized by the following feature sets:

1. A device for fluid display comprising a fluid, wherein the fluid is displaced by an electrowetting process, the device filled with at least 2 immiscible fluids whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode.

2. The device of feature set 1, wherein the displaced fluid is at least one droplet of liquid.

3. The device of feature set 1, wherein the fluids are transparent or translucent or opaque.

4. The device of feature set 1, where the fluids are showing an animation.

5. The device of feature set 1, where the fluids move along an indicia to indicate a measured value.

6. The device of feature set 1, wherein the reference electrode is undivided or divided in several portions.

7. The device of feature set 1, wherein the reference electrode is in direct electrical contact with, or isolated from the fluids.

8. The device of feature set 1, wherein the control electrodes are isolated from the fluids by a dielectric layer.

9. The device of feature set 1, where the reference electrode is located opposite to and/or adjacent to the surface of the control electrodes.

10. A method of switching the control electrodes of the device of feature set 1 in a sequence so that a portion of the fluid is displaced within the device.

11. The method of feature set 10, where the control electrodes are activated by AC or DC voltage.

12. A method of powering the control electrodes of the device of feature set 1 in a sequence so that the position of the fluid relative to the control electrodes is detected.

13. A device including the device of feature set 5, where all electrodes are transparent and where the indicia are placed below the electrodes.

14. A timepiece comprising the device of any one of the foregoing feature sets, said measured value being time.

15. The device of feature set 1, filled with at least 2 immiscible fluids whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode.

16. The device of feature set 15, wherein the displaced fluid is at least one droplet of liquid.

17. The device of feature set 15, wherein the fluids are transparent or translucent or opaque.

18. The device of feature set 15, where the fluids are showing an animation.

19. The device of feature set 15, where the fluids move along an indicia to indicate a measured value.

20. The device of feature set 16, wherein the reference electrode is undivided or divided in several portions.

21. The device of feature set 16, wherein the reference electrode is in direct electrical contact with, or isolated from the fluids.

22. The device of feature set 16, wherein the control electrodes are isolated from the fluids by a dielectric layer.

23. The device of feature set 16, where the reference electrode is located opposite to and/or adjacent to the surface of the control electrodes.

24. A method of switching the control electrodes of the device of feature set 16 in a sequence so that a portion of the fluid is displaced within the device.

25. The method of feature set 24, where the control electrodes are activated by AC or DC voltage.

26. A method of powering the control electrodes of the indicator of feature set 16 in a sequence so that the position of the fluid relative to the control electrodes is detected.

27. A device including the device of feature set 20, where all electrodes are transparent and where the indicia are placed below the electrodes.

28. A timepiece comprising the device of any one of the foregoing feature sets, said measured value being time.

29. A device comprising a fluid which indicates a measured value or creates an aesthetic shape, wherein the fluid is displaced by an electrowetting process, the device filled with at least 2 immiscible fluids whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode, wherein optionally at least one control electrode is of a size greater than 0.01 mm and so large enough to be seen by human eyes.

30. The device of feature set 18, wherein there is at least one control electrode that is designed in order to represent aesthetic shape.

31. The device of feature set 30, wherein there are control electrodes serving to gather the fluids droplets guiding them onto the area where the control electrodes are forming the aesthetic shape.

32. A method of switching the control electrodes of the device of feature set 31 so that the fluid is deformed in order to get at least one closed section of the other fluid.

33. A method of switching the control electrodes of the indicator of feature set 32 so that the fluid droplet get separated in two or more droplets.

34. A device for fluid display comprising a fluid, wherein the fluid is displaced by an electrowetting process, the device filled with at least 2 immiscible fluids whereas one fluid is activated by an electrical field generated by a control electrode wherein activation of the electrode generates a deformation or movement of at least one of the fluids.

It should be appreciated that the particular implementations shown and described herein are representative of the invention and its best mode and are not intended to limit the scope of the present invention in any way. Furthermore, any connecting lines shown in the various figures contained herein are intended to represent exemplary functional relationships and/or physical couplings between various elements. It should be noted that many alternative or additional physical connections or functional relationships may be present and apparent to someone of ordinary skill in the field.

Moreover, the apparatus, system and/or methods contemplates the use, sale and/or distribution of any goods, services or information having similar functionality described herein.

The specification and figures are to be considered in an illustrative manner, rather than a restrictive one and all modifications described herein are intended to be included within the scope of the invention claimed, even if such is not specifically claimed at the filing of the application. Accordingly, the scope of the invention should be determined by the claims appended hereto or later amended or added, and their legal equivalents rather than by merely the examples described above. For instance, steps recited in any method or process claims should be construed as being executable in any order and are not limited to the specific order presented in any claim. Further, the elements and/or components recited in any apparatus claims may be assembled or otherwise operationally configured in a variety of permutations to produce substantially the same result as the present invention. Consequently, the invention is not limited to the specific configuration recited in the claims.

Benefits, other advantages and solutions mentioned herein are not to be construed as necessary, critical, or essential features or components of any or all the claims.

As used herein, the terms "comprises", "comprising", or any variation thereof, are intended to refer to a non-exclusive listing of elements, such that any process, method, article, composition or apparatus of the invention that comprises a list of elements does not include only those elements recited, but may also include other elements described in this specification. The use of the term "consisting" or "consisting of' or "consisting essentially of' is not intended to limit the scope of the invention to the enumerated elements named thereafter, unless otherwise indicated. Other combinations and/or modifications of the above-described elements, materials or structures used in the practice of the present invention may be varied or otherwise adapted by the skilled artisan to other design without departing from the general principles of the invention.

The patents and articles mentioned above are hereby incorporated by reference herein, unless otherwise noted, to the extent that the same are not inconsistent with this disclosure.

Other characteristics and modes of execution of the invention are described in the appended claims.

Further, the invention should be considered as comprising all possible combinations of every feature described in the instant specification, appended claims, and/or drawing figures which may be considered new, inventive and industrially applicable.

Multiple variations and modifications are possible in the embodiments of the invention described here. Although certain illustrative embodiments of the invention have been shown and described here, a wide range of modifications, changes, and substitutions is contemplated in the foregoing disclosure. While the above description contains many specifics, these should not be construed as limitations on the scope of the invention, but rather as exemplifications of one or another preferred embodiment thereof. In some instances, some features of the present invention may be employed without a corresponding use of the other features. In addition, the term "flexible" as used herein encompasses the concept of variable, in that a variable volume reservoir should be considered a flexible chamber, even if no individual components flex. Accordingly, it is appropriate that the foregoing description be construed broadly and understood as being given by way of illustration and example only, the spirit and scope of the invention being limited only by the claims which ultimately issue in this application.

**Claims**

1. A device for fluid display comprising a fluid, wherein the device filled with at least 2 immiscible fluids whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode.

2. The device of claim 1, wherein the fluid is displaced by an electrowetting process.

3. The device of claim 1 or 2, wherein the displaced fluid is at least one droplet of liquid.

4. The device of claim 1 or 2, wherein the fluids are transparent or translucent or opaque, and optionally the fluids are showing an animation, and further optionally the fluids move along an indicia to indicate a measured value, wherein preferably all electrodes are transparent and where the indicia is placed below the electrodes.

5. The device of claim 1 or 2, wherein the reference electrode is undivided or divided in several portions, and the reference electrode is in direct electrical contact with, or isolated from the fluids.

6. The device of claim 1 or 2, wherein the control electrodes are isolated from the fluids by a dielectric layer, and where the reference electrode is located opposite to and/or adjacent to the surface of the control electrodes.

7. The device of claim 3, wherein when one of the electrodes is activated, the one electrode constitutes a capacitor, the capacitor constituting a dielectric adjacent the insulation and hydrophobization layers, and the droplet is a conductive fluid, such as water, being in electrical contact with the ground electrode.

8. The device of claim 7, wherein the electrodes are adapted to drive the droplet and to measure the position of the droplet.

9. A method of switching the control electrodes of the device of claim 1 or 2 in a sequence so that a portion of the fluid is displaced within the device and where the control electrodes are activated by AC or DC voltage.

10. A method of powering the control electrodes of the device of claim 1 or 2 in a sequence so that the position of the fluid relative to the control electrodes is detected.

11. A timepiece comprising the device of any one of the foregoing claims, said measured value being time.

12. A device comprising a fluid which indicates a measured value or creates an aesthetic shape, wherein the fluid is displaced by an electrowetting process, the device filled with at least 2 immiscible fluids whereas one fluid is located within the electrical field generated by a reference electrode and a control electrode and partially within the electrical field generated by the same reference electrode and at least one second control electrode so that the electric activation of the second control electrode generates a deformation or movement of the fluid in the direction of the second control electrode, wherein optionally at least one control electrode is of a size greater than 0.01 mm and so large enough to be seen by human eyes.

13. The device of claim 4, wherein there is at least one control electrode that is designed in order to represent aesthetic shape.

14. The device of claim 13, wherein there are control electrodes serving to gather the fluids droplets guiding them onto the area where the control electrodes are forming the aesthetic shape.

15. A method of switching the control electrodes of the device of claim 14 so that the fluid is deformed in order to get at least one closed section of the other fluid.

16. A method of switching the control electrodes of the indicator of claim 15 so that the fluid droplet gets separated in two or more droplets.

17. A device for fluid display comprising a fluid, wherein the fluid is displaced by an electrowetting process, the device filled with at least 2 immiscible fluids whereas one fluid is activated by an electrical field generated by a control electrode wherein activation of the electrode generates a deformation or movement of at least one of the fluids.

FIG. 1

PRIOR ART

7:51am
120 mg/hr

FIG. 2

FIG. 3

30, 27

10'

FIG. 4A

FIG. 4B

FIG. 5A

56

B

12'

A ml

35

**FIG. 5B**

A

12'

B

**FIG. 5C**

FIG. 6

A-A

FIG. 7

FIG. 8A

FIG. 8B

FIG. 8C

FIG. 8D

FIG. 8E

FIG. 8F

FIG. 9

*Electrowetting effect: no voltage applied.*

**FIG. 10A**

*Electrowetting effect: voltage applied*

**FIG. 10B**

FIG. 11

FIG. 12A

FIG. 12B

FIG. 12C

FIG. 12D

FIG. 13

Ground electrode

Top-plate

Glass substrate

Insulation                    Insulation

Insulation

Glass substrate

Bottom-plate

Control electrodes

High capacity

**FIG. 14**

| ID | Requirement | Type | quantitative / qualitative | Remarks / Answer |
|---|---|---|---|---|
| **1.** | **Minimal requirements** | | | |
| 1.1 | Device size | | | a wrist watch |
| 1.2 | Device lifetime | W | 4years MTBF | 425'000 steps over lifetime including resets and adjustments. |
| 1.3 | Device time resolution | G | 5minutes | Giving 288 steps a day, |
| 1.4 | Device time scale | G | 12hours | Start at 6am end at 5:55pm |
| 1.5 | Device display type | G | Analog | |
| 1.6 | Device display medium | G | Liquid | Liquid in tube as for example a thermometer. Device could also simulate a "digital" display based on actuation of liquid. |
| 1.7 | Corrosion | G | No corrosion | Parts in contact with liquid shall not corrode |
| 1.8 | Device energy supply | TBD, W | | Energy supply is open. Design could be purely or partially mechanically driven. |
| 1.9 | Device power consumption | TBD, W | | Based on coin cell energy budget. |
| 1.10 | Coin cell set size | TBD, W | | Based on energy budget calculations |
| 1.11 | Moving detection sensor resolution | G | 5min or 1 step | |
| 1.12 | Digital clock in device | TBD, W | | Depends on embodiment |
| 1.13 | Digital clock accuracy | TBD | | Similar to market available watches. |
| 1.14 | Digital clock time signal frequency for microcontroller | G | 1/minute | |

**FIG. 15A**

| ID | Requirement | Type | quantitative / qualitative | Remarks / Answer |
|---|---|---|---|---|
| **1.** | **Minimal requirements** | | | |
| 1.15 | Microcontroller reaction time | G<br>G | <1s<br><30s | Each liquid step is set within 1s on display<br>Each reset (full range) is set within 30s on display |
| 1.16 | Potentiometer adjustment range | G | Full range | Adjustable over all display (12hours) |
| 1.17 | Potentiometer setting accuracy | G | 5min or 1 step | |
| 1.18 | Potentiometer linearity | W | linear | Time linearly adjustable over full range |
| 1.19 | Decompression chamber size | TBD | | Depends on embodiment<br>Coupled to Liquid container capacity |
| 1.20 | Decompression chamber material | W | | According to ISO norms. Should withstand pressure cycling |
| 1.21 | Tube display size | TBD | 120mm | Full range, 12steps/10mm<br>150mm |
| 1.22 | Tube display outer shape | TBC, W | cylindrical | Wish for initial URS prototype |
| 1.23 | Tube display hollow channel shape | W | Liquid moves linearly over | |
| 1.24 | Tube display material | TBD | Transparent, Flexible | Bending radius 7.5mm<br>According to ISO norms, should withstand pressure cycling. |
| 1.25 | Scale on enclosure | TBD | Location unde fined | Thin line every 5min, thick line every 15min, Thicker line every hour |

**FIG. 15B**

| ID | Requirement | Type | quantitative / qualitative | Remarks / Answer |
|---|---|---|---|---|
| **1.** | **Minimal requirements** | | | |
| 1.26 | Liquid container capacity | TBD | Min<br><br>Max | Big enough to empty overall scale. Depends on device design. Sufficient liquid in case of tube enclosure exchangeability scenario. |
| 1.27 | Liquid material | TBD | | Transparency/opacity ? |
| 1.28 | Liquid specific material | TBD, W | Fluorescent | Depends on embodiment |
| 1.29 | Liquid color | TBD | | Colors? |
| 1.30 | Gas diffusion into liquid | G | Minimal | No bubble creation due to environmental conditions (ISO) No mixing with counter medium liquid |
| 1.31 | Counter medium to display liquid | TBD | Transparent Air or Liquid | Counter medium encapsulated in decompression chamber can be either air or liquid |
| 1.32 | Borderline | TBD, W | Liquid/Air or Liquid/Liquid | "Clear and not too much concave or convex" |
| 1.33 | Borderline stability versus temperature | W | Insensitive [°C] [-10;+40] | |
| 1.34 | Borderline stability versus gravitational field | G | Insensitive | Borderline not gravitational dependent |
| 1.35 | Borderline stability versus altitude | TBD | Insensitive [0m – 3000m] Above sea level | |

**FIG.15C**

| ID | Requirement | Type | quantitative / qualitative | Remarks / Answer |
|---|---|---|---|---|
| **1.** | **Minimal requirements** | | | |
| 1.36 | Light button | W | Time range [6pm – 5:59am] | "There shall be a light button which is illuminated from 6:00pm to 5:59 am. The light must not be very strong the aim is to show in the dark where the light button is. As a light source a blue low power LED can be used, which is powered from the coin cells (see chapter 4.6). By pressing the light button the light in the tube will be turned on." |
| 1.37 | Tube light | W | | "The tube light when turned on shall illuminate the indicator scale evenly. There shall be enough light to read the time without problems. After turning on the light it shall be turned off automatically after 10 seconds. As a power source the coin cells from the enclosure (see chapter 4.6) will be used." |
| 1.38 | Tube enclosure exchange-ability | W | Exchangeable enclosure | Tube enclosure shall be easily ex-changeable and addressed with a identification pins. Accordingly liquid display length may vary |
| 1.39 | Tube enclosure light dis-play | W | Light source along enclosure | Depends on embodiment |

**FIG. 15D**

Legend:

01  Micro motor
02  Plunger
03  Screw
04  Liquid container with return spring inside
05  Magnet
06  Movement detection circuit
07  Enclosure
08  Clock
09  Controller circuit
10  Potentiometer
11  Battery
12  Tube
13  Scale
14  Borderline
15  Decompression chamber
16  Lock mechanism
17  Light switch
18  Screw plug for batteries

**FIG. 16A**

**FIG. 16B**

**Design specific requirements**
– Time adjustable over entire range (small
adjustments,
winter/summer time,
timezone

- 5min step adjustments min (1min possible)

Clock

Potentiometer

f=1/min

Controller
Circuit

Timer in open loop

Position
sensor

–Maximal deviation per day as defined on market standard (quartz? 1s/day?)
–1 time signal / minute.sent to controller
–Clock has no absolute time reference
–Time reference given by user on a 5minutes accuracy on a 12hour

User and environment
influences

Temp, Pressure, Shocks, Vibrations,
Eél, Bmagn|, Force(compression, stretch,
squeezing), Vsunlight, corrosion, sealing
SurfTens, Aging, Diffusion

Flexible compliant tube

Vs

Offset and
compliant chamber

Offset

Active display length

Actuator

8am

5:55pm

Uncontrolled or unmeasured effects on liquid length
- Sensor could be along tube (electrodes)
- Actuator could be along tube (electrodes)
- Tube could be in closed loop back to actuator

**FIG. 16C**

2. Plunger

3. Screw

1. Micro-motor

11. Battery

9. Controller
circuit

10. Potentiometer

8. Clock

4. Liquid
container &
return spring

15.
Decompressio
n chamber

14. Air/liquid
Interface

Tube Identification signal

6. Movement
detection circuit

5. Magnet

Tube coder

13. Scale

12. Light

17. Light switch

**FIG. 17A**

## Scope of the phase 1

Actuator

Reservoir

15. Decompression chamber

Air/liquid or liquid/ liquid interface

Tube coder

13. Scale

12. Light

11. Battery

9. Controller circuit

Position sensor

Tube identification signal

17. Light switch

10. Potentiometer

8. Clock

**FIG. 17B**

Generate minute signal

Generate actuation signal

Displace liquid

Read liquid level

Generate calibration sginal

Commutate light

**FIG. 17C**

**FIG. 18A**

| ID | Phase interface | Advantages | Disadvantages |
|---|---|---|---|
| **1.1** | Liquid-gas with rigid compression chamber | • Lower volume than liquid-liquid <br> • Easier assembly | • Risks with gas dissolution in liquid <br> • Risk of forming bubbles at the interface <br> • Higher pressures <br> • Sensitivity to variations of pressure and temperature |
| **1.2** | Liquid-gas with compliant compression chamber | • Lower, constant pressure <br> • Easy assembly | • Requires two bellows assemblies <br> • Highest cluttering |
| **2** | Liquid-vacuum | • Minimal volume <br> • Constant pressure | • High pressure difference with ambient <br> • More complex assembly |
| **3** | Liquid-liquid | • Low pressure <br> • Controlled miscibility whatever the environmental parameters | • Higher volume <br> • Need two bellows or a closed-loop system <br> • Possibly harder detection of the interface |

**FIG. 18B**

**FIG. 18C**

FIG. 18D

| ID | N | Description | Advantages / dis-advantages |
|---|---|---|---|
| 1.1 | Squiggle driven piston | A Squiggle drive actuates a piston, which pushes the liquid in the indicator column. | • Existing actuator<br>• Compact size<br>• High force density<br>• Possibly too high energy consumption<br>• Energy consumption for the return as well |
| 1.2.1 | Stepper motor actuating a spiral wheel | A piston is actuated by a spiral wheel. The wheel itself is rotated using one of many possible mechanical solutions. The global advantage for this class of solutions resides in the fact that the return is almost instantaneous, and requires the same energy as a normal step.<br><br>In addition, this class, as well as the 1.3 class, are the only ones which can also be driven by a mechanic watch, with only a minor adaptation. | • Simple, reliable actuators exist<br>• Low energy consumption<br>• Low cost |
| 1.2.2 | SMA (Shape-Memory Alloy) ratchet actuating a spiral wheel, as shown in FIG. 22. | | • Robust actuator<br>• High force density<br>• Compact design possible, without a gearbox |
| 1.2.3 | Spiral wheel actuated by the watch mechanism | | • Simple mechanism coupled to the existing watch mechanism<br>• May require some adaptation |
| 1.2.4 | Thermal bi-stable system actuating a spiral wheel | | • Robust actuator<br>• High force density<br>• Possibly more energy consumption than SMA |

FIG. 19A

| ID | N | Description | Advantages / disadvantages |
|---|---|---|---|
| **1.3.1** | Stepper motor actuating a rack and pinion system | The 1.3 solutions class is similar to the 1.2, except that a rack and pinion are used to actuate the piston, instead of a spiral wheel. Its disadvantage with 1.2 is that the return is not instantaneous. In addition, to perform the return, either a bidirectional actuator or a disengagement system is required. The advantages and disadvantages of each particular solution are similar to the 1.2. | |
| **1.3.2** | Rack and pinion actuated by a SMA ratchet | | |
| **1.3.3** | Rack and pinion actuated directly by the watch mechanism | | |
| **1.3.4** | Rack and pinion system actuated by a thermal bi-stable system | | |
| **2.2** | Fluid moved by electrowetting, as shown in **FIG. 23A** and **23B**. | Electrowetting allows changing the surface tension of some materials by applying an electric potential on them. By lining up electrodes, it allows displacing liquid. | • No mechanical actuator<br>• Actuation distributed on the whole display tube<br>• Possible limitations in the usable liquids |

**FIG. 19B**

| ID | N | Description | Advantages / disadvantages |
|---|---|---|---|
| 3.1.1 | Electromagnetic membrane/piston pump | Each pulse of the pump displaces the liquid in the indicator column. The return is performed by opening the valves of the pump. | • Open-loop actuation possible<br>• Possibly large device |
| 3.1.2 | Piezo membrane pump, as shown in **FIG. 24**. | The membrane of the pump is a piezo actuator. | • Very compact design<br>• Open-loop actuation possible<br>• Applications exist in the medical domain |
| 3.2.1 | Circular peristaltic pump, as shown in **FIG. 25**. | In both solutions of the 3.2 class, the liquid is pushed through the tube with a peristaltic actuation. The choice between linear and circular will depend on the geometry of the final device. | • Compatible with closed-loop liquid-liquid interface<br>• The actuator can be placed anywhere in the device, not only at the end<br>• Applications exist in the medical domain<br>• The return of the liquid has to be actuated |
| 3.2.2 | Linear peristaltic pump | | |

**FIG. 19C**

| ID | N | Description | Advantages / dis-advantages |
|---|---|---|---|
| 4.1 | Thermocapillary actuation | Similar to the electrowetting: the surface tension of the material is changed by changing its temperature. | • No mechanical actuator<br>• Actuation distributed on the whole display tube |

**FIG. 19D**

| ID | Criterion | Description | Weight | Ranking | | |
|---|---|---|---|---|---|---|
| | | | | 1 | 3 | 9 |
| 1 | Energy consumption | Average, overall energy consumption over the life of the device | 9 | high energy consumption, requires frequent changes of batteries | The device has a risk of running low on batteries before 2 years | The device can run two years on a coin cell |
| 2 | Robustness to ageing | MTBF | 9 | MTBF << 4 years | MTBF ~= 4 years | MTBF >> 4 years |
| 3 | Size | Volume occupied by the actuator assembly | 9 | Very large actuator, constrains the shape of the device | Small actuator | Insignificant actuator volume with respect to the reservoir/tube |
| 4 | Technological risk | | 9 | The solution is a novel application. Little experience is available on it. | Some challenge exists with the solution. | The solution is well established, with known examples. |
| 5 | Complexity | Complexity of the final device | 3 | The device is extremly complex | The device presents moderate complexity | The device has no particular complexity |
| 6 | Scalability | Possibility to mount different tube diameters | 3 | The device is restricted to a thin range of tube diameters | The device can be scaled to a wider range | The device can be scaled at will |
| 7 | Manual setting speed | Reaction speed of the system in case the user wants to set it by hand | 3 | Slow reaction | Possible to actuate the system faster than 1step/second, but still lagging behind manual setting | No delay with respect to the manual setting |
| 8 | Cost | Production cost of the device | 1 | | The device relies on relatively expensive, albeit known fabrication | Low cost device that can be mass-produced |

FIG. 20

| Ref | Name | Energy consumption | Robustness to ageing | Size | Technological risk | Complexity | Manual setting speed | Scalability | Cost | TOTAL | RANK |
|---|---|---|---|---|---|---|---|---|---|---|---|
| | Importance | 9 | 9 | 9 | 9 | 3 | 3 | 3 | 1 | | |
| 1.1 | Squiggle driven piston | Low energy efficiency of dynamic piezo actuators due to hysteresis losses Return has to be powered. **3** | No known effect of ageing **9** | Very small actuator possible. **9** | Known technology **9** | Existing product **9** | Slower than actuation **3** | Inadapted for very large displays **3** | Relatively costly actuator **3** | **318** | **5** |
| 1.2.1 | Stepper motor actuating a spiral wheel | Low energy consumption **9** | Very reliable actuators exist **9** | The whole assembly has a non-negligible size **3** | Existing actuators **9** | Existing actuator, low mechanical complexity **9** | As fast as actuation **9** | Scalable at will **9** | Low cost **9** | **360** | **1** |
| 1.2.3 | Spiral wheel actuated by the watch mechanism | Low energy consumption **9** | MTBF equal to that of the watch mechanism **3** | The whole assembly has a non-negligible size **3** | No specific technology **9** | Low mechanical complexity **9** | As fast as actuation **9** | May require several steps per increment for larger tubes **3** | Low cost **9** | **342** | **3** |
| 2.2 | Fluid moved by electrowetting | Low energy consumption **9** | No known effect of ageing **9** | Minimal size **9** | Few existing applications, concerns regarding the manipulation of a column of liquid. **1** | Low complexity **9** | Very fast setting possible **9** | Medium scalability **3** | Micromachining techniques are required **3** | **318** | **5** |
| 3.1.1 | Electromagnetic membrane/ piston pump | Low energy consumption **9** | Very reliable actuators exist **9** | Relatively large assembly **1** | No known risk **9** | Low complexity **9** | Slower than actuation **3** | Scalable at will **9** | Low cost **9** | **324** | **4** |
| 3.1.2 | Piezo membrane pump | Low energy consumption **9** | No known effect of ageing **9** | MEMS pumps exist **9** | Known technology **9** | The MEMS may require a development effort **3** | Slower than actuation **3** | Low scalability **1** | Piezo actUators tend to be costly **3** | **348** | **2** |

<p align="center"><strong>FIG. 21</strong></p>

EP 3 528 060 A1

**FIG. 22**

**FIG. 23A**

**FIG. 23B**

**FIG. 24**

**FIG. 25**

**FIG. 26A**

**FIG. 26B**

**FIG. 27**

**FIG. 28**

**FIG. 29A**

**FIG. 29B**

**FIG. 30**

**FIG. 31**

**FIG. 32**

**FIG. 33**

**FIG. 34**

| ID | Name | Description | Advantages / disadvantages |
|---|---|---|---|
| 1.1 | Capacitive sensor | A single or multiple electrodes are placed on the tube. The capacity indicates the progression of the liquid | • Simple direct reading of the liquid level<br>• Linear variation of the capac- ity value |
| 1.4 | Resistive sensor | Multiple electrodes are placed in the tube. The liquid connects them together | • Direct reading of the liquid level |
| 2 | Open-loop regulation | The actuator pro- vides a sufficient precision to be able to avoid using a sensor | • Simplest solution<br>• Requires a calibration routine to avoid adding errors |
| 3.1 | Inductive sensor on actuator | The actuator dis- places a ferrite in a coil. The inductance of the coil is measured and indicates the position of the actuator | • Mechanically simple solution<br>• Already in use in many precision devices |
| 3.2 | Encoder on actuator | An absolute encoder is placed on the actuator | • Simple, exact reading of the position<br>• Requires a more complex apparatus than the inductive sensor |
| 3.3 | Pressure sensor | The pressure in the compression cham- ber is measured, and indicates the pro- gression of the liquid | • Compact sensors exist<br>• Would require a calibration for the temperature |

**FIG. 35**

| Ranking of the solutions | | | | | | |
|---|---|---|---|---|---|---|
| ID | Criterion | Description | Weight | Ranking | | |
| | | | | 1 | 3 | 9 |
| 1 | Sensitivity to environmental parameters | Risk of variation of the display with environmental parameters | 9 | The sensor is highly sensitive to the environment | Some sensitivity exists, but can be compensated for | The sensor is insensitive to the environment |
| 2 | Robustness to ageing | MTBF | 9 | MTBF << 4 years | MTBF ~= 4 years | MTBF >> 4 years |
| 3 | Max likely error | Maximal error that can have a significant probability of appearing on the display | 9 | > 1 step | ~= 1 step | < 1 step |
| 4 | Complexity | Overall design complexity of the device | 3 | Very complex sensor | Moderate complexity | Simple system |

**FIG. 36**

| Ref | Name | Sensitivity to environmental parameters | Robustness to ageing | Maximal likely error | Complexity | TOTAL | RANK |
|---|---|---|---|---|---|---|---|
| Ref | Importance | 9 | 9 | 9 | 3 | | |
| 1.1 | **Capacitive sensor** | Insensitive: full closed-loop | No known issues | closed-loop: error inferior to 1 step | Very simple system | | 1 |
| | | 9 | 9 | 9 | 9 | 270 | |
| 1.4 | **Resistive sensor** | Insensitive: full closed-loop regulation | | closed-loop: error inferior to 1 step | More complex system, involves electrodes in the liquid | | 4 |
| | | 9 | 1 | 9 | 3 | 180 | |
| 2 | **Open-loop** | Insensitive for true volumetric dispensers | No issues on sensing side, particular caution required on actuator side | error ~= 1 step | Very simple system | | 3 |
| | | 9 | 6 | 3 | 9 | 189 | |
| 3.1 | **Inductive sensor on the actuator** | Insensitive: the displacement of the liquid is tracked | No known issues | error ~= 1 step | Very simple system | | 2 |
| | | 9 | 9 | 3 | 9 | 216 | |
| 3.2 | **Encoder on the actuator** | Insensitive: the displacement of the liquid is tracked | No known issues | error ~= 1 step | Very simple system | | 2 |
| | | 9 | 9 | 3 | 9 | 216 | |
| 3.3 | **Pressure sensor** | Very sensitive, temperature variations have to be compensated | Possible drift of the reference pressure | Large error possible in case of miscalibration | Complex sensor, has to integrate temperature sensor as well | | 5 |
| | | 1 | 3 | 1 | 1 | 48 | |

**FIG. 37**

EP 3 528 060 A1

**GND**

$V_{cap}$

FIG. 38A

Ground electrode
Top-plate
Glass substrate
Insulation  Connected to GND  Insulation
Insulation
Glass substrate
Bottom-plate
Control electrodes
High capacity

FIG. 38B

*Coil*

**Ferrite**

FIG. 39

**FIG. 40A**

**FIG. 40B**

**FIG. 41**

**FIG. 42**

**FIG. 43**

**FIG. 44**

**FIG. 45**

**FIG. 46**

**FIG. 47**

**FIG. 48**

Piston return time as a function of the return force, piston radius and tube radius considered for a silicone-silicone interface

Return time = 0.1 [s]
Return time = 0.5 [s]
Return time = 1 [s]
Return time = 5 [s]
Return time = 10 [s]
Return time = 20 [s]
Return time = 30 [s]

Tube radius [mm]

Piston radius [mm]

Return force [N]

FIG. 49

Piston return time as a function of the return force, piston radius and tube radius considered for a water-water interface

Return time = 0.1 [s]
Return time = 0.5 [s]
Return time = 1 [s]
Return time = 5 [s]
Return time = 10 [s]
Return time = 20 [s]

Tube radius [mm]

Piston radius [mm]

Return force [N]

FIG. 50

*Red: the spiral itself, corrected in order to take into account the friction*

**FIG. 51**

**FIG. 52**

**FIG. 53**

**FIG. 54**

FIG. 55

FIG. 56

**FIG. 57**

**FIG. 58**

| Function | Solution 1 | Solution 2 | Solution 3 | Solution 4 | Solution 5 |
|---|---|---|---|---|---|
| Phases inter-face | S1.1 Liquid-gas with compliance | S3. Liquid-liquid | | | |
| Displace liquid | S1.2.1 Stepper + spiral wheel | S3.1.2 Piezo membrane pump | S3.1.1 Electromagnetic pump | S2.2 Electrowetting | S1.1 Squiggle drive |
| Detect liquid position | S1.1 Capacitive sensor | S3.1 Inductive sensor on actuator | S3.2 Encoder on actuator | S2. Open-loop | |

FIG. 59

FIG. 60

| ID | Object | Assumption | Explanation |
|---|---|---|---|
| | Tube inner diameter | 1 [mm] | easily available |
| | Tube material | Polyurethane | easily available high CTE |
| | Reservoir material | PET | easily available high CTE |
| | Liquid #1 | Water | easily available low viscosity low thermal expansion |
| | Liquid #2 | Heptane | colorless easily available low viscosity similar density as water non toxic |
| | Dye | Sulforhodamine B (kiton red) | easily available strong color fully soluble in water, but not in heptane |
| | K2 (ratio between reservoir and tube diameter) | 2 | relatively low while allowing for an easy assembly |
| | Movement | 6 ¾ '' x 8 '' watch movement, according to **FIG. 62**. | easily available low-cost representative of mechanical and electrical performances of watches |

**FIG. 61**

**FIG. 62**

| | | | |
|---|---|---|---|
| | | | |
| Longain LG-02<br><br>Ø22 x 3.2mm<br><br>Plastic | Panyu pearl SL-28<br><br>Ø16x 3.2mm<br><br>Plastic | Longain Y628<br><br>? x 16 x 3.2mm<br><br>Plastic | ETA 802.101<br><br>17.8 x 15.3 x 3.15mm<br><br>Metallic |

**FIG. 63**

| | |
|---|---|
| 1) Battery, providing the power<br>2) Integrated circuit, controlling the quartz and the stepping motor<br>3) Oscillating quartz, dividing the time<br>4) Trimmer, regulating the frequency<br>5) Stepping motor, transforming the electrical impulses into mechanical power<br>6) Gear train, activating the hours, minutes, seconds hands<br>7) Analog display | |

*Source: http://www.fhs.ch*

**FIG. 64A**

1) Barrel/mainspring providing the power
2) Gear train, transmitting the power
3) Escapement, distributing the impulses
4) Balance wheel & hairspring, oscillating, making the division of time
5a) Winding stem, for manual winding and setting
5b) Oscillating weight, for automatic winding
6) Dial train, activating the hours, minutes, seconds hands

Source: http://www.fhs.ch

**FIG. 64B**

**FIG. 65**

Ø 44 [mm]

**FIG. 66A**

**FIG. 66B**

**FIG. 66C**

**FIG. 67**

**FIG. 68**

Ø 44 [mm]

**FIG. 69A**

h = 8.5

**FIG. 69B**

**FIG. 70**

**FIG. 71**

FIG. 72A

FIG. 72B

FIG. 72C

FIG. 73

l = 30 [mm]

h = 9 [mm]

FIG. 74

FIG. 75

**FIG.76**

**FIG. 77**

| | Mean torque | | |
|---|---|---|---|
| | Teflon-steel, μ = 0.05 | WC-WC, μ = 0.2 | WC-steel, μ = 0.4 |
| Flat design | 176 [μNm] | 595 [μNm] | 1100 [μNm] |
| Long design, circular | 187 [μNm] | 324 [μNm] | 520 [μNm] |

**FIG. 78**

**FIG. 79**

**FIG. 80**

**FIG. 81**

**FIG. 82**

| ID | Component | # | Specifications | Manufacturer | Model | Size [mm$^3$] |
|---|---|---|---|---|---|---|
| 1 | Microcontroller | 1 | Ultra low power consumption | OKI | ML610Q4xx Family | 9 x 9 x 1.2 |
| 2 | Capacitive sensor reading chip | 1 | i$^2$c interface 13 channels | Analog Devices | AD7147ACPZ-1 | 4 x 4 x 1 |
| 3 | Multiplexer | 4 | 4 bit analog multiplexer | Analog Devices | ADG1606 | 5 x 5 x 1 |
| 4 | Flip-flop | 1 | 3 gates for the commutation 3 gates for the astable circuit for the pulse | ST Microelectronics | 74LCX574 | 6.4 x 6.2 x 1.2 |
| 5 | Driving voltage source | 1 | 28 [V] max output voltage low power Low quiescent current (28 [µA]) | Texas Instruments | TPS61040DRVT | 2.1 x 2.1 x 0.8 |
| 6 | Switch | 1 | 3 channels 25 [V] max voltage | Analog Devices | ADG1233YRUZ | 4 x 4 x 1 |
| 7 | Coin cell | 1 | 25 [mAh], 3 [V] | Varta | CR1216 | Ø 12 x 1.6 |

FIG. 83

FIG. 84

FIG. 85A

watch movement including coin cell
and time adjustment button

Cam wheel

Piston

Lip-seal

Liquid 2 chamber
(empty)

Liquid 1
chamber
(full)

Liquid 1
channel port

Rolling diaphragm

Location for a compression spring or spiral spring
for stroke reset force

**FIG. 85B**

28.85mm

15.2mm

**FIG. 85C**

Liquid 2 channel port
(on the side)

8.1mm

18.2mm

**FIG. 85D**

**FIG. 85E**

902

904

Fluidic connection

**FIG. 86A**

Fluidic connection

**FIG. 86B**

FIG. 86C

FIG. 86D

**FIG. 87A**

Ø48mm
x 9mm

**FIG. 87B**

**FIG. 87C**

**FIG. 87D**

Channel display at outer edge

Possibility to display seconds

Display of mechanical / fluidic coupling

For a pure mechanical movement, the back side of watch can be transparent as well

Time display is not in exact 360° display due to channel overlap

**FIG. 87E**

Start

End

5° unused
Time display on 355°

Actual time

**FIG. 87F**

**FIG. 88A**

**FIG. 88B**

**FIG. 88C**

**FIG. 89A**

**FIG. 89B**

**FIG. 89C**

FIG. 89D

FIG. 89E

FIG. 89F

9mm

30mm

16.5mm

FIG. 89G

FIG. 89H

Watch with hands

Fluidic display

**FIG. 90**

Fluidic display

**FIG. 91**

Channels inside of both branches

**FIG. 92A**

6pm (or am)

12

6am (or pm)

**FIG. 92B**

**FIG. 92C**

**FIG. 92D**

**FIG. 92E**

**FIG. 92F**

**FIG. 93**

**FIG. 94**

**FIG. 95A**

**FIG. 95B**

**FIG. 96**

**FIG. 97**

EP 3 528 060 A1

**FIG. 98**

**FIG. 99**

111

| | | |
|---|---|---|
| Europäisches Patentamt | **DECLARATION** | Application Number |
| European Patent Office | which under Rule 63 of the European Patent Convention EP 18 21 5944 | |
| Office européen des brevets | shall be considered, for the purposes of subsequent proceedings, as the European search report | |

| The Search Division considers that the present application, does not comply with the provisions of the EPC to such an extent that it is not possible to carry out a meaningful search into the state of the art on the basis of all claims | CLASSIFICATION OF THE APPLICATION (IPC) |
|---|---|
| Reason: | INV. G04C17/00 G02B26/00 G04G9/02 |

The present reasoned declaration is issued under Rule 63(2) EPC.

The search division has found that no meaningful search is possible for claims 1-17 of the present application, as the entire subject-matter of claims 1-17 violates Article 76 EPC (see also Guidelines B-VIII-3, point (iv) in particular).
For the details, the applicant is invited to refer to the attached separate sheet.

The applicant's attention is drawn to the fact that a search may be carried out during examination following a declaration of no search under Rule 63 EPC, should the problems which led to the declaration being issued be overcome (see EPC Guideline C-IV, 7.2).

| Place of search | Date | Examiner |
|---|---|---|
| The Hague | 3 July 2019 | Pirozzi, Giuseppe |

EPO FORM 1504 (P04F37)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 10752392 A
- US 61235725 A
- US 61349897 A
- WO 2010002054 W
- US 3783598 A
- FR 1552838

**Non-patent literature cited in the description**

- Horloges Anciennes. Office du Livre. February 1978, 9